# EUROPEAN PATENT APPLICATION

(11) **EP 4 636 082 A2**
(43) Date of publication of application: **22.10.2025**
(21) Application number: 23885148.9
(22) Date of filing: 12.12.2023
(51) Int. Cl.: C12N 9/12, C07K 19/00, C12N 9/22, C12N 15/54, C12N 15/55, C12N 15/62, A61K 48/00, C12N 15/85, A61K 38/46, A61P 17/00, A61K 38/45

(54) **PBASE PROTEIN, FUSION PROTEIN, NUCLEIC ACID, GENE INTEGRATION SYSTEM AND USE**

(30) Priority: 12.12.2022 CN 202211600490
(71) Applicant: GeCell Therapeutics, LLC., Beijing 102600 (CN)
(72) Inventor: XU, Naiqing, Shanghai 200437 (CN); LIU, Jingxian, Shanghai 200437 (CN); XU, Tian, Shanghai 200437 (CN)
(74) Representative: Winter, Brandl - Partnerschaft mbB
(86) International application number: PCT/CN2023/138225
(87) International publication number: WO 2024/094224

(57) **Abstract**

Provided in the present invention are a PBase protein, a fusion protein, a nucleic acid, a gene integration system, a gene delivery system and the use. The PBase protein retains the abilities of binding to the ITR sequences of a Piggybac transposon and cleaving the Piggybac transposon to make the Piggybac transposon separate from a donor plasmid in a linear manner; and the PBase protein has a lower ability for binding to the double strands of DNA within 10 bp upstream and downstream of "TTAA". When the PBase protein is linked to a site-specific DNA nuclease to prepare the fusion protein, the PBase protein can be efficiently integrated with a long fragment gene of interest at the double strand breaks generated by the site-specific DNA nuclease, thereby achieving the effect of site-directed insertion of the long fragment gene.

## Description

This application claims priority to Chinese patent application 202211600490X filed on December 12, 2022. This application cites the full text of the aforementioned Chinese patent application.

### Technical field

The present disclosure belongs to the field of gene editing, and specifically relates to a PBase protein, fusion protein, nucleic acid, gene integration system, and use.

### Background Art

The loss of protein function caused by endogenous gene mutations has led to many human diseases. Currently, one of the main strategies for gene therapy is to introduce the correct sequence of exogenous genes (Gene of Interest, GOI) and stably express them to compensate for the loss of endogenous protein function. One main strategy for achieving stable expression of exogenous genes is to integrate exogenous gene sequences into genes through methods such as virus integration, transposase transposon system, or CRISPR Cas system mediated homologous recombination (HR). Virus integration and transposase transposon systems can efficiently integrate exogenous genes into the genome, but their random integration characteristics result in high genetic toxicity. CRISPR Cas mediated DSB can achieve targeted integration of exogenous genes through homologous recombination (HR) pathway repair, but the length of integrated gene fragments is short and the integration efficiency is low. In addition, preparing linear donor DNA containing homologous arms is time-consuming and expensive. Therefore, developing new gene editing tools that can efficiently integrate exogenous DNA sequences, especially long fragments, into genome specific loci has become a major challenge in the current field of gene therapy.

The PB transposase transposon system can efficiently perform gene transposition integration in mammalian cells, providing a good molecular basis for the development of efficient targeted integration tools through molecular evolution. PB transposase can cleave transposon DNA carrying GOI and efficiently integrate it into the "TTAA" target site in the mammalian genome in a seamless manner. Previous studies have shown that by mutating some sites on PBase enzyme that bind to genomic target DNA, the PBase enzyme can retain its cutting activity and ITR binding ability to PB transposon DNA, but almost completely lose its activity of integrating transposon DNA into the "TTAA" site of the genome, which can alter the efficiency of transposon integration ("Exc+Int-" mutant). And some studies have found that combining the corresponding "Exc+Int-" mutant with Cas9 gRNA can integrate Transposon DNA at specific sites guided by gRNA. However, it still faces the problem of low efficiency in fixed-point integration and high off target rate.

In 2013, a PBase paper was published in a laboratory in the United States (DOI: 10.1073/ pnas.1305987110). The R372A K375A D450N mutant exhibited transposon donor DNA cleavage activity, but had low "TTAA" integration ability. So, patent WO2020250181 utilizes the R372A K375A D450N mutant developed by American researchers to bind with nucleases such as Cas9, which has targeted integration function, and has made some extensions to the PB mutant. However, the existing technology still lacks PB mutants with higher integration efficiency and lower "TTAA" integration ability.

### Summary

In response to the technical defects of low efficiency and high off target rate of PBase site integration in the existing technology, the present disclosure provides a PBase protein, fusion protein, nucleic acid, gene integration system, gene delivery system, and use. The PBase protein retains the ability to bind to the ITR sequence of the Piggybac transposon and cleaves it to isolate the Piggybac transposon from the donor plasmid in a linear manner, and has a lower ability to bind to DNA double strands within 10 bp upstream and downstream of "TTAA". When the PBase protein is linked to site-specific DNA nucleases to prepare a fusion protein, the PBase protein can efficiently integrate long foreign genes at the double strand breaks produced by site-specific DNA nucleases, thereby achieving the effect of targeted insertion of long genes.

In order to solve the above technical problems, the present disclosure provides a PBase protein, which is based on the amino acid sequence shown in SEQ ID NO: 3, and contains amino acid mutations in at least three of the following amino acid sites: positions 166, 286, 289, 302, 315, 347, 375, 376, 441, 450, 498, 520, 521, 523, and 557. The PBase protein retains or enhances gene integration ability, and the target site and recognition ability of the PBase protein are reduced.

The gene integration ability is reflected in the combination of the ITR binding ability of PBase and the activity of cleaving transposon donors.

In the present disclosure, the ITR binding ability refers to the affinity of the PBase protein binding sequence specific Piggybac transposon ITR.

In the present disclosure, the cleavage transposon donor activity refers to the ability of PBase protein to cleave and free the Piggybac transposon from the corresponding donor DNA double strand.

In the present disclosure, the target site recognition ability refers to the ability of PBase protein to bind double stranded DNA within 10 bp upstream and downstream of the target site "TTAA" by relying on its own DNA binding region.

Preferably, the PBase protein includes an amino acid mutation at least one of the following amino acid sites based on the amino acid sequence shown in SEQ ID NO: 3: R315, N347, K375, and R376, and at least one of the following amino acid sites: A166, N286, S289, G302, Q441, D450, S498, E520, A521, T523, and T557. In particular, amino acid mutations on R315, N347, K375, and R376 can reduce the target site recognition ability of the PBase protein, while amino acid mutations on A166, N286, S289, G302, Q441, D450, S498, E520, A521, T523, and T557 can enhance the ITR binding ability or cleavage transposon donor activity of the PBase protein.

Preferably, the PBase protein includes at least the following amino acid mutation combinations based on the amino acid sequence shown in SEQ ID NO: 3:
(1) R315, N347, and D450; or
(2) N347, R376, and D450.

Preferably, the amino acid mutation is an amino acid substitution, preferably as shown in Table 1.

**Table 1 Amino acid substitutions at PBase mutation sites**

| site | wild amino acid residues | mutant amino acid residues |
|---|---|---|
| 166 | A | H, R, K |
| 286 | N | H, R, K |
| 289 | S | H, R, K |
| 302 | G | H, R, K |
| 315 | R | A, E, D |
| 347 | N | S, T, Y, Q, C, G, A, E, D |
| 375 | K | A, E, D |
| 376 | R | A, E, D |
| 441 | Q | H, R, K |
| 450 | D | N, S, T, C, Y, A, R |
| 498 | S | N |
| 520 | E | H, R, K |
| 521 | A | H, R, K |
| 523 | T | R, N |
| 557 | T | H, R, K |

In a preferred embodiment of the present disclosure, the PBase protein includes at least the following amino acid mutation combinations based on the amino acid sequence shown in SEQ ID NO: 3:
(1) R315A/E/D,N347S/T/Y/Q/C/G/A/E/D and D450N; or
(2) N347S/T/Y/Q/C/G/A/E/D,R376E and D450N.

More preferably, the PBase protein includes at least the following amino acid mutation combinations based on the amino acid sequence shown in SEQ ID NO: 3:
(1) R315A, N347E and D450N; or
(2) N347A, R376E and D450N; or
(3) N347E,R376E and D450N.

More preferably, the PBase protein includes an amino acid mutation combination N347A, R376E, and D450N based on the amino acid sequence shown in SEQ ID NO: 3, and further includes one or more of the following amino acid mutations: A166H/R/K, N286H/R/K, S289H/R/K, G302H/R/K, Q441H/R/K, S498N,E520H/R/K,A521H/R/K, T523R/N Mutations in T557H/R/K.

Furthermore, the PBase protein includes amino acid mutation combinations N347A, R376E, and D450N based on the amino acid sequence shown in SEQ ID NO: 3, as well as one or more of A166K, N286R, S289R, G302R, Q441R, S498N, E520R, A521R, T523R, and T557R.

In a specific embodiment of the present disclosure, the amino acid sequence of the PBase protein is shown in SEQ ID NO: 5, 7, or 9.

To solve the above technical problems, the present disclosure provides a fusion protein including a PBase protein fused to a site-specific DNA nuclease as described herein, and the original functions of the PBase protein and the specific DNA nuclease remain unchanged after fusion.

In the present disclosure, the site-specific DNA nucleases are nucleases that have the function of specifically recognizing and binding to nucleic acid sequences, and can cleave the recognized nucleic acid sequence to produce double strand breaks (DSBs), such as meganuclease, Cas, ZFN, and TALEN.

In the present disclosure, the Cas is a Cas enzyme in the CRISPR system that can generate site-specific DNA double strand breaks. Common examples include Cas9 and Cas12, which require recognition of the target site mediated by guide RNA (sgRNA).

In the present disclosure, the ZFN is a zinc finger structure recognition sequence fusion DNA endonuclease, such as Zinc Finger FokI.

In the present disclosure, the TALEN is a transcription activator like effector recognition sequence fusion endonuclease, such as TALE FokI.

In the present disclosure, the meganuclease is a homing endonuclease with a longer recognition sequence, such as I-SceI.

In the present disclosure, the PBase protein has the following function: inserting the target gene from the donor into the double strand breaks produced by the site-specific DNA nuclease.

Preferably, in the fusion protein, the site-specific DNA nuclease and the PBase protein are directly connected by a chemical bond or connected through a linker, preferably 3 * G₄S, and the chemical bond is preferably a peptide bond. More preferably, the structure connected by a linker from the N-terminus to the C-terminus is a site-specific DNA nuclease linker PBase protein.

Preferably, the fusion protein further includes a Flag sequence between the site-specific DNA nuclease and the PBase protein, which is only used for detecting the fusion protein and does not affect its function.

Preferably, the specific DNA nuclease includes an NLS sequence, which refers to a nuclear localization sequence. The amino acid sequence of the NLS sequence can be at positions 1373-1379 of the amino acid sequence shown in SEQ ID NO: 1. When the specific DNA nuclease includes an NLS sequence, the structure of the fusion protein from N-terminus to C-terminus is: specific DNA nuclease NLS linker PBase protein.

In a specific embodiment of the present disclosure, the PBase protein in the fusion protein lacks methionine (M) at position 1 of the amino acid sequence.

In a specific embodiment of the present disclosure, the amino acid sequence of the fusion protein is shown as SEQ ID NO: 6, 8, or 10.

To solve the above technical problems, the present disclosure provides a technical solution of an isolated nucleic acid encoding PBase protein and/or fusion protein as described herein

In a specific example of the present disclosure, the isolated nucleic acid includes a nucleotide sequence as shown in positions 4183-5964 of SEQ ID NO: 135-137, or the isolated nucleic acid includes a nucleotide sequence as shown in SEQ ID NO: 135-137.

The M encoded by the start codon ATG is cleaved during the maturation of PBase protein, and the cleavage of the starting amino acid M does not affect the function of PBase protein; And fusion proteins often do not require downstream PBase coding sequences containing the start codon ATG. Therefore, the isolated nucleic acid may or may not include the start codon ATG upstream of the coding region of PBase.

To solve the above technical problems, the present disclosure provides a gene integration system including a PBase protein or a fusion protein as described herein, or an isolated nucleic acid as described herein; and, Piggybac transposon donor nucleic acid carrying exogenous target genes.

Preferably, the fusion protein includes a PBase protein and a Cas system as described herein, wherein the Cas system includes Cas proteases and guide RNA (sgRNA), such as Cas9 and Cas12 proteases.

In a specific embodiment of the present disclosure, the Piggybac transposon donor nucleic acid has RE ITR and LE ITR sequences on both sides of the exogenous target gene, which can be both RE ITR and LE ITR, or both RE ITR and LE ITR, respectively.

To solve the above technical problems, the present disclosure provides a gene delivery system including one or more vectors, wherein the vectors include PBase protein as described herein, fusion protein as described herein, isolated nucleic acid as described herein, and one or more of the gene integration system as described herein. The vectors also include Piggybac transposon donor nucleic acid carrying exogenous target genes, such as adenovirus vectors, lipid nanoparticles, lentiviral vectors, ribonucleoprotein complexes, and virus like particles.

A gene integration method including the steps of integrating an exogenous target gene into the genome of a host cell using a gene integration system as described herein.

Preferably, the gene integration system is introduced into the host cell through a gene delivery system as described herein.

Preferably, the method is for non diagnostic and/or therapeutic purposes.

To solve the above technical problems, the present disclosure provides a pharmaceutical composition including one or more of PBase protein as described herein, fusion protein as described herein, isolated nucleic acid as described herein, gene integration system as described herein, and gene delivery system as described herein.

In order to solve the above technical problems, the present disclosure provides a reagent kit including one or more of PBase protein as described herein, fusion protein as described herein, isolated nucleic acid as described herein, gene integration system as described herein, gene delivery system as described herein, and drug composition as described herein.

To solve the above technical problems, the present disclosure provides a kit set including kit A, wherein kit A includes one or more of PBase protein as described herein, fusion protein as described herein, isolated nucleic acid as described herein, gene integration system as described herein, gene delivery system as described herein, drug composition as described herein, and reagent kit as described herein.

Preferably, the kit set further includes kit B, which includes reagents for detecting or screening whether gene integration is successful, such as detection reagents that specifically bind to the integrated gene, such as probes and antibodies.

In order to solve the above technical problems, the present disclosure provides a use of one or more of the PBase protein, fusion protein, isolated nucleic acid, gene integration system, gene delivery system, drug composition, and reagent kit described herein in the preparation of reagents for integrating genes or treating genetic diseases.

Preferably, the application is for non-diagnostic or non-therapeutic purposes.

Preferably, the exemplary genetic disease is epidermolysis bullosa.

To solve the above technical problems, the present disclosure provides a method for integrating genes or treating gene diseases, which includes administering one or more of PBase protein, fusion protein, gene integration system, gene delivery system, drug composition, and reagent kit as described in this article to a subject in need. The genetic disease, such as epidermolysis bullosa.

To solve the above technical problems, the present disclosure provides a technical solution including one or more of PBase protein, fusion protein, gene integration system, gene delivery system, drug composition, and reagent kit for integrating genes or treating gene diseases as described in this article. The exemplary genetic disease is epidermolysis bullosa.

In the present disclosure, the mutant is named after the mutation it contains, and the template for the mutation is wild-type PBase (SEQ ID NO: 3). For example, PBase N347A_R376E_D450N includes three amino acid mutations based on SEQ ID NO:3 including A at position 347, E at position 376, and N at position 450.

The preparation of PBase protein according to the present disclosure can be carried out using conventional methods for preparing proteins in this field.

Based on common knowledge in this field, the above preferred conditions can be combined arbitrarily to obtain the preferred examples of the present disclosure.

The reagents and raw materials used in the present disclosure are commercially available.

The positive progressive effect of the present disclosure is that:
Compared with the PBase mutants in the existing technology (such as R372A K375A D450N and further mutations based on it), the PBase protein provided by the present disclosure has higher transposon DNA cleavage activity (theoretically, higher cleavage activity indicates higher targeted integration efficiency), and the ability to bind DNA double strands within 10 bp upstream and downstream of "TTAA" decreases, resulting in a decrease in "TTAA" dependent integration ability (theoretically, lower "TTAA" dependent integration ability indicates lower off target). When the PBase protein is linked to site-specific DNA nucleases to prepare the fusion protein of the present disclosure, the PBase protein can efficiently integrate long foreign genes at the double strand breaks produced by site-specific DNA nucleases, thereby achieving the effect of targeted insertion of long genes. In addition, the PBase protein, fusion protein, nucleic acid, and gene integration system provided by the present disclosure can achieve longer length knock in of exogenous genes (e.g. up to 12kb) compared to conventional homologous recombination, and can use circular plasmids as donors, simplifying the donor DNA preparation process and reducing costs, thus enhancing the value of industrial applications.

### Description of the drawings

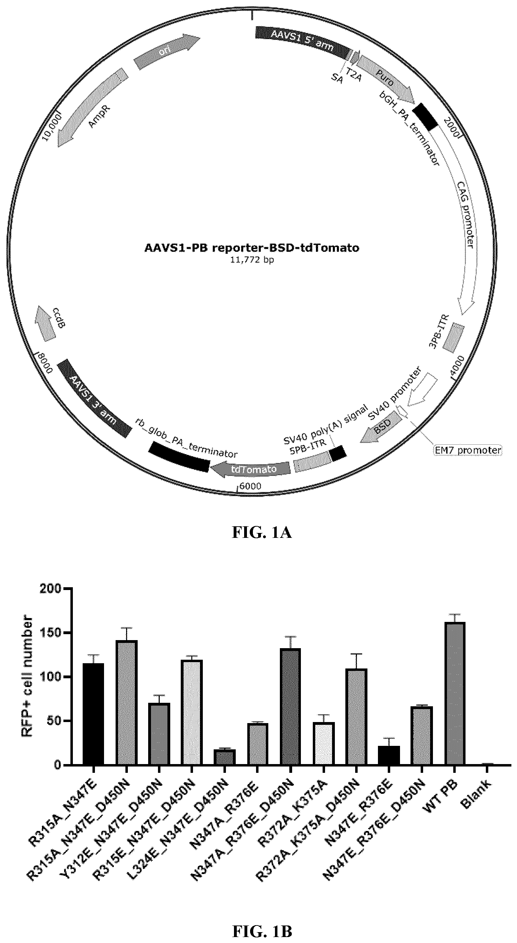

FIG. 1A shows the plasmid map of the RFP reporter gene. This map has AAVS1 homologous arms, which can be homologous recombined into the AAVS1 site of the human cell genome, and positive cells can be screened using puromycin. The expression of the RFP gene driven by the CAG promoter is blocked by the Piggybac transposon sequence containing the SV40 promoter Bacillus subtilis resistance gene polyA expression cassette. The cleavage of the Piggybac transposon sequence can restore RFP expression. The expression efficiency of RFP can represent the efficiency of PBase cleavage of transposons.
FIG. 1B shows the number of RFP positive cells transfected with different PBase mutant plasmids in reporter cells to reflect their transposon cleavage activity. The RFP reporter gene (as the profile shown in FIG. 1A) was integrated into the AAVS1 site of 293F cells using Cas9 gRNA homologous recombination method and positive monoclonal cell lines were selected as reporter cells. PBase and its mutants were constructed in the pcDNA3.1 vector and transfected into reporter cells. Count the number of RFP positive cells 5 days after transfection.

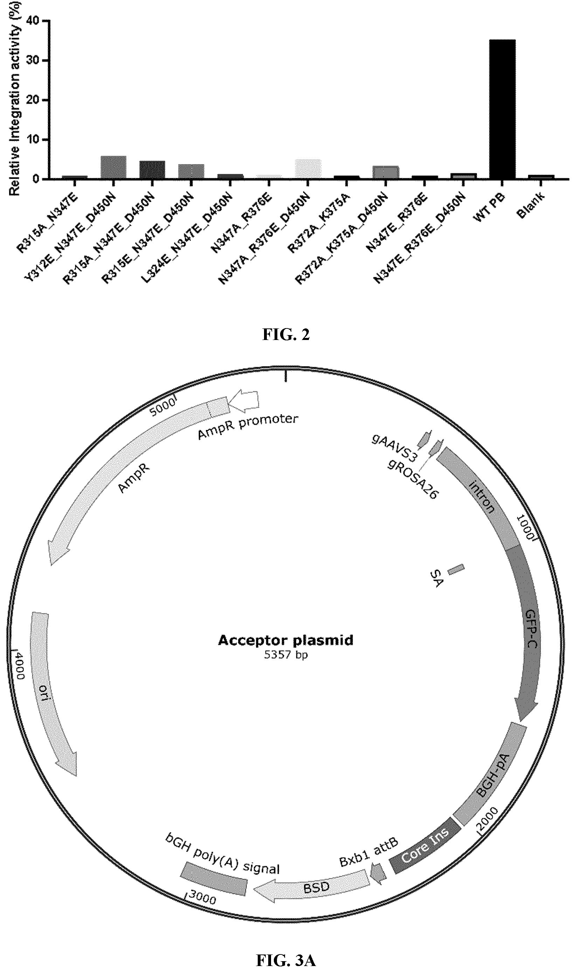

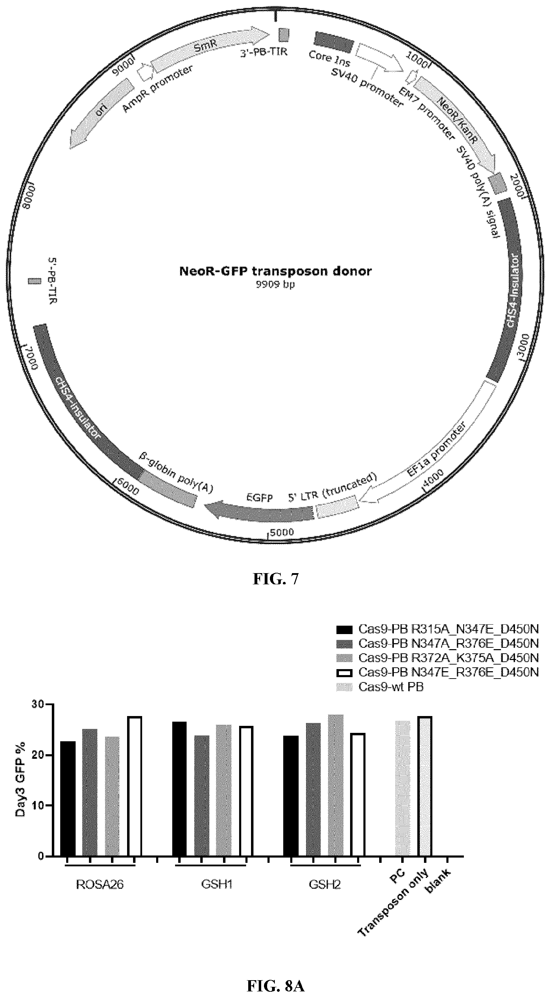

FIG. 2 shows the relative integration efficiency of wt Hyperactive PBase and its mutants in HeLa cells. Co transfect NeoR GFP transposon donor DNA plasmid (as shown in FIG. 7) and pcDNA3.1-PBase plasmid in HeLa cells. Two days and 14 days after transfection, the GFP positive ratio was detected using flow cytometry, representing transfection efficiency and integration efficiency, respectively. The relative integration efficiency is calculated based on the ratio of GFP on the 14th day to GFP on the 2nd day.
FIG. 3A shows the plasmid profile of GFP C-terminal receptor. Add a gRNA recognition site target region and an mRNA splicing acceptor upstream of the GFP C-terminal coding sequence on the plasmid. The Bxb1attB-BSD sequence of the receptor plasmid does not have a promoter upstream, and can only be driven by the specific recombination of Bxb1 integrase to the Bxb1attP site downstream of the pre placed PGK promoter on the genome, in order to select positive recombinant cells with Bacillus subtilis and establish a single clone cell line.

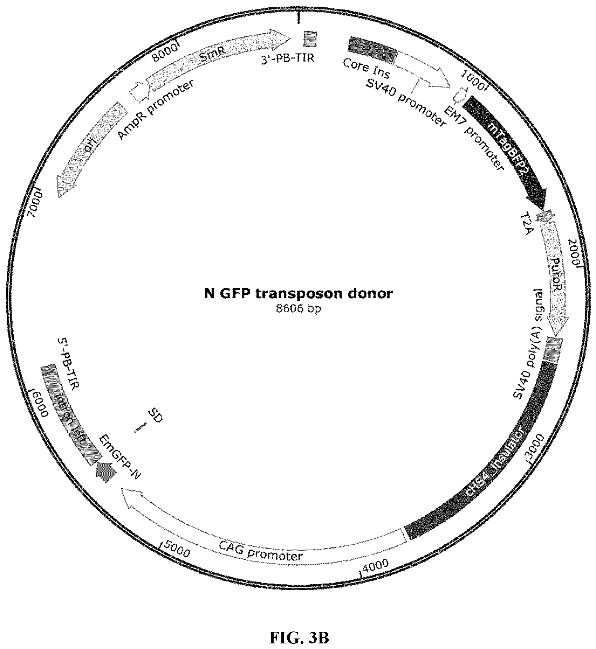

FIG. 3B shows the plasmid profile of PB transposon donor with GFP N-terminal coding sequence. The transposon donor plasmid contains the mTagBFP2-2A-Puro expression cassette, which continuously expresses BFP-2APuro in transfected positive cells and can be screened with puromycin. The downstream of the CAG promoter contains the GFP N-terminal coding sequence and an mRNA splicing donor downstream. If the transposon donor DNA is targeted and integrated into the upstream gRNA target region of the GFP C-terminal coding sequence, complete green fluorescent protein can be expressed through mRNA mature cleavage after transcription of the CAG promoter, and the proportion of GFP positive cells can be detected to represent the efficiency of site integration.

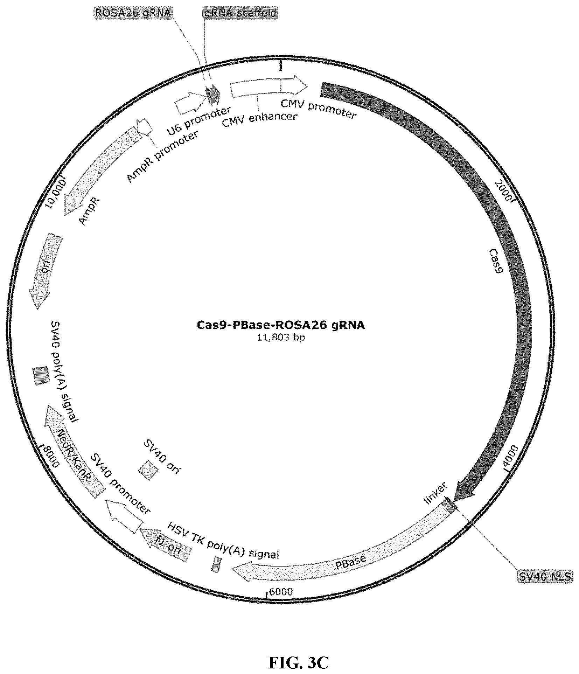

FIG. 3C shows the plasmid profile of CMV promoter expressing spCas9-PBase and U6 promoter driving ROSA26gRNA transcription. NLS (nuclear localization sequence) refers to the nuclear localization sequence.

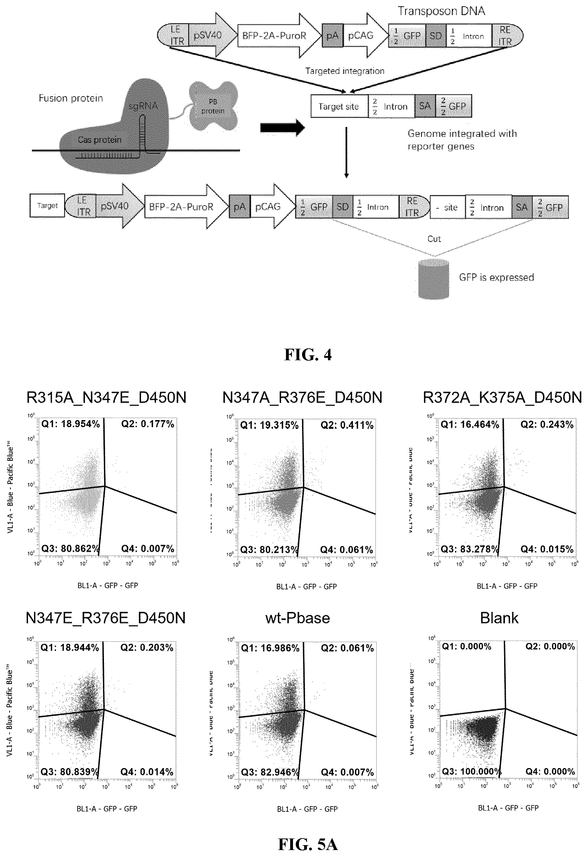

FIG. 4 is a schematic diagram of the principle for analyzing the efficiency of integrating specific positioning points. The transposon donor sequence is integrated into the sgRNA recognition target site upstream of the GFP C-terminal coding sequence, resulting in BFP⁺GFP⁺ cells through forward integration, and BFP⁺GFP⁺ cells through reverse integration and non site integration.
FIG. 5A shows the mTagBFP2 and GFP positivity rates co transfected with different Cas9-PBase-ROSA gRNA plasmids (shown in FIG. 3C) and transposon donor plasmids (shown in FIG. 3B) in cells containing the target sequence (sequence shown in FIG. 3A). The positivity rates of mTagBFP2 and GFP were detected by flow cytometry after transfection for 4 days. According to the schematic diagram in FIG. 4, the proportion of mTagBFP2 represents transfection efficiency, while the proportion of GFP represents the efficiency of targeted integration.

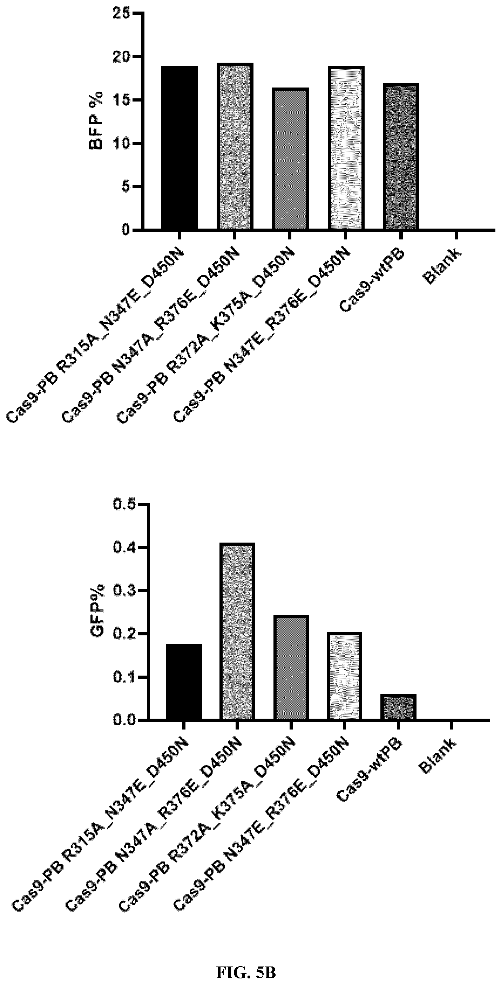

FIG. 5B shows the statistical results of the percentage of mTagBFP2 and GFP (according to FIG. 5A).

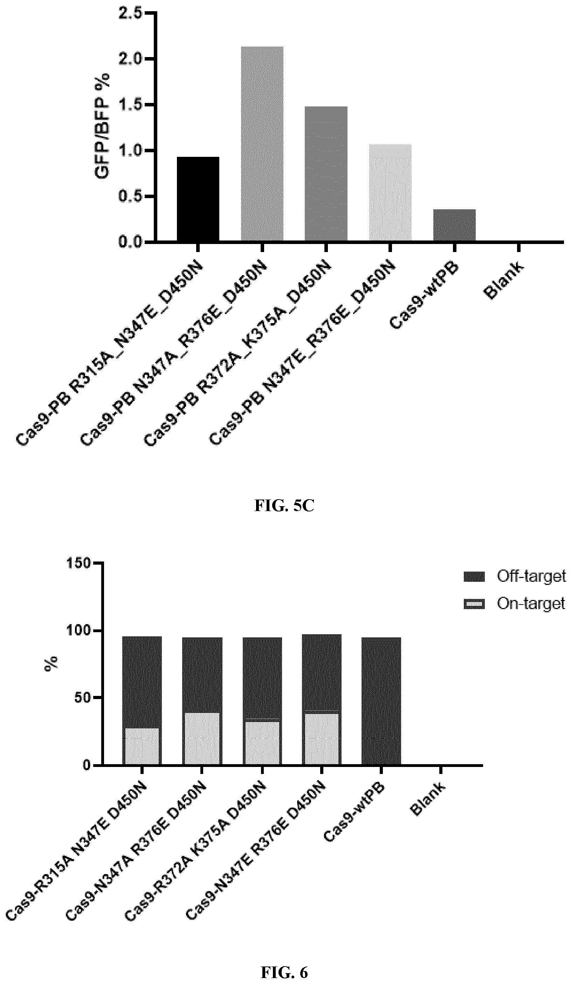

FIG. 5C shows the ratio of targeted integration efficiency/transfection efficiency for different Cas9-PBase mutants. This ratio reflects the site-specific integration activity of Cas9-PBase mutants.
FIG. 6 shows the ratio of targeted integration and off target integration in a stable integrated cell population enriched by puromycin screening. Cells co transfected with Cas9-PBase enzyme and transposon donor plasmid were cultured continuously for 12 days after transfection, and then screened and enriched with puromycin for 10 days. The proportion of mTagBFP2 and GFP positive cells was detected by flow cytometry. The proportion of targeted integration is calculated based on a 2 * GFP positive percentage.
FIG. 7 shows another transposon map that can continuously express the neomycin resistance gene and GFP (NeoR GFP transposon).
FIGs. 8A- 8C demonstrate the efficiency of the Cas9-PBase system in integrating NeoR GFP transposons at three safe sites (ROSA, GSH1, GSH2) in the genome. 8A displays Cas-PBase-gRNA plasmid (carrying 3 different gRNAs) and NeoR-GFP transposon plasmid co transfected in HeLa cells, and GFP transfection efficiency is detected by flow cytometry 3 days after co transfection. After 14 days of transfection, the stable integration expression efficiency of GFP was detected by flow cytometry. 8C displays the ratio of stable integration efficiency to transfection efficiency.

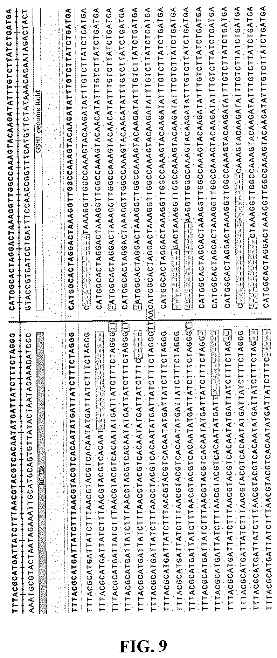

FIG. 9 shows partial sequencing of the Cas9-PBase-gRNA mediated transposon DNA specific RE-ITR sequence at the junction with the site integrated GSH1 site. After co transfection of Cas9-PBase(N347A_R376ED450N)-GSH1gRNA plasmid and NeoR-GFP transposon into HeLa cells, GFP positive cells were sorted by flow cytometry for 15 days, and single-cell clones were cultured and amplified to extract genomic DNA. And PCR amplification was performed by targeting specific primer combinations to screen for monoclonal cells with specific integration of the GSH1 site. Sequence the PCR amplified bands and compare them with the theoretical sequence. Compared with theoretical sequences, ITR sequences are disrupted in some clones, and most clones contain a small number of base insertions or deletions.

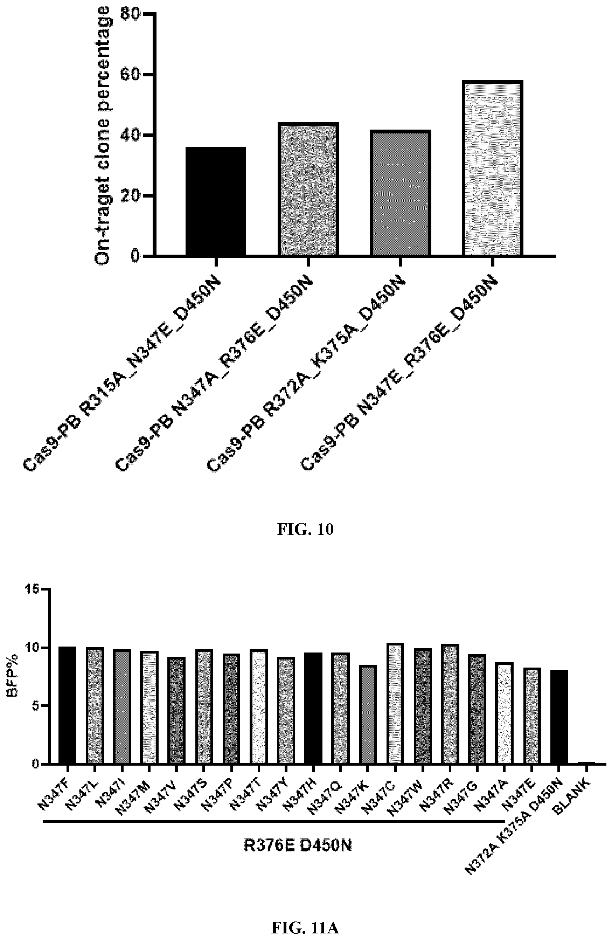

FIG. 10 shows the percentage of cell clones with different Cas9-PB mutants that integrate transposons at the GSH1 site. After co transfection of Cas9-PBase-GSH1gRNA plasmid and NeoR-GFP transposon into HeLa cells, GFP positive cells were sorted by flow cytometry for 15 days, and single-cell clones were cultured and amplified to extract genomic DNA. PCR amplification was performed by integrating specific primer combinations at specific sites, and monoclonal cells with GSH1 site specific integration were identified and screened based on the PCR results. Calculate the site integration ratio of each Cas9-PB mutant based on the ratio of the number of GSH1 site integrated clones to the total number of clones (R315E_N347E_D450N is 29/80; N347A_R376E_D450N is 38/86; R372A_K375A_D450N is 40/96; N347E_R376E_D450N is 56/96).
FIGs. 11A-11C show the integration efficiency of Cas9-PBase three mutants with different N347 mutation forms and R376E_D450N combinations in specific target regions. 11A displays the co transfection efficiency (mTagBFP2 positivity rate) of different Cas9-PBase three mutants and transposon DNA (as shown in FIG. 3B). 11B demonstrates the efficiency of targeted integration (2 * GFP percentage). The ratio of target integration efficiency to transfection efficiency is displayed at 11C.

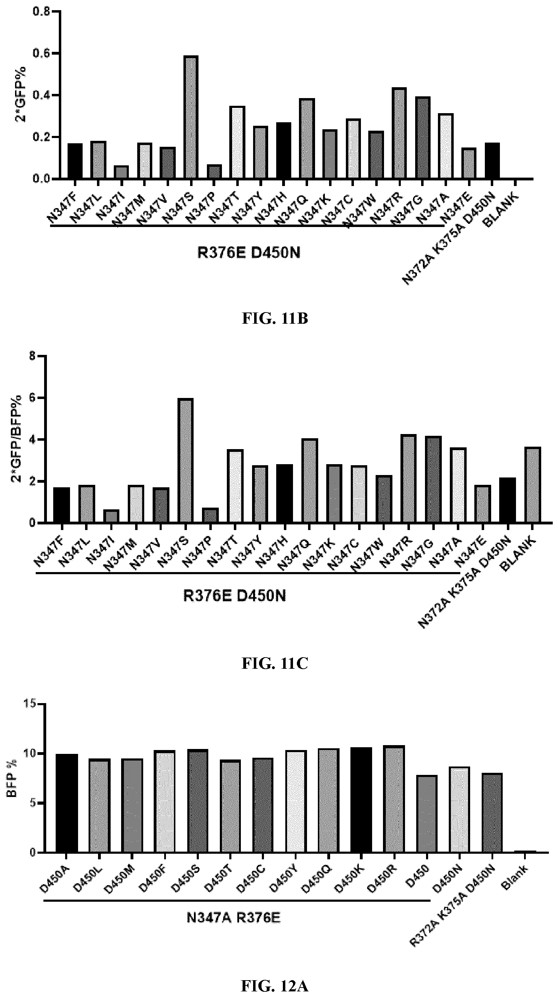

FIGs. 12A-12C show the integration efficiency of Cas9-PBase three mutants with different D450 mutation forms and N347A_R376E combinations in specific target regions. 12A displays the co transfection efficiency (mTagBFP2 positivity rate) of different Cas9-PBase three mutants and transposon DNA (as shown in FIG. 3B). 12B demonstrates the efficiency of targeted integration (2 * GFP percentage). 12C displays the ratio of in target integration efficiency to transfection efficiency.

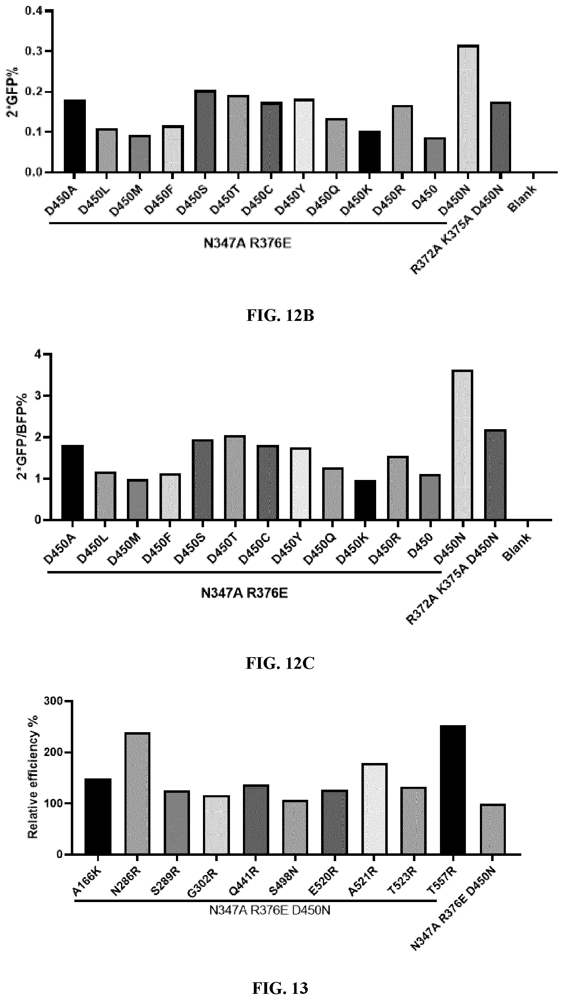

FIG. 13 shows the site directed integration efficiency of Cas9-PBase N347A_R376E_D450N_MutX mutants. The site-specific integration efficiency of different Cas9-PBase N347A_R376E_D450N_MutX mutants has been improved to varying degrees compared to Cas9-PBase N347A_R376E_D450N.

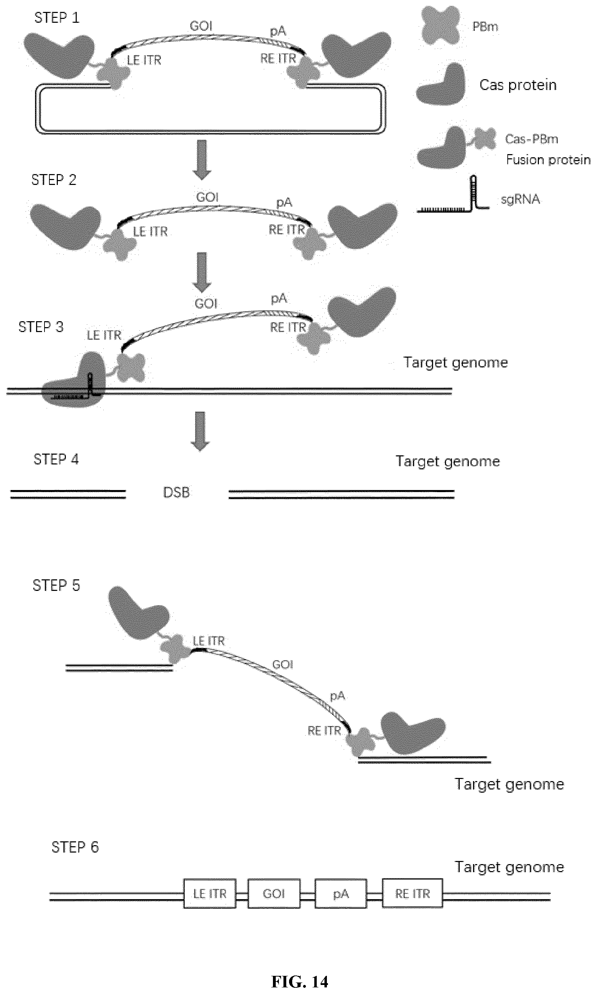

FIG. 14 is a schematic diagram of the site integration mechanism of transposon DNA in the Cas9-PB system. Step 1 involves the PBase portion of the fusion protein cleaving the Piggybac transposon containing the target gene (GOI) from the donor. Steps 2 and 3 involve the fusion protein pulling the target gene to the target site on the target genome through the targeting action of Cas9 and sgRNA. Step 4 involves the Cas9 in the fusion protein cleaving the target site to produce double strand breaks (DSBs). Step 5 involves the PBase portion of the fusion protein integrating the target gene to the double strand break at the target site. Step 6 involves integrating the target genome with the target gene.

### Detailed description

The present disclosure will be further illustrated through examples, but this does not limit the present disclosure to the scope of the embodiments described. The experimental methods without specific conditions specified in the following examples shall be selected according to conventional methods and conditions, or according to the product manual.

The reagents, instruments, consumables, and sequence information used in the embodiments of the present disclosure are shown in Table 2-4.

**Table 2: Reagent names, suppliers, and item numbers used**

| **Name** | **Supplier** | **Cat. No.** |
|---|---|---|
| 2 × Taq Master Mix (Dye Plus) | Vazyme | P112-01 |
| 2 × Phanta Max Master Mix (Dye Plus) | Vazyme | P525-01 |
| T4 DNA Ligase | Vazyme | C301-01 |
| Agarose Gel | Sangon Biotech | A620014 |
| HindIII | New England Biolabs | R0104S |
| BamHI | New England Biolabs | R0136S |
| AscI | New England Biolabs | R0558S |
| AgeI-HF | New England Biolabs | R3552S |
| pcDNA3.1 Vector | ShangHai GECELL Biotechnology Co., Ltd | / |
| Transposon BFP NGFP (plasmid) | ShangHai GECELL Biotechnology Co., Ltd | / |
| Primer | BioSune | / |
| LB Broth Medium | Sangon Biotech | A507002-0250 |
| Agar powder | Sangon Biotech | A100637 |
| 50X TAE Buffer | Sangon Biotech | B548101 |
| Ordinary Agarose Gel DNA Recovery Kit | TIANGEN^{®} | DP209 |
| Non Endotoxin Plasmid Small | TIANGEN^{®} | DP118-02 |
| Extraction Medium Dose Test Kit | | |
| Lipofectamine^{™} 3000 Transfection Reagent | Thermo | L3000008 |
| DMEM High Sugar Medium | Biosharp | BL301A |
| Fetal Bovine Serum | Yeasen | 40130ES76 |
| Penicillin -Streptomycin | Gibco | 15140-122 |
| HeLa Cell | ShangHai GECELL Biotechnology Co., Ltd | / |
| 293F Cell | ShangHai GECELL Biotechnology Co., Ltd | / |
| Opti-MEM^{™} Serum Reducing Culture Medium | Thermo | 51985034 |
| Puromycin Dihydrochloride | Thermo | A1113803 |
| GENETICIN | Gibco | 10131-027 |
| DH5a Competent Cell (CB101) | TIANGEN^{®} | CB101-02 |
| 0.25% Trypsin-EDTA (1X) | Thermo | 25200-056 |
| Cas9 Protein | GenScript | Z03469-500 |

**Table 3: Consumables, Instrument Suppliers, and Item Numbers Used**

| **Name** | **Supplier** | **Cat. No.** |
|---|---|---|
| 1.5ml centrifuge tube | Axygen | MCT-150-C |
| 15ml centrifuge tube | Themo | 339650 |
| 50ml centrifuge tube | Themo | 339652 |
| 0.2ml flat top PCR tube | Axygen | PCR-02-C |
| Electrophoresis Apparatus | Bio-Rad | 1645050 |
| Gel Imager | Shanghai Qinxiang Scientific Instrument Co., Ltd | 185068-8B |
| PCR Instrument | BIO-RAD | 621BR44541 |
| 5mL pipet | Themo | 170355N |
| 10mL pipet | Themo | 170356N |
| 25mL pipet | Themo | 170357N |
| Biosafety Cabinet | Thermo | 1374 |
| Inverted Fluorescence Microscope | Olympus | BX53 |
| Table | SHTIPS | TIPSOS-019 |
| Cell Culture Incubator | Themo | Forma 371 |
| 6cm dish | Themo | 150462 |
| 10cm dish | Themo | 150466 |
| 6-hole plate | Themo | 140675 |
| 12-hole plate | Themo | 150628 |
| NC250 Counter | Chemometec | NC-250 |
| Electro-Thermostatic Water Bath | Shanghai Jinghong Experimental Equipment Co., Ltd | DK-S22 |
| Balance | Sartorius | GL822I-1SCN |
| Ultra Micro Spectrophotometer | Themo | AZY1810420 |
| Attune NxT Flow Cytometer | Themo | A24860 |
| Electric Pipette | Themo | S1-9501 |
| Centrifuge | Themo | ST16R |

**Table 4: Primers and sequences involved in the construction of relevant molecules**

| Name | Sequence | SEQ ID NO: |
|---|---|---|
| AscI-up-Cas-PF N1 | CACTACCTTGATGAGATCATC | 22 |
| AgeI-down-pA-PR N1 | TGAACAAACGACCCAACACC | 23 |
| Cas9-AscI-PF | TGTTTACTCTGACCAACTTGGGCGCGCCTGCAGCCTTCAAG | 24 |
| AgeI-PB-PR | TTACTAACCGGTAGGGATCGAACCCTTTCAGAAACAGCTCTGGCACA TGTC | 25 |
| pcDNA3.1-PR-N1 | CCACTGTGCTGGATATCTGCAGAATTCC | 26 |
| CMV-PF-N1 | CTCTCTGGCTAACTAGAGAACC | 27 |
| HindIII-PB-PF | AAACTTAAGCTTGCCACCATGGGCTCTAGCCTGGACGAC | 28 |
| BamHI-PB-PR | ACTAGTGGATCCTCAGAAACAGCTCTGGC | 29 |
| A166K PF | GAGCATGACCAGCAAGACCTTCAGAGACACCAACG | 30 |
| A166K PR | GTCTCTGAAGGTCTTGCTGGTCATGCTCTCCCGCC | 31 |
| N286R PF | GTGTACATCCCCAGAAAGCCCAGCAAGTACGGCATC | 32 |
| N286R PR | CTTGCTGGGCTTTCTGGGGATGTACACTCTGAAGG | 33 |
| S289R PF | CCCAACAAGCCCAGAAAGTACGGCATCAAGATCCTG | 34 |
| S289R PR | GATGCCGTACTTTCTGGGCTTGTTGGGGATGTACAC | 35 |
| G302R PF | GATGTGCGACAGCagaACCAAGTACATGATCAACGGC | 36 |
| G302R PR | CATGTACTTGGTtctGCTGTCGCACATCATCAGGATC | 37 |
| Q441R PF | GATGTACTACAACagaACCAAGGGCGGCGTGGACACC | 38 |
| Q441R PR | GCCGCCCTTGGTtctGTTGTAGTACATCACCATCTG | 39 |
| S498N PF | GAGAAGGTGCAGAACCGGAAGAAATTCATGCGGAAC | 40 |
| S498N PR | GAATTTCTTCCGGTTCTGCACCTTCTCGCCCTTGC | 41 |
| E520R PF | GAGAAAGAGACTGagaGCCCCCACCCTGAAGAGATAC | 42 |
| E520R PR | CAGGGTGGGGGCtctCAGTCTCTTTCTCATGAAGCTG | 43 |
| A521R PF | GAAAGAGACTGGAAAGACCCACCCTGAAGAGATACC | 44 |
| A521R PR | CTTCAGGGTGGGTCTTTCCAGTCTCTTTCTCATGAAG | 45 |
| T523R PF | CTGGAAGCCCCCAGACTGAAGAGATACCTGCGGGAC | 46 |
| T523R PR | GTATCTCTTCAGTCTGGGGGCTTCCAGTCTCTTTC | 47 |
| T557R PF | GATGAAGAAGAGGAGATACTGCACCTACTGTCCCAG | 48 |
| T557R PR | GTAGGTGCAGTATCTCCTCTTCTTCATCACGGGTTC | 49 |
| N347A PF | GCAGGAACATCACCTGCGACgcCTGGTTCACCAGCATCCCCCTG | 50 |
| N347A PR | CCAGgcGTCGCAGGTGATGTTCCTGCAGC | 51 |
| R376E PF | AACAAGgagGAGATCCCAGAGGTGCTGAAG | 52 |
| R376E PR | GCACCTCTGGGATCTCctcCTTGTTGCTCCGCACGGTGCCCAC | 53 |
| D450N PF | CGTGGACACCCTGaACCAGATGTGCAGCGTGATGAC | 54 |
| D450N PR | GCACATCTGGTtCAGGGTGTCCACGCCGCCCTTG | 55 |
| R372A PF | GTGGGCACCGTGgccAGCAACAAGCGGGAGATCCCAG | 56 |
| R372A PR | GCTTGTTGCTggcCACGGTGCCCACGATGGTCAG | 57 |
| K375A PF | GGAGCAACgccCGGGAGATCCCAGAGGTGCTG | 58 |
| K375A PR | TCTGGGATCTCCCGggcGTTGCTCCGCACGGTGCCCACGATG | 59 |
| N347E PF | GCAGGAACATCACCTGCGACgagTGGTTCACCAGCATCCCCCTG | 60 |
| N347E PR | CCActcGTCGCAGGTGATGTTCCTGCAGCTGCCATGCAC | 61 |
| R315A-PF | TGCCCTACCTGGGCgccGGCACCCAGACAAACGGCGTG | 62 |
| R315A-PR | TGGGTGCCggcGCCCAGGTAGGGCATGCCGTTG | 63 |
| Y312E-PF | ACGGCATGCCCgagCTGGGCAGAGGCACCCAGAC | 64 |
| Y312E-PR | CCTCTGCCCAGetcGGGCATGCCGTTGATCATGTAC | 65 |
| R315E-PF | TGCCCTACCTGGGCgagGGCACCCAGACAAACGGCGTG | 66 |
| R315E-PR | TGGGTGCCctcGCCCAGGTAGGGCATGCCGTTG | 67 |
| L324E-PF | GGCGTGCCCgagGGCGAGTACTACGTGAAAGAACTG | 68 |
| L324E-PR | CGTAGTACTCGCCctcGGGCACGCCGTTTGTCTGGGTG | 69 |
| N347F-PF | CATCACCTGCGACttcTGGTTCACCAGCATCCCCCTG | 70 |
| N347F-PR | GCTGGTGAACCAgaaGTCGCAGGTGATGTTCCTGC | 71 |
| N347L-PF | CATCACCTGCGACctgTGGTTCACCAGCATCCCCCTG | 72 |
| N347L-PR | GCTGGTGAACCAcagGTCGCAGGTGATGTTCCTGC | 73 |
| N3471-PF | CATCACCTGCGACatcTGGTTCACCAGCATCCCCCTG | 74 |
| N3471-PR | GCTGGTGAACCAgatGTCGCAGGTGATGTTCCTGC | 75 |
| N347M-PF | CATCACCTGCGACatgTGGTTCACCAGCATCCCCCTG | 76 |
| N347M-PR | GCTGGTGAACCAcatGTCGCAGGTGATGTTCCTGC | 77 |
| N347V-PF | CATCACCTGCGACgtgTGGTTCACCAGCATCCCCCTG | 78 |
| N347V-PR | GCTGGTGAACCAcacGTCGCAGGTGATGTTCCTGC | 79 |
| N347S-PF | CATCACCTGCGACagcTGGTTCACCAGCATCCCCCTG | 80 |
| N347S-PR | GCTGGTGAACCAgctGTCGCAGGTGATGTTCCTGC | 81 |
| N347P-PF | CATCACCTGCGACcccTGGTTCACCAGCATCCCCCTG | 82 |
| N347P-PR | GCTGGTGAACCAgggGTCGCAGGTGATGTTCCTGC | 83 |
| N347T-PF | CATCACCTGCGACaccTGGTTCACCAGCATCCCCCTG | 84 |
| N347T-PR | GCTGGTGAACCAggtGTCGCAGGTGATGTTCCTGC | 85 |
| N347Y-PF | CATCACCTGCGACtacTGGTTCACCAGCATCCCCCTG | 86 |
| N347Y-PR | GCTGGTGAACCAgtaGTCGCAGGTGATGTTCCTGC | 87 |
| N347H-PF | CATCACCTGCGACcacTGGTTCACCAGCATCCCCCTG | 88 |
| N347H-PR | GCTGGTGAACCAgtgGTCGCAGGTGATGTTCCTGC | 89 |
| N347Q-PF | CATCACCTGCGACcagTGGTTCACCAGCATCCCCCTG | 90 |
| N347Q-PR | GCTGGTGAACCActgGTCGCAGGTGATGTTCCTGC | 91 |
| N347K-PF | CATCACCTGCGACaagTGGTTCACCAGCATCCCCCTG | 92 |
| N347K-PR | GCTGGTGAACCActtGTCGCAGGTGATGTTCCTGC | 93 |
| N347D-PF | CATCACCTGCGACgacTGGTTCACCAGCATCCCCCTG | 94 |
| N347D-PR | GCTGGTGAACCAgtcGTCGCAGGTGATGTTCCTGC | 95 |
| N347C-PF | CATCACCTGCGACtgcTGGTTCACCAGCATCCCCCTG | 96 |
| N347C-PR | GCTGGTGAACCAgcaGTCGCAGGTGATGTTCCTGC | 97 |
| N347W-PF | CATCACCTGCGACtggTGGTTCACCAGCATCCCCCTG | 98 |
| N347W-PR | GCTGGTGAACCAccaGTCGCAGGTGATGTTCCTGC | 99 |
| N347R-PF | CATCACCTGCGACagaTGGTTCACCAGCATCCCCCTG | 100 |
| N347R-PR | GCTGGTGAACCAtctGTCGCAGGTGATGTTCCTGC | 101 |
| N347G-PF | CATCACCTGCGACggcTGGTTCACCAGCATCCCCCTG | 102 |
| N347G-PR | GCTGGTGAACCAgccGTCGCAGGTGATGTTCCTGC | 103 |
| D450A-PF | CGTGGACACCCTGgccCAGATGTGCAGCGTGATGAC | 104 |
| D450A-PR | GCACATCTGggcCAGGGTGTCCACGCCGCCCTTG | 105 |
| D450L-PF | CGTGGACACCCTGetgCAGATGTGCAGCGTGATGAC | 106 |
| D450L-PR | GCACATCTGcagCAGGGTGTCCACGCCGCCCTTG | 107 |
| D450M-PF | CGTGGACACCCTGatgCAGATGTGCAGCGTGATGAC | 108 |
| D450M-PR | GCACATCTGcatCAGGGTGTCCACGCCGCCCTTG | 109 |
| D450F-PF | CGTGGACACCCTGttcCAGATGTGCAGCGTGATGAC | 110 |
| D450F-PR | GCACATCTGgaaCAGGGTGTCCACGCCGCCCTTG | 111 |
| D450S-PF | CGTGGACACCCTGagcCAGATGTGCAGCGTGATGAC | 112 |
| D450S-PR | GCACATCTGgctCAGGGTGTCCACGCCGCCCTTG | 113 |
| D450T-PF | CGTGGACACCCTGaccCAGATGTGCAGCGTGATGAC | 114 |
| D450T-PR | GCACATCTGggtCAGGGTGTCCACGCCGCCCTTG | 115 |
| D450C-PF | CGTGGACACCCTGtgcCAGATGTGCAGCGTGATGAC | 116 |
| D450C-PR | GCACATCTGgcaCAGGGTGTCCACGCCGCCCTTG | 117 |
| D450Y-PF | CGTGGACACCCTGtacCAGATGTGCAGCGTGATGAC | 118 |
| D450Y-PR | GCACATCTGgtaCAGGGTGTCCACGCCGCCCTTG | 119 |
| D450Q-PF | CGTGGACACCCTGcagCAGATGTGCAGCGTGATGAC | 120 |
| D450Q-PR | GCACATCTGctgCAGGGTGTCCACGCCGCCCTTG | 121 |
| D450K-PF | CGTGGACACCCTGaagCAGATGTGCAGCGTGATGAC | 122 |
| D450K-PR | GCACATCTGcttCAGGGTGTCCACGCCGCCCTTG | 123 |
| D450R-PF | CGTGGACACCCTGagaCAGATGTGCAGCGTGATGAC | 124 |
| D450R-PR | GCACATCTGtctCAGGGTGTCCACGCCGCCCTTG | 125 |
| GSH1 genome-PF | CAGAACACAGAAGTGCAGTC | 126 |
| GSH1 genome-PR | GATGCACTTCAGGATATGAG | 127 |
| NeoR-GFP donor-PF | ATGTCCTAAATGCACAGCGAC | 128 |
| NeoR-GFP donor-PR | AACCTCGATATACAGACCGA | 129 |

The molecular construction method used in the present disclosure is as follows:

### Molecular construction method 1

1) Firstly, the wt Hyperactive PBase sequence was optimized according to the human codon and synthesized by Sangon Biotech (see SEQ ID NO: 138 for the wt Hyperactive PBase sequence). After synthesis, HindIII and BamHI were used to subclone into the multi clone cleavage site of the conventional expression vector pcDNA3.1, resulting in the pcDNA3.1-wt PB plasmid. Using this as a template, primers CMV-PF-N1/PBm PR for amplifying the upstream fragment of PB mutant (PBm), primers pcDNA3.1-PR-N1/PBm PF for downstream fragments, and primers HindIII-PB-PFBamH I-PB-PR for fusing upstream and downstream fragments were designed using Snapgene 3.2.1 software. The primers were synthesized by Biosune Biotechnology (Shanghai) Co., Ltd;
2) Use a PCR instrument to amplify the upstream and downstream fragments of PBm. The reaction system is 50 µL: 2 × Phanta Max Master Mix (Dye Plus) 25 µL, ddH₂O 20 µL, 10 µg/µL plasmid template 1.0 µL, upstream fragment primer CMV-PF-N1/PBm PR, downstream fragment primer pcDNA3.1-PR-N1/PBm PF 2 µL each. PCR reaction conditions: pre denature at 95 °C for 2 minutes; Denaturation at 95 °C for 20 seconds, annealing at 58 °C for 30 seconds, extension at 72 °C for 1 minute, 30 cycles; Extend at 72 °C for another 5 minutes;
3) Ordinary agarose gel DNA recovery kit (Tiangen Biochemical Technology Co., Ltd.) purified and recovered PBm upstream and downstream fragments;
4) Amplification of full-length PBm fragments: Using purified and recovered PBm upstream and downstream fragments as templates, primers were HindIII-PB PF/BamH I-PB-PR. 50 µL reaction system: 2 × Phanta Max Master Mix (Dye Plus) 25 µL, ddH₂O 20 µL, 10 µg/µL PBm upstream and downstream fragments each 0.5 µL, forward and reverse primers each 2 µL. PCR reaction conditions: pre denature at 95 °C for 2 minutes; Denaturation at 95 °C for 20 seconds, annealing at 58 °C for 30 seconds, extension at 72 °C for 1.5 minutes, 30 cycles; Extend at 72 °C for another 5 minutes;
5) Ordinary agarose gel DNA recovery kit (Tiangen Biochemical Technology Co., Ltd.) purified and recovered PBm full-length fragments;
6) After recovery and purification of the PCR product of the full-length PBm fragment, the PBm fragment was digested using restriction enzymes Hind III-HF and BamH I-HF. The reaction system consisted of 0.2 µg of PBm fragment, 1 µL of restriction enzymes Hind III-HF and 1 µL of BamH I-HF, and 5 µL of 10 × rCutSmart buffer. Water was added to 50 µL. React at 37 °C for 30 minutes; The plasmid pcDNA3.1 was digested using the same enzyme as the vector. The reaction system contained 2 µg of plasmid, 1 µL of restriction endonucleases Hind III-HF and BamH I-HF, and 5 µL of 10 × rCutSmart buffer. Water was added to 50 µL. React at 37 °C for 1 hour;
7) Ordinary agarose gel DNA recovery kit (Tiangen Biochemical Technology Co., Ltd.) is used to purify and recover the full-length fragment of PBm digested by enzyme and plasmid vector;
8) Enzyme cut full-length PBm fragments were used as plasmid vectors using T4DNA Ligase purchased from Novozymes. The reaction system was described in the T4DNA Ligase manual, and the ligation reaction was carried out under reaction conditions of 25 °C for 30 minutes;
9) Take 5 µL of the connecting product and gently add it to 50 µL of DH5α clonal competent cells. Gently mix and shake well, then ice bath for 30 minutes; Immediately place on ice for 2 minutes after being subjected to heat shock in a 42 °C water bath for 30 seconds; Add 200 µL of LB free medium equilibrated to room temperature. Shake at 37 °C and 180rpm for 1 hour; evenly apply 200 µL of bacterial solution onto a solid LB plate (containing antibiotic: ampicillin with a working concentration of 50 µg/ml); Invert the LB plate and incubate overnight at 37 °C in the incubator;
10) Select positive clones and send them to Biosune Biotechnology (Shanghai) Co., Ltd. for sequencing;
11) The correctly sequenced bacterial solution was expanded and used to extract pcDNA3.1-PBm using the endotoxin free plasmid small extraction medium dose kit from Tiangen Biochemical Technology Co., Ltd. After extraction, the concentration and quality of the plasmid were detected using an ultra micro spectrophotometer, and the pcDNA3.1-PBm plasmid was cleaved using the endonucleases Hind III and BamH I-HF to verify its size.

### Molecular Construction Method 2

1) First, the D10A of the hCas9-D10A plasmid (addgene # 41816) was mutated in situ to D, and then the U6-ROSA sgRNA (SEQ ID NO: 131) element (synthesized by GenScript) was synthesized and inserted into the MfeI and MluI sites of the vector; On this basis, the linker wt Hyperactive PBase sequence (synthesized by GenScript) was further synthesized at the C-terminus of the Cas9 coding sequence to obtain the Cas9-PB-ROSA sgRNA vector (SEQ ID NO: 139). Subsequent mutations were performed using this plasmid as a template, and Snapgene 3.2.1 software was used to design primers AscI-up-Cas-PF-N1/PBm PR for amplifying upstream fragments of PB mutants (PBm), AgeI-down-pA-PR N1/PBm PF for downstream fragments, and Cas9-AscI-PF/AgeI-PB-PR for fusing upstream and downstream fragments. The primers were synthesized by Biosune Biotechnology (Shanghai) Co., Ltd;
2) Use a PCR instrument to amplify the upstream and downstream fragments of PBm. The reaction system is 50 µL: 2 × Phanta Max Master Mix (Dye Plus) 25 µL, ddH₂O 20 µL, 10 µg/µL plasmid template 1.0 µL, upstream fragment primer AscI-up-Cas-PF N1/PBm PR, downstream fragment primer AgeI-down-pA-PR N1/PBm PF 2 µL each. PCR reaction conditions: pre denature at 95 °C for 2 minutes; Denaturation at 95 °C for 20 seconds, annealing at 58 °C for 30 seconds, extension at 72 °C for 1 minute, 30 cycles; Extend at 72 °C for another 5 minutes;
3) Ordinary agarose gel DNA recovery kit (Tiangen Biochemical Technology Co., Ltd.) purified and recovered PBm upstream and downstream fragments;
4) Amplification of full-length PBm fragments: Using purified and recovered PBm upstream and downstream fragments as templates, primers were Cas9-AscI-PF/AgeI-PB-PR. 50 µL reaction system: 2 × Phanta Max Master Mix (Dye Plus) 25 µL, ddH₂O 20 µL, 10 µg/µL PBm upstream and downstream fragments each 0.5 µL, forward and reverse primers each 2 µL. PCR reaction conditions: pre denature at 95 °C for 2 minutes; Denaturation at 95 °C for 20 seconds, annealing at 58 °C for 30 seconds, extension at 72 °C for 2 minutes, 30 cycles; Extend at 72 °C for another 5 minutes;
5) Ordinary agarose gel DNA recovery kit (Tiangen Biochemical Technology Co., Ltd.) purified and recovered PBm full-length fragment;
6) After recovery and purification of the PCR product of the full-length PBm fragment, the PBm fragment was digested using restriction enzymes AscI and AgeI-HF. The reaction system consisted of 0.2 µg PBm fragment, 1 µL each of restriction enzymes AscI and Agel-HF, 10 × rCutSmart buffer 5 µL, and water was added to 50 µL. React at 37 °C for 30 minutes; The plasmid pcDNA3.1-Cas9-PB-N347A R376E D450N was digested using the same enzyme as the vector. The reaction system contained 2 µg of plasmid, 1 µL of restriction endonucleases AscI and AgeI-HF, and 5 µL of 10 × rCutSmart buffer. Water was added to 50 µL. React at 37 °C for 1 hour;
7) Ordinary agarose gel DNA recovery kit (Tiangen Biochemical Technology Co., Ltd.) is used to purify and recover the PBm full-length fragment digested by enzyme and plasmid vector;
8) Enzyme cut full-length PBm fragment, plasmid vector using T4DNA Ligase purchased from Novozymes, reaction system according to T4DNA Ligase instruction manual, reaction at 25 °C for 30 minutes;
9) Take 5 µL of the connecting product and add it to 50 µL of Escherichia coli DH5α competent cells. Take 5 µL of the connecting product and gently add it to 50 µL of DH5α clonal competent cells. Gently mix and shake well, then ice bath for 30 minutes; Immediately place on ice for 2 minutes after being subjected to heat shock in a 42 °C water bath for 30 seconds; Add 200 µL of LB medium equilibrated to room temperature. Shake at 37 °C and 180rpm for 1 hour; evenly apply 200 µL of bacterial solution onto a solid LB plate (containing antibiotic: ampicillin with a working concentration of 50 µg/ml); Invert the LB plate and incubate overnight at 37 °C in the incubator;
10) Select positive clones and send them to Biosune Biotechnology (Shanghai) Co., Ltd. for sequencing;
11) After amplification, Cas9-PBm was extracted from the correctly sequenced bacterial solution using the endotoxin free plasmid small extraction medium dose kit from Tiangen Biochemical Technology Co., Ltd. After extraction, the concentration and quality of the plasmid were detected using an ultra micro spectrophotometer, and the Cas9-PBm plasmid was cleaved using the endonucleases AscI and AgeI-HF to verify its size.

### Example 1 Detection of PBase Mutant Shear Transposon Donor Activity

The purpose of this experiment is to test the efficiency of different PBase mutants in cutting transposon donors. Firstly, a cell line with tdTomato reporter gene was constructed on 293F cells, and then PBase mutant plasmids were transfected into the cell line. The efficiency of PBase mutant transposon donor was determined by observing the expression of tdTomato gene.

Firstly, a homologous recombination donor at the AAVS1 site was synthesized (FIG. 1A, synthesized by GenScript, sequence as shown in SEQ ID NO: 15), which contains a homologous recombination arm integrated at the AAVS1 site and can be site directed recombined into the intron of the PPP1R12C gene (located on chromosome 19). The donor contains mRNA splicing acceptors (SA) and T2A-Puro, which can be expressed by the endogenous PPP1R12C promoter. At the same time, the expression of tdTomato driven by the CAG promoter on the donor is blocked by an intermediate transposon (expressing BSD). After the transposon is removed by PBase or its mutants, tdTomato will be expressed.

AAVS1sgRNA (targeting sequence CACCCCACAGTGGCCACT, SEQ ID NO: 130) was synthesized by GenScript, and Cas9 protein (GenScript, Z03469-500) was purchased. Linear donors were amplified and purified using high fidelity DNA polymerase (Vazyme, P525-01). Transfer RNP and linear donor to 293F cells using NeonTM electroporation. The experimental process involves mixing 150pmol Cas9 protein and 300pmol sgRNA and incubating at room temperature for 10 minutes to obtain RNP (ribonucleoprotein complex); Simultaneously add 5E6 cells and 1.0 µg linear donor DNA to a 100 µL electroporation system, and then add RNP to the electroporation system. The electrical conversion condition is 1100V, 20ms/pulse * 2pulse. Use Puromycin (1 µg/mL) to screen for positive single-cell clones and determine the reporter gene site knock in AAVS1 site through Sanger sequencing.

The amino acid sequences of PBase mutants PBase R315A_N347E_D450N, PBase N347E_R376E_D450N, PBase N3 47A_R376E_D450N, and PBase R372A_K375A_D450N are shown in SEQ ID NO: 5, 7, 9, and 11. The amino acid sequence of wild-type PBase (wt highly active PBase) is shown in SEQ ID NO:3, and the amino acid sequence of PBase R315A_N347E is the amino acid sequence replaced by R315A and N347E based on the amino acid sequence shown in SEQ ID NO:3

The construction of PBase mutants was achieved by introducing corresponding mutations through overlap PCR, using the synthesized pcDNA3.1 PBase as a template, and cloning it into the pcDNA3.1 expression vector using HindIII and BamHI. The specific experimental details can be found in Molecular Construction Method 1. The coding nucleic acid sequence of wt Hyperactive PBase is shown in SEQ ID NO: 13, and the coding nucleic acid sequences of each PBase mutant are shown in 4183-5964 positions of SEQ ID NO: 135-137, with the addition of ATG nucleotide sequence at the 5 'end (PBases all contain the M encoded by ATG, but the ATG of PBase is removed from the coding sequence of the fusion protein). Transfect 1 µg of pcDNA3.1-PBase mutant plasmid into the aforementioned tdTomato reporter cells using lipofectamine 3000 reagent, and count the number of tdTomato positive cells 5 days after transfection. The number of positive cells represents the cutting activity of the corresponding PBase mutant towards transposon donors.

Results and Conclusion: Different PBase mutants showed varying degrees of decreased cleavage activity compared to wild-type PBase. In particular, R315A_N347E, R315A_N347E_D450N, R315E_N347E_D450N, and N347A_R376E_D450N all have higher shear activity than R372A_K375A_D450N (this mutant can be found in the articles piggyBac transportase tools for genome engineering and Find and cut and transfer (FiCAT) mammalian genome engineering); Y312E_N347E_D450N and N347E_R376E_D450N also exhibit certain shear activity; In addition, the three mutations containing D450N exhibited higher cleavage activity than the corresponding double mutants. (FIG. 1B)

### Example 2: Detection of PBase Mutant's Activity in Integrating Transposon Donor into Genome (TTAA Dependent)

The purpose of this experiment is to test the activity of different PBase mutant splicing transposon donors and integrating them into the "TTAA" site of the genome. The experiment first constructed transposon donors with dual promoters expressing NeoR resistance gene and enhanced green fluorescent protein eGFP, respectively (FIG. 7). Then, 1 µg NeoR GFP transposon donor (sequence as shown in SEQ ID NO: 21) and 0.7 µg pcDNA3.1-PBase mutant from Example 1 were co transfected. The GFP positivity rate, transfection efficiency, and stable integration efficiency were detected by flow cytometry at 2 and 14 days after transfection, respectively. And calculate the integration activity of different PBase mutants based on the ratio of GFP on the 14th day to GFP on the 2nd day.

Results and Conclusion: The overall integration activity of the double mutant and triple mutant is at a low level, and the vast majority of mutants have almost no integration activity; Partial mutants include Y312E_N347E_D450N, R315A_N347E_D450N, R315E_N347E_D450N, and N347A_R376E_D450N, which have integration activity similar to or slightly higher than R372A_K375A_D450N. (FIG. 2)

Taking into account the activities of different PBase mutants in cutting transposon donors (Example 1) and integrating transposon donors into the genome (TTAA target) (Example 2), we selected some mutants with higher cutting activity and lower integration activity (referred to as Exc+Int- mutants), mainly including R315A_N347E_D450N, N347A_R376E_D450N, N347A_R376E_D450N, and the R372A_K375A_D450N mutant in the existing technology, and combined them with CRISPR-Cas9 protein that can generate site directed DNA double strand breaks to test the efficiency of site directed integration of Piggybac transposon donors carrying exogenous target genes.

### Example 3 Construction of Cas9-PBase Mutant and Testing of Site directed Integration Efficiency

The purpose of this experiment is to fuse the above-mentioned Exc+Int- mutants with the Cas9 gene to construct Cas9-PBase mutants, and to test the site integration efficiency of different Cas9-PBase mutants by designing and constructing a Split GFP testing system. The functional principle of Cas9-PBase fusion protein is shown in FIG. 14.

Cas9-PBase mutant construction: First, the D10A of hCas9_D10A plasmid (addgene # 41816) was mutated in situ to D, and then U6-ROSA sgRNA (SEQ ID NO: 131) element (synthesized by GenScript) was synthesized and inserted into the MfeI and MluI sites of the vector; On this basis, 3 * G4S linker wt Hyperactive PBase was further synthesized and connected to the C-terminus of Cas9 (sequence as shown in SEQ ID NO: 1), resulting in the Cas9-PB-ROSA sgRNA vector (SEQ ID NO: 139). On this basis, the four different PBase mutants mentioned above were replaced with wt Hyperactive PBase and connected to the C-terminus of Cas9. For specific experimental details, please refer to Molecular Construction Method 2. The constructed Cas9-PB-ROSA sgRNA plasmid profile is shown in FIG. 3C (taking Cas9 wt PBase-ROSA sgRNA as an example). Considering that the first amino acid M (encoded by ATG) is usually cleaved during protein maturation, in all fusion proteins, the PBase portion lacks the first amino acid M compared to the free PBase, while retaining amino acids 2-594 (GSSLDD---QSCF). The portion of the coding nucleic acid sequence for PBase in Cas9-PBase of each mutant (as shown in site 4183-5964 on SEQ ID NO: 14 and 135-137 nucleic acid sequences, all underlined) corresponds to amino acids 2-594 of the PBase amino acid sequence. The obtained Cas9-PBase fusion protein Cas9-wt Hyperactive PBase,Cas9-PBase R315A_N347E_D450N,Cas9-PBase N347E_R376E_D450N,Cas9-PBase N347A_R376E_D450N The amino acid sequences of Cas9-PBase R372A_K375A_D450N and Cas9-PBase R372A_K375A_D450N are shown in SEQ ID NO: 4, 6, 8, 10, and 12, respectively.

Split GFP system: The schematic diagram of the working principle of this system is shown in FIG. 4. The basic principle is to place the N-terminal and C-terminal GFP coding sequences separately on the N GFP transposon donor plasmid (FIG. 3B, sequence shown as SEQ ID NO: 17) and the receiving plasmid (FIG. 3A, sequence shown as SEQ ID NO: 16), and to split the intron sequence between exon 7 and exon 8 of the endogenous SMN1 gene into two parts (1/2 intron and 2/2 intron) and place them downstream of N GFP (1/2 intron) and upstream of C GFP (2/2 intron), respectively. If the N GFP transposon donor can be integrated upstream of C GFP, the intron can be cleaved through mRNA splicing and GFP protein can be expressed. Add the target sequence (including specific sgRNA sequences such as ROSA sgRNA mentioned above), 2/2 intron sequence, and splicing receptor sequence upstream of C GFP. At the same time, add the BxblattB-BSD resistance gene to the plasmid, which can be integrated into the Bxb1attP site using Bxb1 integrase. The N GFP transposon donor contains continuously expressed BFP-2A-Puro for flow cytometry detection of transfection, integration efficiency, and screening. Additionally, splicing donor sequences and 1/2 intron sequences are added downstream of N GFP (FIG. 4). The specific principle can refer to doi: 10.1016/j.molcel.2018.10.037.

Split GFP cell line construction: First, the pPGK-Bxb1attP sequence was site directed recombined to the ROSA site in HeLa cells using Cas9 protein (GenScript Z03469-500) mediated homologous recombination, and single-cell cloning was performed. Sanger sequencing and NGS sequencing were used to determine the single copy sequence integration sequence (conventional homologous recombination method knock in, not repeated here). Then, the synthesized pcDNA3.1-Bxb1 integrase plasmid 1 µg (Bxb1 integrase sequence reference addgene # 127519) and receptor plasmid 1.5 µg (FIG. 3A) were co transfected, and single-cell clones were selected for targeted integration into the pPGK-Bxb1attP site using Blasticidin antibiotic (10 µg/mL). The accurate sequence was determined by Sanger sequencing.

Site directed integration efficiency test of Cas9-PBase mutant: The constructed Cas9-PBase mutant plasmids (1.3 µg, where Cas9-PBase N347A_R376E_D450N-ROSA gRNA plasmid sequence is shown in SEQ ID NO: 18) and N GFP transposon donors (1.2 µg, sequence is shown in SEQ ID NO: 17) were co transfected into Split GFP cell lines using lipofectamine 3000 according to standard operating procedures. Four days after transfection, BFP and GFP positivity rates were detected by flow cytometry (FIG. 5A) and data statistics were performed (FIG. 5B). Since BFP positivity rate represents transfection efficiency, GFP positivity rate represents site directed integration ratio, the GFP/BFP ratio can reflect this. Site integration efficiency of different Cas9-PBase mutants (FIG. 5C).

Results and Conclusion: Through fixed-point integration efficiency testing comparison, it was found that Cas9-PBase N347A_R376A_D450N has higher efficiency than Cas9-PBase R372A_K375A_D450N, while Cas9-PBase N347A_R376A_D450N and Cas9-PBase R315A_N347A_D450N have lower efficiency than Cas9-PBase R372A_K375A_D450N. Select Cas9-PBase N347A_R376ED450N as the basis, add new mutations and screen for mutants with improved efficiency.

### Example 4: Testing the on/off-target ratio of different Cas9-PBase mutants using Split GFP cell lines

The purpose of this experiment is to test the proportion of cells undergoing fixed-point integration among all stable integrated cells. The Cas9-PBase tool has two main sources of off target activity: on the one hand, Cas9 undergoes off target cleavage guided by sgRNA, and on the other hand, PBase mutants still have low "TTAA" integration activity. By detecting the positive rates of stable integration of BFP and on target integration of GFP in cells, the proportion of on target cells can be calculated.

The cells transfected in Example 3 were continuously cultured in normal HeLa cell culture medium (DMEM+10% FBS+P/S) for 12 days after transfection, and then screened and enriched with 1.0 µg/mL puromycin for 10 days. The proportion of BFP and GFP positive cells was detected by flow cytometry. The proportion of targeted integration is calculated based on 2 times the percentage of GFP positivity.

Results and Conclusion: After Puromycin screening and enrichment, the BFP positive rate reached over 90%, and the proportion of BFP and GFP double positive cells varied among different Cas9-PBase mutants (FIG. 6). The two proportions of each Cas9-PBase mutant are: Cas9-PBase N347A_R376E_D450N (97.5% and 20.3%), Cas9-PBase R372A_K375A_D450N (97.3% and 17.2%), Cas9-PBase N347E_R376E_DA450N (94.7% and 20.2%), and Cas9-PBase R315A_N347E_D450N (96.0% and 14.8%).

From this data, it can be concluded that the new Cas9-PBase fusion mutant has lower off target rates for Cas9-PBase N347A_R376E_D450N and Cas9-PBase N347E_R376E_D450N compared to Cas9-PBase R372A_K375A_D450N, and slightly higher off target rates for Cas9-PBase R315A_N347E_D450N than Cas9-PBase R372A_K375A_D450N.

Overall, the results of comparing these three mutant Cas-PB with Cas9-PBase R372A_K375A_D450N are as follows: In terms of specific effects, it was found that the fixed-point integration efficiency of Cas9-PBase N347A_R376A_D450N was significantly higher than that of Cas9-PBase R372A_K375A_D450N, and the off target probability of the former was slightly lower than that of the latter. The fixed-point integration efficiency of Cas9-PBase N347E_R376E_D450N is slightly lower than that of Cas9-PBase R372A_K375A_D450N, but the off target probability of the former is significantly reduced. In most cases, the fixed-point integration efficiency of Cas9-PBase R315A_N347A_D450N is comparable to that of Cas9-PBase R372A_K375A_D450N, but the former has a higher off target rate.

### Example 5: Site directed integration testing at the Genome safe harbor locus in HeLa cells

The purpose of this experiment is to further test the integration efficiency of different Cas9-PBase mutants at different endogenous safe harbor sites. Design three safe harbor sites for sgRNA and synthesize them onto different Cas9-PBase mutant vectors. The three sites and sgRNA targeting sequences are: ROSA sgRNA targeting sequence (GTCGAGTCGCTTTCGATTA, SEQ ID NO: 132), GSH1sgRNA targeting sequence (TTAGTCCTAGTGCCCATGAAAG, SEQ ID NO: 133), and GSH2sgRNA targeting sequence (CATCAGACTTGATGATG, SEQ ID NO: 134).

Different Cas9-PBase mutant gRNA plasmids constructed in Example 3 (1.3 µg, Cas9-PBase N347A_R376E_D450N enzyme and plasmid sequences expressing three safe harbor sgRNAs are shown in SEQ ID NO: 18-20, respectively. The 4501-6282 positions of sequence 18-20 are replaced with the 4183-5964 positions of SEQ ID NO: 135-137 to obtain the gRNA sequences of the other three mutants for the three safe harbors) were co transfected into HeLa cells with NeoR-GFP transposon donor (1.2 µg) using lipofectamine 3000 according to standard operating procedures. After transfection for 3 days and 14 days, the gRNA sequences of the remaining three mutants for the three safe harbors were obtained. The GFP positivity rate was detected by flow cytometry. The positive rate measured 3 days after transfection is the transfection efficiency of the corresponding sample, and the positive rate measured 14 days after transfection is the stable integration efficiency of the corresponding sample. The experimental methods for HeLa cell transfection and flow cytometry detection are as follows:
1) Before transfecting HeLa cells with plasmids, place the HeLa cells in a 6-well plate and control the cell density between 60-70%. Plasmid ratio Cas9-PBm 1.3 µg, NeoR-GFP transposon donor 1.2 µg. Using Lipofectamine TM3000 transfection reagent purchased from Thermo Scientific for cell transfection, the reaction system was described in the Lipofectamine TM3000 transfection reagent manual and incubated overnight at 37 °C for 20 hours in a cell culture incubator.
2) Remove the transfection reagent from HeLa cells, add 250 µL trypsin, incubate at 37 °C for 3 minutes, add 1.5mL DMEM high glucose medium containing 10% Fetal Bovine Serum to terminate the reaction, centrifuge at 200g for 5 minutes, remove the supernatant, resuspend the cells and culture them in 6cm dish (or add corresponding concentration of antibiotics), continue to culture at 37 °C for the described time.
3) On the 3rd and 14th day after transfection, the supernatant of HeLa cells was removed, and 600 µL trypsin was added. The cells were incubated at 37 °C for 3 minutes, and the reaction was terminated by adding 5mL of DMEM high glucose medium. The cells were then centrifuged at 1300rpm for 5 minutes, and the supernatant was removed. The cells were resuspended and used for flow cytometry analysis.
4) Flow cytometry was performed using the Attune NxT flow cytometer purchased from Thermo, using a flow tube for detection. The voltage parameters for lateral scattered light (FSC) and lateral scattered light (SSC) were set to FSC 60 and SSC 300, respectively, and the selected channel was BL1. The number of detected cells is 50000; Detect the proportion of green fluorescence. The ratio of GFP ratio on day 14 to GFP ratio on day 3 represents the integration efficiency of the corresponding Cas9-PBase mutant at the corresponding site.

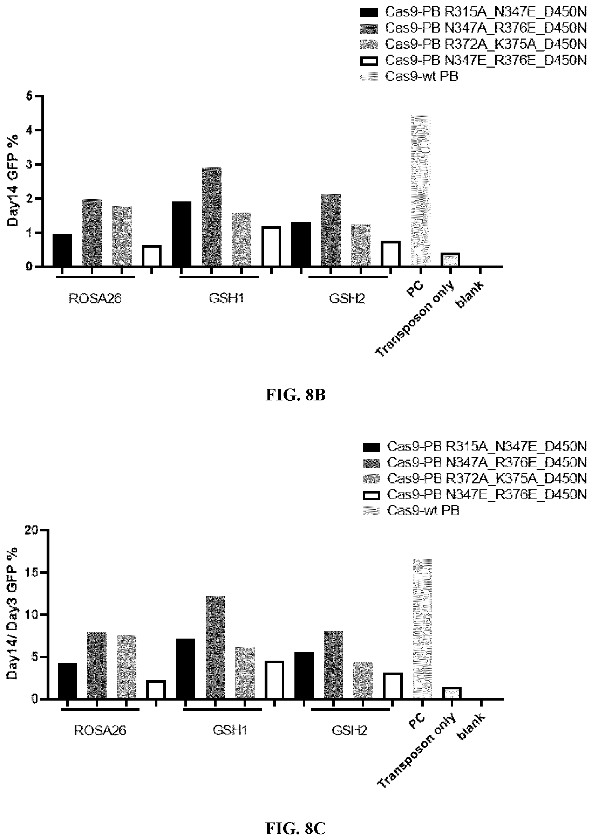

Results and Conclusion: The efficiency of co transfection with different Cas9-PBase mutant plasmids and NeoR-GFP transposon donors is relatively stable, about 20% -30% (FIG. 8A). In terms of stable integration efficiency at the three safe harbor sites, GSH1 has the highest efficiency, while ROSA and GSH2 sites have similar efficiencies, which may be due to the higher efficiency of sgRNA at the GSH1 site; Comparing different Cas9-PBase mutants at three endogenous loci, it was found that Cas9-PBase N347A_R376A_D450N had higher efficiency than Cas9-PBase R372A_K375A_D450N, while Cas9-PBase N347A_R376A_D450N and Cas9-PBase R315A_N347A_D450N had lower efficiency than Cas9-PBase R372A_K375A_D450N, consistent with the results obtained in the Split GFP cell line in Example 3 (FIG. 8B and FIG. 8C).

### Example 6 Detection of Targeted Integration Ratio and Integration Junction Sequence Characteristics of Cas9-PBase Mutant at GSH1 Site in HeLa Cells

The purpose of this experiment is to further test the targeted integration ratio of different Cas9-PBase mutants at the GSH1 safe harbor site and detect the sequence characteristics of the integration junction region.

GFP positive sorting was performed on stable integrated cells of different Cas9-PBase mutants at the GSH1 site in Example 5, and approximately 80-90 single-cell clones were cultured separately. Then, PCR was performed to detect the site integration of these clones, and PCR primers (SEQ ID NO: 126, 127 were paired with SEQ ID NO: 128, 129) were used to bind to the donor and upstream and downstream of the sgRNA target, respectively. And sequence a portion of the amplified bands, compare and observe the sequence of the donor genome junction region.

Experimental Results and Conclusion: Based on the site integration detection of all clones, the site integration ratios of different Cas9-PBase mutants were statistically obtained as follows: Cas9-PBase N347A_R376E_D450N was 38/86, Cas9-PBase R315E_N347E_D450N was 29/80, Cas9-PBase R372A_K375A_D450N was 40/96, and Cas9-PBase N347E_R376E_D450N was 56/96 (FIG. 10). Based on the results identified on single cells, it can be concluded that the new Cas9-PBase fusion mutants have lower off target rates for Cas9-PBase N347A_R376E_D450N and Cas9-PBase N347E_R376E_D450N compared to Cas9-PBase R372A_K375A_D450N, and slightly higher off target rates for Cas9-PBase R315AN347A_D450N than Cas9-PBase R372A_K375A_D450N. This conclusion is consistent with the off target ratio conclusion tested in the Split GFP cell line in Example 4.

Sequencing of PCR bands from partially integrated single-cell clones revealed small base deletions or insertions (Indel) in the linker region of most clones compared to the theoretical integrated sequence, and there was no clear quantity or sequence pattern of base deletions or insertions, consistent with the gene insertion characteristics of non homologous recombination (FIG. 9).

### Example 7 Testing the Site directed Integration Efficiency of Other Mutants at N347 Site

The N347 site is a key binding site for PBase and the "TTAA" targeting DNA upstream and downstream. The purpose of this experiment is to test other mutations at this site and assess the corresponding site integration efficiency.

Using Cas9-PBase R376E_D450N as a template, PBase N347 was mutated into 19 other amino acid forms, and different Cas9-PBase N347x_R376E_D450N (x represents various amino acid mutations) mutants were subjected to site integration testing on Split GFP cells. The experimental method is the same as described in Example 3, and will not be repeated here.

Experimental results and conclusions: The efficiency of co transfection between different Cas9-PBase N347x_R376E_D450N mutants and N GFP transposon donors is not significantly different (FIG. 11A), but the difference in site integration ratio is quite significant. In particular, the N347 mutation to S, T, Y, Q, C, G, A, E and other amino acids has a high site integration efficiency (FIG. 11B and FIG. 11C). Since both E and D amino acid residues are negatively charged, it is speculated that the mutation to D can also achieve similar effects based on the fact that E can reduce off target rate.

### Example 8 Testing the Site directed Integration Efficiency of Other Mutants at D450 Site

The D450 site is a key site that affects the efficiency of PBase splicing transposon donor. The purpose of this experiment is to test other mutations at this site and assess the corresponding site integration efficiency.

Using Cas9-PBase N347A_R376E as a template, PBase D450 was mutated into other amino acid forms, and different Cas9-PBase N347A_R376E_D450x (where x represents different amino acid mutations) mutants were subjected to site integration testing on Split GFP cells. The experimental method is the same as described in Example 3, and will not be repeated here.

Experimental results and conclusions: The efficiency of co transfection between different Cas9-PBase N347A_R376E_D450x mutants and N GFP transposon donors is not significantly different (FIG. 12A), but the difference in site integration ratio is quite significant. In particular, D450N has the highest efficiency, while D450 has a lower efficiency without mutation. Mutations in amino acids such as A, S, T, C, Y have a significant improvement compared to non mutations (FIG. 12B and FIG. 12C).

### Example 9: Adding other PBase site mutations on Cas9-PBase N347A_R376E_D450N to improve site integration efficiency

The binding site between PBase and ITR sequences may affect the efficiency of PBase cleavage of transposon donors, thereby affecting the efficiency of Cas9-PBase mutant cleavage and site integration of transposon donors. Therefore, mutating the binding site of the ITR sequence may enhance the site integration efficiency of Cas9-PBase mutants. The purpose of this experiment is to further explore the effect of ITR sequence binding site mutations on site integration efficiency on the Cas9-PBase N347A_R376E_D450N mutant.

Based on the analyzed structures of PBase protein and ITR (PDB number 6x67), analyze the key binding sites of PBase and ITR, and mutate these sites into positively charged or other polar amino acids. The process of constructing mutant plasmids is the same as described in Example 1, and will not be repeated here.

Different Cas9-PBase N347A_R376E_D450N MutX mutants will be constructed and tested for transfection and site integration efficiency in Split cells using the same method as in Examples 3, 7, and 8. And the fixed-point integration efficiency of Cas9-PBase N347A_R376E_D450N was defined as 100%, and the Cas9-PBase N347A_R376E_D450N MutX mutant with higher screening efficiency was selected.

Experimental Results and Conclusion: After screening, it was found that on the basis of the Cas9-PBase N347A_R376E_D450N triple mutation, a fourth mutation was added, and the proportion of site integration efficiency improvement by the newly added fourth site mutation MutX is shown in the parentheses after the mutation A166K(49.30%), N286R(138.52%), S289R(25.74%), G302R(15.16%), Q441R(36.87%), S498N(7.89%), E520R(27.01%), A521R(78.63%), T523R(32.89%), T557R(153.26%). (FIG. 13)

Although the specific embodiments of the present disclosure have been described above, those skilled in the art should understand that these are only examples, and various changes or modifications can be made to these embodiments without departing from the principles and essence of the present disclosure. Therefore, the scope of protection of the present disclosure is limited by the appended claims.
Cas9 amino acid sequence(including NLS)(SEQ ID NO:1) 
MDKKYSIGLDIGTNSVGWAVITDEYKVPSKKFKVLGNTDRHSIKKNLIGALLFDSGETAEATRLKRTARRRYTRRK
NRICYLQEIFSNEMAKVDDSFFHRLEESFLVEEDKKHERHPIFGNIVDEVAYHEKYPTIYHLRKKLVDSTDKADLRLI
YLALAHMIKFRGHFLIEGDLNPDNSDVDKLFIQLVQTYNQLFEENPINASGVDAKAILSARLSKSRRLENLIAQLPGE
KKNGLFGNLIALSLGLTPNFKSNFDLAEDAKLQLSKDTYDDDLDNLLAQIGDQYADLFLAAKNLSDAILLSDILRVN
 
TEITKAPLSASMIKRYDEHHQDLTLLKALVRQQLPEKYKEIFFDQSKNGYAGYIDGGASQEEFYKFIKPILEKMDGT
EELLVKLNREDLLRKQRTFDNGSIPHQIHLGELHAILRRQEDFYPFLKDNREKIEKILTFRIPYYVGPLARGNSRFAW
MTRKSEETITPWNFEEVVDKGASAQSFIERMTNFDKNLPNEKVLPKHSLLYEYFTVYNELTKVKYVTEGMRKPAFL
SGEQKKAIVDLLFKTNRKVTVKQLKEDYFKKIECFDSVEISGVEDRFNASLGTYHDLLKIIKDKDFLDNEENEDILED
IVLTLTLFEDREMIEERLKTYAHLFDDKVMKQLKRRRYTGWGRLSRKLINGIRDKQSGKTILDFLKSDGFANRNFM
QLIHDDSLTFKEDIQKAQVSGQGDSLHEHIANLAGSPAIKKGILQTVKVVDELVKVMGRHKPENIVIEMARENQTT
QKGQKNSRERMKRIEEGIKELGSQILKEHPVENTQLQNEKLYLYYLQNGRDMYVDQELDINRLSDYDVDHIVPQSF
LKDDSIDNKVLTRSDKNRGKSDNVPSEEVVKKMKNYWRQLLNAKLITQRKFDNLTKAERGGLSELDKAGFIKRQL
VETRQITKHVAQILDSRMNTKYDENDKLIREVKVITLKSKLVSDFRKDFQFYKVREINNYHHAHDAYLNAVVGTA
LIKKYPKLESEFVYGDYKVYDVRKMIAKSEQEIGKATAKYFFYSNIMNFFKTEITLANGEIRKRPLIETNGETGEIVW
DKGRDFATVRKVLSMPQVNIVKKTEVQTGGFSKESILPKRNSDKLIARKKDWDPKKYGGFDSPTVAYSVLVVAKV
EKGKSKKLKSVKELLGITIMERSSFEKNPIDFLEAKGYKEVKKDLIIKLPKYSLFELENGRKRMLASAGELQKGNEL
ALPSKYVNFLYLASHYEKLKGSPEDNEQKQLFVEQHKHYLDEIIEQISEFSKRVILADANLDKVLSAYNKHRDKPIR
EQAENIIHLFTLTNLGAPAAFKYFDTTIDRKRYTSTKEVLDATLIHQSITGLYETRIDLSQLGGDSRADPKKKRKV
Linker 3*G4S amino acid sequence(SEQ ID NO:2)
   GGGGSGGGGSGGGGS
wt Hyperactive PBase amino acid sequence(SEQ ID NO:3) 
MGSSLDDEHILSALLQSDDELVGEDSDSEVSDHVSEDDVQSDTEEAFIDEVHEVQPTSSGSEILDEQNVIEQPGSSLA
SNRILTLPQRTIRGKNKHCWSTSKSTRRSRVSALNIVRSQRGPTRMCRNIYDPLLCFKLFFTDEIISEIVKWTNAEISL
KRRESMTSATFRDTNEDEIYAFFGILVMTAVRKDNHMSTDDLFDRSLSMVYVSVMSRDRFDFLIRCLRMDDKSIRP
TLRENDVFTPVRKIWDLFIHQCIQNYTPGAHLTIDEQLLGFRGRCPFRVYIPNKPSKYGIKILMMCDSGTKYMINGM
PYLGRGTQTNGVPLGEYYVKELSKPVHGSCRNITCDNWFTSIPLAKNLLQEPYKLTIVGTVRSNKREIPEVLKNSRS
RPVGTSMFCFDGPLTLVSYKPKPAKMVYLLSSCDEDASINESTGKPQMVMYYNQTKGGVDTLDQMCSVMTCSRK
TNRWPMALLYGMINIACINSFIIYSHNVSSKGEKVQSRKKFMRNLYMSLTSSFMRKRLEAPTLKRYLRDNISNILPK
EVPGTSDDSTEEPVMKKRTYCTYCPSKIRRKANASCKKCKKVICREHNIDMCQSCF
Cas9-wt Hyperactive PBase amino acid sequence(SEQ ID NO:4) 
MDKKYSIGLDIGTNSVGWAVITDEYKVPSKKFKVLGNTDRHSIKKNLIGALLFDSGETAEATRLKRTARRRYTRRK
NRICYLQEIFSNEMAKVDDSFFHRLEESFLVEEDKKHERHPIFGNIVDEVAYHEKYPTIYHLRKKLVDSTDKADLRLI
YLALAHMIKFRGHFLIEGDLNPDNSDVDKLFIQLVQTYNQLFEENPINASGVDAKAILSARLSKSRRLENLIAQLPGE
KKNGLFGNLIALSLGLTPNFKSNFDLAEDAKLQLSKDTYDDDLDNLLAQIGDQYADLFLAAKNLSDAILLSDILRVN
TEITKAPLSASMIKRYDEHHQDLTLLKALVRQQLPEKYKEIFFDQSKNGYAGYIDGGASQEEFYKFIKPILEKMDGT
EELLVKLNREDLLRKQRTFDNGSIPHQIHLGELHAILRRQEDFYPFLKDNREKIEKILTFRIPYYVGPLARGNSRFAW
MTRKSEETITPWNFEEVVDKGASAQSFIERMTNFDKNLPNEKVLPKHSLLYEYFTVYNELTKVKYVTEGMRKPAFL
SGEQKKAIVDLLFKTNRKVTVKQLKEDYFKKIECFDSVEISGVEDRFNASLGTYHDLLKIIKDKDFLDNEENEDILED
IVLTLTLFEDREMIEERLKTYAHLFDDKVMKQLKRRRYTGWGRLSRKLINGIRDKQSGKTILDFLKSDGFANRNFM
 
QLIHDDSLTFKEDIQKAQVSGQGDSLHEHIANLAGSPAIKKGILQTVKVVDELVKVMGRHKPENIVIEMARENQTT
QKGQKNSRERMKRIEEGIKELGSQILKEHPVENTQLQNEKLYLYYLQNGRDMYVDQELDINRLSDYDVDHIVPQSF
LKDDSIDNKVLTRSDKNRGKSDNVPSEEVVKKMKNYWRQLLNAKLITQRKFDNLTKAERGGLSELDKAGFIKRQL
VETRQITKHVAQILDSRMNTKYDENDKLIREVKVITLKSKLVSDFRKDFQFYKVREINNYHHAHDAYLNAVVGTA
LIKKYPKLESEFVYGDYKVYDVRKMIAKSEQEIGKATAKYFFYSNIMNFFKTEITLANGEIRKRPLIETNGETGEIVW
DKGRDFATVRKVLSMPQVNIVKKTEVQTGGFSKESILPKRNSDKLIARKKDWDPKKYGGFDSPTVAYSVLVVAKV
EKGKSKKLKSVKELLGITIMERSSFEKNPIDFLEAKGYKEVKKDLIIKLPKYSLFELENGRKRMLASAGELQKGNEL
ALPSKYVNFLYLASHYEKLKGSPEDNEQKQLFVEQHKHYLDEIIEQISEFSKRVILADANLDKVLSAYNKHRDKPIR
EQAENIIHLFTLTNLGAPAAFKYFDTTIDRKRYTSTKEVLDATLIHQSITGLYETRIDLSQLGGDSRADPKKKRKVGG
GGSGGGGSGGGGSGSSLDDEHILSALLQSDDELVGEDSDSEVSDHVSEDDVQSDTEEAFIDEVHEVQPTSSGSEILD
EQNVIEQPGSSLASNRILTLPQRTIRGKNKHCWSTSKSTRRSRVSALNIVRSQRGPTRMCRNIYDPLLCFKLFFTDEIIS
EIVKWTNAEISLKRRESMTSATFRDTNEDEIYAFFGILVMTAVRKDNHMSTDDLFDRSLSMVYVSVMSRDRFDFLI
RCLRMDDKSIRPTLRENDVFTPVRKIWDLFIHQCIQNYTPGAHLTIDEQLLGFRGRCPFRVYIPNKPSKYGIKILMMC
DSGTKYMINGMPYLGRGTQTNGVPLGEYYVKELSKPVHGSCRNITCDNWFTSIPLAKNLLQEPYKLTIVGTVRSNK
REIPEVLKNSRSRPVGTSMFCFDGPLTLVSYKPKPAKMVYLLSSCDEDASINESTGKPQMVMYYNQTKGGVDTLD
QMCSVMTCSRKTNRWPMALLYGMINIACINSFIIYSHNVSSKGEKVQSRKKFMRNLYMSLTSSFMRKRLEAPTLKR
YLRDNISNILPKEVPGTSDDSTEEPVMKKRTYCTYCPSKIRRKANASCKKCKKVICREHNIDMCQSCF
 Note: The underline represents the PBase section.
PBase R315A_N347E_D450N amino acid sequence(SEQ ID NO:5) 
MGSSLDDEHILSALLQSDDELVGEDSDSEVSDHVSEDDVQSDTEEAFIDEVHEVQPTSSGSEILDEQNVIEQPGSSLA
SNRILTLPQRTIRGKNKHCWSTSKSTRRSRVSALNIVRSQRGPTRMCRNIYDPLLCFKLFFTDEIISEIVKWTNAEISL
KRRESMTSATFRDTNEDEIYAFFGILVMTAVRKDNHMSTDDLFDRSLSMVYVSVMSRDRFDFLIRCLRMDDKSIRP
TLRENDVFTPVRKIWDLFIHQCIQNYTPGAHLTIDEQLLGFRGRCPFRVYIPNKPSKYGIKILMMCDSGTKYMINGM
PYLGAGTQTNGVPLGEYYVKELSKPVHGSCRNITCDEWFTSIPLAKNLLQEPYKLTIVGTVRSNKREIPEVLKNSRS
RPVGTSMFCFDGPLTLVSYKPKPAKMVYLLSSCDEDASINESTGKPQMVMYYNQTKGGVDTLNQMCSVMTCSRK
TNRWPMALLYGMINIACINSFIIYSHNVSSKGEKVQSRKKFMRNLYMSLTSSFMRKRLEAPTLKRYLRDNISNILPK
EVPGTSDDSTEEPVMKKRTYCTYCPSKIRRKANASCKKCKKVICREHNIDMCQSCF
Cas9-PBase R315A_N347E_D450N amino acid sequence(SEQ ID NO:6) 
MDKKYSIGLDIGTNSVGWAVITDEYKVPSKKFKVLGNTDRHSIKKNLIGALLFDSGETAEATRLKRTARRRYTRRK
NRICYLQEIFSNEMAKVDDSFFHRLEESFLVEEDKKHERHPIFGNIVDEVAYHEKYPTIYHLRKKLVDSTDKADLRLI
YLALAHMIKFRGHFLIEGDLNPDNSDVDKLFIQLVQTYNQLFEENPINASGVDAKAILSARLSKSRRLENLIAQLPGE
KKNGLFGNLIALSLGLTPNFKSNFDLAEDAKLQLSKDTYDDDLDNLLAQIGDQYADLFLAAKNLSDAILLSDILRVN
TEITKAPLSASMIKRYDEHHQDLTLLKALVRQQLPEKYKEIFFDQSKNGYAGYIDGGASQEEFYKFIKPILEKMDGT
EELLVKLNREDLLRKQRTFDNGSIPHQIHLGELHAILRRQEDFYPFLKDNREKIEKILTFRIPYYVGPLARGNSRFAW
MTRKSEETITPWNFEEVVDKGASAQSFIERMTNFDKNLPNEKVLPKHSLLYEYFTVYNELTKVKYVTEGMRKPAFL
SGEQKKAIVDLLFKTNRKVTVKQLKEDYFKKIECFDSVEISGVEDRFNASLGTYHDLLKIIKDKDFLDNEENEDILED
 
IVLTLTLFEDREMIEERLKTYAHLFDDKVMKQLKRRRYTGWGRLSRKLINGIRDKQSGKTILDFLKSDGFANRNFM
QLIHDDSLTFKEDIQKAQVSGQGDSLHEHIANLAGSPAIKKGILQTVKVVDELVKVMGRHKPENIVIEMARENQTT
QKGQKNSRERMKRIEEGIKELGSQILKEHPVENTQLQNEKLYLYYLQNGRDMYVDQELDINRLSDYDVDHIVPQSF
LKDDSIDNKVLTRSDKNRGKSDNVPSEEVVKKMKNYWRQLLNAKLITQRKFDNLTKAERGGLSELDKAGFIKRQL
VETRQITKHVAQILDSRMNTKYDENDKLIREVKVITLKSKLVSDFRKDFQFYKVREINNYHHAHDAYLNAVVGTA
LIKKYPKLESEFVYGDYKVYDVRKMIAKSEQEIGKATAKYFFYSNIMNFFKTEITLANGEIRKRPLIETNGETGEIVW
DKGRDFATVRKVLSMPQVNIVKKTEVQTGGFSKESILPKRNSDKLIARKKDWDPKKYGGFDSPTVAYSVLVVAKV
EKGKSKKLKSVKELLGITIMERSSFEKNPIDFLEAKGYKEVKKDLIIKLPKYSLFELENGRKRMLASAGELQKGNEL
ALPSKYVNFLYLASHYEKLKGSPEDNEQKQLFVEQHKHYLDEIIEQISEFSKRVILADANLDKVLSAYNKHRDKPIR
EQAENIIHLFTLTNLGAPAAFKYFDTTIDRKRYTSTKEVLDATLIHQSITGLYETRIDLSQLGGDSRADPKKKRKVGG
GGSGGGGSGGGGSGSSLDDEHILSALLQSDDELVGEDSDSEVSDHVSEDDVQSDTEEAFIDEVHEVQPTSSGSEILD
EQNVIEQPGSSLASNRILTLPQRTIRGKNKHCWSTSKSTRRSRVSALNIVRSQRGPTRMCRNIYDPLLCFKLFFTDEIIS
EIVKWTNAEISLKRRESMTSATFRDTNEDEIYAFFGILVMTAVRKDNHMSTDDLFDRSLSMVYYSVMSRDRFDFLI
RCLRMDDKSIRPTLRENDVFTPVRKIWDLFIHQCIQNYTPGAHLTIDEQLLGFRGRCPFRVYIPNKPSKYGIKILMMC
DSGTKYMINGMPYLGAGTQTNGVPLGEYYVKELSKPVHGSCRNITCDEWFTSIPLAKNLLQEPYKLTIVGTVRSNK
REIPEVLKNSRSRPVGTSMFCFDGPLTLVSYKPKPAKMVYLLSSCDEDASINESTGKPQMVMYYNQTKGGVDTLN
QMCSVMTCSRKTNRWPMALLYGMINIACINSFIIYSHNVSSKGEKVQSRKKFMRNLYMSLTSSFMRKRLEAPTLKR
YLRDNISNILPKEVPGTSDDSTEEPVMKKRTYCTYCPSKIRRKANASCKKCKKVICREHNIDMCQSCF
 Note: The underline represents the PBase section.
PBase N347E_R376E_D450N amino acid sequence(SEQ ID NO:7) 
MGSSLDDEHILSALLQSDDELVGEDSDSEVSDHVSEDDVQSDTEEAFIDEVHEVQPTSSGSEILDEQNVIEQPGSSLA
SNRILTLPQRTIRGKNKHCWSTSKSTRRSRVSALNIVRSQRGPTRMCRNIYDPLLCFKLFFTDEIISEIVKWTNAEISL
KRRESMTSATFRDTNEDEIYAFFGILVMTAVRKDNHMSTDDLFDRSLSMVYVSVMSRDRFDFLIRCLRMDDKSIRP
TLRENDVFTPVRKIWDLFIHQCIQNYTPGAHLTIDEQLLGFRGRCPFRVYIPNKPSKYGIKILMMCDSGTKYMINGM
PYLGRGTQTNGVPLGEYYVKELSKPVHGSCRNITCDEWFTSIPLAKNLLQEPYKLTIVGTVRSNKEEIPEVLKNSRSR
PVGTSMFCFDGPLTLVSYKPKPAKMVYLLSSCDEDASINESTGKPQMVMYYNQTKGGVDTLNQMCSVMTCSRKT
NRWPMALLYGMINIACINSFIIYSHNVSSKGEKVQSRKKFMRNLYMSLTSSFMRKRLEAPTLKRYLRDNISNILPKE
VPGTSDDSTEEPVMKKRTYCTYCPSKIRRKANASCKKCKKVICREHNIDMCQSCF
Cas9-PBase N347E_R376E_D450N amino acid sequence(SEQ ID NO:8) 
MDKKYSIGLDIGTNSVGWAVITDEYKVPSKKFKVLGNTDRHSIKKNLIGALLFDSGETAEATRLKRTARRRYTRRK
NRICYLQEIFSNEMAKVDDSFFHRLEESFLVEEDKKHERHPIFGNIVDEVAYHEKYPTIYHLRKKLVDSTDKADLRLI
YLALAHMIKFRGHFLIEGDLNPDNSDVDKLFIQLVQTYNQLFEENPINASGVDAKAILSARLSKSRRLENLIAQLPGE
KKNGLFGNLIALSLGLTPNFKSNFDLAEDAKLQLSKDTYDDDLDNLLAQIGDQYADLFLAAKNLSDAILLSDILRVN
TEITKAPLSASMIKRYDEHHQDLTLLKALVRQQLPEKYKEIFFDQSKNGYAGYIDGGASQEEFYKFIKPILEKMDGT
EELLVKLNREDLLRKQRTFDNGSIPHQIHLGELHAILRRQEDFYPFLKDNREKIEKILTFRIPYYVGPLARGNSRFAW
MTRKSEETITPWNFEEVVDKGASAQSFIERMTNFDKNLPNEKVLPKHSLLYEYFTVYNELTKVKYVTEGMRKPAFL
 
SGEQKKAIVDLLFKTNRKVTVKQLKEDYFKKIECFDSVEISGVEDRFNASLGTYHDLLKIIKDKDFLDNEENEDILED
IVLTLTLFEDREMIEERLKTYAHLFDDKVMKQLKRRRYTGWGRLSRKLINGIRDKQSGKTILDFLKSDGFANRNFM
QLIHDDSLTFKEDIQKAQVSGQGDSLHEHIANLAGSPAIKKGILQTVKVVDELVKVMGRHKPENIVIEMARENQTT
QKGQKNSRERMKRIEEGIKELGSQILKEHPVENTQLQNEKLYLYYLQNGRDMYVDQELDINRLSDYDVDHIVPQSF
LKDDSIDNKVLTRSDKNRGKSDNVPSEEVVKKMKNYWRQLLNAKLITQRKFDNLTKAERGGLSELDKAGFIKRQL
VETRQITKHVAQILDSRMNTKYDENDKLIREVKVITLKSKLVSDFRKDFQFYKVREINNYHHAHDAYLNAVVGTA
LIKKYPKLESEFVYGDYKVYDVRKMIAKSEQEIGKATAKYFFYSNIMNFFKTEITLANGEIRKRPLIETNGETGEIVW
DKGRDFATVRKVLSMPQVNIVKKTEVQTGGFSKESILPKRNSDKLIARKKDWDPKKYGGFDSPTVAYSVLVVAKV
EKGKSKKLKSVKELLGITIMERSSFEKNPIDFLEAKGYKEVKKDLIIKLPKYSLFELENGRKRMLASAGELQKGNEL
ALPSKYVNFLYLASHYEKLKGSPEDNEQKQLFVEQHKHYLDEIIEQISEFSKRVILADANLDKVLSAYNKHRDKPIR
EQAENIIHLFTLTNLGAPAAFKYFDTTIDRKRYTSTKEVLDATLIHQSITGLYETRIDLSQLGGDSRADPKKKRKVGG
GGSGGGGSGGGGSGSSLDDEHILSALLQSDDELVGEDSDSEVSDHVSEDDVQSDTEEAFIDEVHEVQPTSSGSEILD
EQNVIEQPGSSLASNRILTLPQRTIRGKNKHCWSTSKSTRRSRVSALNIVRSQRGPTRMCRNIYDPLLCFKLFFTDEIIS
EIVKWTNAEISLKRRESMTSATFRDTNEDEIYAFFGILVMTAVRKDNHMSTDDLFDRSLSMVYVSVMSRDRFDFLI
RCLRMDDKSIRPTLRENDVFTPVRKIWDLFIHQCIQNYTPGAHLTIDEQLLGFRGRCPFRVYIPNKPSKYGIKILMMC
DSGTKYMINGMPYLGRGTQTNGVPLGEYYVKELSKPVHGSCRNITCDEWFTSIPLAKNLLQEPYKLTIVGTVRSNK
EEIPEVLKNSRSRPVGTSMFCFDGPLTLVSYKPKPAKMVYLLSSCDEDASINESTGKPQMVMYYNQTKGGVDTLNQ
MCSVMTCSRKTNRWPMALLYGMINIACINSFIIYSHNVSSKGEKVQSRKKFMRNLYMSLTSSFMRKRLEAPTLKRY
LRDNISNILPKEVPGTSDDSTEEPVMKKRTYCTYCPSKIRRKANASCKKCKKVICREHNIDMCQSCF
 Note: The underline represents the PBase section.
PBase N347A_R376E_D450N amino acid sequence(SEQ ID NO:9) 
MGSSLDDEHILSALLQSDDELVGEDSDSEVSDHVSEDDVQSDTEEAFIDEVHEVQPTSSGSEILDEQNVIEQPGSSLA
SNRILTLPQRTIRGKNKHCWSTSKSTRRSRVSALNIVRSQRGPTRMCRNIYDPLLCFKLFFTDEIISEIVKWTNAEISL
KRRESMTSATFRDTNEDEIYAFFGILVMTAVRKDNHMSTDDLFDRSLSMVYVSVMSRDRFDFLIRCLRMDDKSIRP
TLRENDVFTPVRKIWDLFIHQCIQNYTPGAHLTIDEQLLGFRGRCPFRVYIPNKPSKYGIKILMMCDSGTKYMINGM
PYLGRGTQTNGVPLGEYYVKELSKPVHGSCRNITCDAWFTSIPLAKNLLQEPYKLTIVGTVRSNKEEIPEVLKNSRS
RPVGTSMFCFDGPLTLVSYKPKPAKMVYLLSSCDEDASINESTGKPQMVMYYNQTKGGVDTLNQMCSVMTCSRK
TNRWPMALLYGMINIACINSFIIYSHNVSSKGEKVQSRKKFMRNLYMSLTSSFMRKRLEAPTLKRYLRDNISNILPK
EVPGTSDDSTEEPVMKKRTYCTYCPSKIRRKANASCKKCKKVICREHNIDMCQSCF
Cas9-PBase N347A_R376E_D450N amino acid sequence(SEQ ID NO:10) 
MDKKYSIGLDIGTNSVGWAVITDEYKVPSKKFKVLGNTDRHSIKKNLIGALLFDSGETAEATRLKRTARRRYTRRK
NRICYLQEIFSNEMAKVDDSFFHRLEESFLVEEDKKHERHPIFGNIVDEVAYHEKYPTIYHLRKKLVDSTDKADLRLI
YLALAHMIKFRGHFLIEGDLNPDNSDVDKLFIQLVQTYNQLFEENPINASGVDAKAILSARLSKSRRLENLIAQLPGE
KKNGLFGNLIALSLGLTPNFKSNFDLAEDAKLQLSKDTYDDDLDNLLAQIGDQYADLFLAAKNLSDAILLSDILRVN
TEITKAPLSASMIKRYDEHHQDLTLLKALVRQQLPEKYKEIFFDQSKNGYAGYIDGGASQEEFYKFIKPILEKMDGT
EELLVKLNREDLLRKQRTFDNGSIPHQIHLGELHAILRRQEDFYPFLKDNREKIEKILTFRIPYYVGPLARGNSRFAW
 
MTRKSEETITPWNFEEVVDKGASAQSFIERMTNFDKNLPNEKVLPKHSLLYEYFTVYNELTKVKYVTEGMRKPAFL
SGEQKKAIVDLLFKTNRKVTVKQLKEDYFKKIECFDSVEISGVEDRFNASLGTYHDLLKIIKDKDFLDNEENEDILED
IVLTLTLFEDREMIEERLKTYAHLFDDKVMKQLKRRRYTGWGRLSRKLINGIRDKQSGKTILDFLKSDGFANRNFM
QLIHDDSLTFKEDIQKAQVSGQGDSLHEHIANLAGSPAIKKGILQTVKVVDELVKVMGRHKPENIVIEMARENQTT
QKGQKNSRERMKRIEEGIKELGSQILKEHPVENTQLQNEKLYLYYLQNGRDMYVDQELDINRLSDYDVDHIVPQSF
LKDDSIDNKVLTRSDKNRGKSDNVPSEEVVKKMKNYWRQLLNAKLITQRKFDNLTKAERGGLSELDKAGFIKRQL
VETRQITKHVAQILDSRMNTKYDENDKLIREVKVITLKSKLVSDFRKDFQFYKVREINNYHHAHDAYLNAVVGTA
LIKKYPKLESEFVYGDYKVYDVRKMIAKSEQEIGKATAKYFFYSNIMNFFKTEITLANGEIRKRPLIETNGETGEIVW
DKGRDFATVRKVLSMPQVNIVKKTEVQTGGFSKESILPKRNSDKLIARKKDWDPKKYGGFDSPTVAYSVLVVAKV
EKGKSKKLKSVKELLGITIMERSSFEKNPIDFLEAKGYKEVKKDLIIKLPKYSLFELENGRKRMLASAGELQKGNEL
ALPSKYVNFLYLASHYEKLKGSPEDNEQKQLFVEQHKHYLDEIIEQISEFSKRVILADANLDKVLSAYNKHRDKPIR
EQAENIIHLFTLTNLGAPAAFKYFDTTIDRKRYTSTKEVLDATLIHQSITGLYETRIDLSQLGGDSRADPKKKRKVGG
GGSGGGGSGGGGSGSSLDDEHILSALLQSDDELVGEDSDSEVSDHVSEDDVQSDTEEAFIDEVHEVQPTSSGSEILD
EQNVIEQPGSSLASNRILTLPQRTIRGKNKHCWSTSKSTRRSRVSALNIVRSQRGPTRMCRNIYDPLLCFKLFFTDEIIS
EIVKWTNAEISLKRRESMTSATFRDTNEDEIYAFFGILVMTAVRKDNHMSTDDLFDRSLSMVYVSVMSRDRFDFLI
RCLRMDDKSIRPTLRENDVFTPVRKIWDLFIHQCIQNYTPGAHLTIDEQLLGFRGRCPFRVYIPNKPSKYGIKILMMC
DSGTKYMINGMPYLGRGTQTNGVPLGEYYVKELSKPVHGSCRNITCDAWFTSIPLAKNLLQEPYKLTIVGTVRSNK
EEIPEVLKNSRSRPVGTSMFCFDGPLTLVSYKPKPAKMVYLLSSCDEDASINESTGKPQMVMYYNQTKGGVDTLNQ
MCSVMTCSRKTNRWPMALLYGMINIACINSFIIYSHNVSSKGEKVQSRKKFMRNLYMSLTSSFMRKRLEAPTLKRY
LRDNISNILPKEVPGTSDDSTEEPVMKKRTYCTYCPSKIRRKANASCKKCKKVICREHNIDMCQSCF
 Note: The underline represents the PBase section.
PBase R372AK375A D450N amino acid sequence(SEQ ID NO:11) 
MGSSLDDEHILSALLQSDDELVGEDSDSEVSDHVSEDDVQSDTEEAFIDEVHEVQPTSSGSEILDEQNVIEQPGSSLA
SNRILTLPQRTIRGKNKHCWSTSKSTRRSRVSALNIVRSQRGPTRMCRNIYDPLLCFKLFFTDEIISEIVKWTNAEISL
KRRESMTSATFRDTNEDEIYAFFGILVMTAVRKDNHMSTDDLFDRSLSMVYVSVMSRDRFDFLIRCLRMDDKSIRP
TLRENDVFTPVRKIWDLFIHQCIQNYTPGAHLTIDEQLLGFRGRCPFRVYIPNKPSKYGIKILMMCDSGTKYMINGM
PYLGRGTQTNGVPLGEYYVKELSKPVHGSCRNITCDNWFTSIPLAKNLLQEPYKLTIVGTVASNAREIPEVLKNSRS
RPVGTSMFCFDGPLTLVSYKPKPAKMVYLLSSCDEDASINESTGKPQMVMYYNQTKGGVDTLNQMCSVMTCSRK
TNRWPMALLYGMINIACINSFIIYSHNVSSKGEKVQSRKKFMRNLYMSLTSSFMRKRLEAPTLKRYLRDNISNILPK
EVPGTSDDSTEEPVMKKRTYCTYCPSKIRRKANASCKKCKKVICREHNIDMCQSCF
Cas9-PBase R372A_K375A_D450N amino acid sequence(SEQ ID NO:12) 
MDKKYSIGLDIGTNSVGWAVITDEYKVPSKKFKVLGNTDRHSIKKNLIGALLFDSGETAEATRLKRTARRRYTRRK
NRICYLQEIFSNEMAKVDDSFFHRLEESFLVEEDKKHERHPIFGNIVDEVAYHEKYPTIYHLRKKLVDSTDKADLRLI
YLALAHMIKFRGHFLIEGDLNPDNSDVDKLFIQLVQTYNQLFEENPINASGVDAKAILSARLSKSRRLENLIAQLPGE
KKNGLFGNLIALSLGLTPNFKSNFDLAEDAKLQLSKDTYDDDLDNLLAQIGDQYADLFLAAKNLSDAILLSDILRVN
TEITKAPLSASMIKRYDEHHQDLTLLKALVRQQLPEKYKEIFFDQSKNGYAGYIDGGASQEEFYKFIKPILEKMDGT
 
EELLVKLNREDLLRKQRTFDNGSIPHQIHLGELHAILRRQEDFYPFLKDNREKIEKILTFRIPYYVGPLARGNSRFAW
MTRKSEETITPWNFEEVVDKGASAQSFIERMTNFDKNLPNEKVLPKHSLLYEYFTVYNELTKVKYVTEGMRKPAFL
SGEQKKAIVDLLFKTNRKVTVKQLKEDYFKKIECFDSVEISGVEDRFNASLGTYHDLLKIIKDKDFLDNEENEDILED
IVLTLTLFEDREMIEERLKTYAHLFDDKVMKQLKRRRYTGWGRLSRKLINGIRDKQSGKTILDFLKSDGFANRNFM
QLIHDDSLTFKEDIQKAQVSGQGDSLHEHIANLAGSPAIKKGILQTVKVVDELVKVMGRHKPENIVIEMARENQTT
QKGQKNSRERMKRIEEGIKELGSQILKEHPVENTQLQNEKLYLYYLQNGRDMYVDQELDINRLSDYDVDHIVPQSF
LKDDSIDNKVLTRSDKNRGKSDNVPSEEVVKKMKNYWRQLLNAKLITQRKFDNLTKAERGGLSELDKAGFIKRQL
VETRQITKHVAQILDSRMNTKYDENDKLIREVKVITLKSKLVSDFRKDFQFYKVREINNYHHAHDAYLNAVVGTA
LIKKYPKLESEFVYGDYKVYDVRKMIAKSEQEIGKATAKYFFYSNIMNFFKTEITLANGEIRKRPLIETNGETGEIVW
DKGRDFATVRKVLSMPQVNIVKKTEVQTGGFSKESILPKRNSDKLIARKKDWDPKKYGGFDSPTVAYSVLVVAKV
EKGKSKKLKSVKELLGITIMERSSFEKNPIDFLEAKGYKEVKKDLIIKLPKYSLFELENGRKRMLASAGELQKGNEL
ALPSKYVNFLYLASHYEKLKGSPEDNEQKQLFVEQHKHYLDEIIEQISEFSKRVILADANLDKVLSAYNKHRDKPIR
EQAENIIHLFTLTNLGAPAAFKYFDTTIDRKRYTSTKEVLDATLIHQSITGLYETRIDLSQLGGDSRADPKKKRKVGG
GGSGGGGSGGGGSGSSLDDEHILSALLQSDDELVGEDSDSEVSDHVSEDDVQSDTEEAFIDEVHEVQPTSSGSEILD
EQNVIEQPGSSLASNRILTLPQRTIRGKNKHCWSTSKSTRRSRVSALNIVRSQRGPTRMCRNIYDPLLCFKLFFTDEIIS
EIVKWTNAEISLKRRESMTSATFRDTNEDEIYAFFGILVMTAVRKDNHMSTDDLFDRSLSMVYVSVMSRDRFDFLI
RCLRMDDKSIRPTLRENDVFTPVRKIWDLFIHQCIQNYTPGAHLTIDEQLLGFRGRCPFRVYIPNKPSKYGIKILMMC
DSGTKYMINGMPYLGRGTQTNGVPLGEYYVKELSKPVHGSCRNITCDNWFTSIPLAKNLLQEPYKLTIVGTVASNA
REIPEVLKNSRSRPVGTSMFCFDGPLTLVSYKPKPAKMVYLLSSCDEDASINESTGKPQMVMYYNQTKGGVDTLN
QMCSVMTCSRKTNRWPMALLYGMINIACINSFIIYSHNVSSKGEKVQSRKKFMRNLYMSLTSSFMRKRLEAPTLKR
YLRDNISNILPKEVPGTSDDSTEEPVMKKRTYCTYCPSKIRRKANASCKKCKKVICREHNIDMCQSCF
 Note: The underline represents the PBase section.
wt Hyperactive PBase encoding DNA sequence(SEQ ID NO:13) 
ATGGGCTCTAGCCTGGACGACGAGCACATCCTGAGCGCCCTGCTGCAGAGCGACGACGAACTGGTGGGCGAG
GACAGCGACAGCGAGGTCAGCGACCACGTGTCCGAGGACGACGTGCAGTCCGACACCGAGGAAGCCTTCATC
GACGAGGTGCACGAAGTGCAGCCTACCAGCAGCGGCTCCGAGATCCTGGACGAGCAGAACGTGATCGAGCAG
CCTGGCAGCTCCCTGGCCAGCAACAGAATCCTGACCCTGCCCCAGAGAACCATCAGAGGCAAGAACAAGCAC
TGCTGGTCCACCTCCAAGAGCACCAGGCGGAGCAGAGTGTCCGCCCTGAACATCGTGCGGAGCCAGAGGGGC
CCCACCAGAATGTGCAGAAACATCTACGACCCCCTGCTGTGCTTCAAGCTGTTCTTCACCGACGAGATCATCA
GCGAGATCGTGAAGTGGACCAACGCCGAGATCAGCCTGAAGAGGCGGGAGAGCATGACCAGCGCCACCTTCA
GAGACACCAACGAGGACGAGATCTACGCCTTCTTCGGCATCCTGGTGATGACCGCCGTGAGAAAGGACAACC
ACATGAGCACCGACGACCTGTTCGACAGATCCCTGAGCATGGTGTACGTGTCCGTGATGAGCAGAGACAGATT
CGACTTCCTGATCAGATGCCTGAGAATGGACGACAAGAGCATCAGACCCACCCTGCGGGAGAACGACGTGTT
CACCCCCGTGCGGAAGATCTGGGACCTGTTCATCCACCAGTGCATCCAGAACTACACCCCTGGCGCCCACCTG
ACCATCGATGAGCAGCTGCTGGGCTTCAGAGGCAGATGCCCCTTCAGAGTGTACATCCCCAACAAGCCCAGCA
AGTACGGCATCAAGATCCTGATGATGTGCGACAGCGGCACCAAGTACATGATCAACGGCATGCCCTACCTGG
GCAGAGGCACCCAGACAAACGGCGTGCCCCTGGGCGAGTACTACGTGAAAGAACTGAGCAAGCCTGTGCATG
 
GCAGCTGCAGGAACATCACCTGCGACAACTGGTTCACCAGCATCCCCCTGGCCAAGAACCTGCTGCAGGAACC
CTACAAGCTGACCATCGTGGGCACCGTGCGGAGCAACAAGCGGGAGATCCCAGAGGTGCTGAAGAACAGCAG
ATCCAGACCTGTGGGAACAAGCATGTTCTGCTTCGACGGCCCCCTGACCCTGGTGTCCTACAAGCCCAAGCCC
GCCAAGATGGTGTACCTGCTGTCCAGCTGCGACGAGGACGCCAGCATCAACGAGAGCACCGGCAAGCCCCAG
ATGGTGATGTACTACAACCAGACCAAGGGCGGCGTGGACACCCTGGACCAGATGTGCAGCGTGATGACCTGC
AGCAGAAAGACCAACAGATGGCCCATGGCCCTGCTGTACGGCATGATCAATATCGCCTGCATCAACAGCTTCA
TCATCTACAGCCACAACGTGTCCAGCAAGGGCGAGAAGGTGCAGAGCCGGAAGAAATTCATGCGGAACCTGT
ACATGAGCCTGACCTCCAGCTTCATGAGAAAGAGACTGGAAGCCCCCACCCTGAAGAGATACCTGCGGGACA
ACATCAGCAACATCCTGCCCAAGGAAGTGCCAGGAACAAGCGACGACAGCACCGAGGAACCCGTGATGAAG
AAGAGGACCTACTGCACCTACTGTCCCAGCAAGATCAGAAGAAAGGCCAACGCCAGCTGCAAGAAATGCAAA
AAAGTGATCTGCCGGGAGCACAACATCGACATGTGCCAGAGCTGTTTCTGA
Cas9-wt Hyperactive PBase encoding DNA sequence(SEQ ID NO:14) 
ATGGACAAGAAGTACTCCATTGGGCTCGacATCGGCACAAACAGCGTCGGCTGGGCCGTCATTACGGACGAGT
ACAAGGTGCCGAGCAAAAAATTCAAAGTTCTGGGCAATACCGATCGCCACAGCATAAAGAAGAACCTCATTG
GCGCCCTCCTGTTCGACTCCGGGGAGACGGCCGAAGCCACGCGGCTCAAAAGAACAGCACGGCGCAGATATA
CCCGCAGAAAGAATCGGATCTGCTACCTGCAGGAGATCTTTAGTAATGAGATGGCTAAGGTGGATGACTCTTT
CTTCCATAGGCTGGAGGAGTCCTTTTTGGTGGAGGAGGATAAAAAGCACGAGCGCCACCCAATCTTTGGCAAT
ATCGTGGACGAGGTGGCGTACCATGAAAAGTACCCAACCATATATCATCTGAGGAAGAAGCTTGTAGACAGT
ACTGATAAGGCTGACTTGCGGTTGATCTATCTCGCGCTGGCGCATATGATCAAATTTCGGGGACACTTCCTCAT
CGAGGGGGACCTGAACCCAGACAACAGCGATGTCGACAAACTCTTTATCCAACTGGTTCAGACTTACAATCAG
CTTTTCGAAGAGAACCCGATCAACGCATCCGGAGTTGACGCCAAAGCAATCCTGAGCGCTAGGCTGTCCAAAT
CCCGGCGGCTCGAAAACCTCATCGCACAGCTCCCTGGGGAGAAGAAGAACGGCCTGTTTGGTAATCTTATCGC
CCTGTCACTCGGGCTGACCCCCAACTTTAAATCTAACTTCGACCTGGCCGAAGATGCCAAGCTTCAACTGAGC
AAAGACACCTACGATGATGATCTCGACAATCTGCTGGCCCAGATCGGCGACCAGTACGCAGACCTTTTTTTGG
CGGCAAAGAACCTGTCAGACGCCATTCTGCTGAGTGATATTCTGCGAGTGAACACGGAGATCACCAAAGCTCC
GCTGAGCGCTAGTATGATCAAGCGCTATGATGAGCACCACCAAGACTTGACTTTGCTGAAGGCCCTTGTCAGA
CAGCAACTGCCTGAGAAGTACAAGGAAATTTTCTTCGATCAGTCTAAAAATGGCTACGCCGGATACATTGACG
GCGGAGCAAGCCAGGAGGAATTTTACAAATTTATTAAGCCCATCTTGGAAAAAATGGACGGCACCGAGGAGC
TGCTGGTAAAGCTTAACAGAGAAGATCTGTTGCGCAAACAGCGCACTTTCGACAATGGAAGCATCCCCCACCA
GATTCACCTGGGCGAACTGCACGCTATCCTCAGGCGGCAAGAGGATTTCTACCCCTTTTTGAAAGATAACAGG
GAAAAGATTGAGAAAATCCTCACATTTCGGATACCCTACTATGTAGGCCCCCTCGCCCGGGGAAATTCCAGAT
TCGCGTGGATGACTCGCAAATCAGAAGAGACCATCACTCCCTGGAACTTCGAGGAAGTCGTGGATAAGGGGG
CCTCTGCCCAGTCCTTCATCGAAAGGATGACTAACTTTGATAAAAATCTGCCTAACGAAAAGGTGCTTCCTAA
ACACTCTCTGCTGTACGAGTACTTCACAGTTTATAACGAGCTCACCAAGGTCAAATACGTCACAGAAGGGATG
AGAAAGCCAGCATTCCTGTCTGGAGAGCAGAAGAAAGCTATCGTGGACCTCCTCTTCAAGACGAACCGGAAA
GTTACCGTGAAACAGCTCAAAGAAGACTATTTCAAAAAGATTGAATGTTTCGACTCTGTTGAAATCAGCGGAG
TGGAGGATCGCTTCAACGCATCCCTGGGAACGTATCACGATCTCCTGAAAATCATTAAAGACAAGGACTTCCT
 
GGACAATGAGGAGAACGAGGACATTCTTGAGGACATTGTCCTCACCCTTACGTTGTTTGAAGATAGGGAGATG
ATTGAAGAACGCTTGAAAACTTACGCTCATCTCTTCGACGACAAAGTCATGAAACAGCTCAAGAGGCGCCGAT
ATACAGGATGGGGGCGGCTGTCAAGAAAACTGATCAATGGGATCCGAGACAAGCAGAGTGGAAAGACAATC
CTGGATTTTCTTAAGTCCGATGGATTTGCCAACAGGAACTTCATGCAGTTGATCCATGATGACTCTCTCACCTT
TAAGGAGGACATCCAGAAAGCACAAGTTTCTGGCCAGGGGGACAGTCTTCACGAGCACATCGCTAATCTTGC
AGGTAGCCCAGCTATCAAAAAGGGAATACTGCAGACCGTTAAGGTCGTGGATGAACTCGTCAAAGTAATGGG
AAGGCATAAGCCCGAGAATATCGTTATCGAGATGGCCCGAGAGAACCAAACTACCCAGAAGGGACAGAAGA
ACAGTAGGGAAAGGATGAAGAGGATTGAAGAGGGTATAAAAGAACTGGGGTCCCAAATCCTTAAGGAACAC
CCAGTTGAAAACACCCAGCTTCAGAATGAGAAGCTCTACCTGTACTACCTGCAGAACGGCAGGGACATGTAC
GTGGATCAGGAACTGGACATCAATCGGCTCTCCGACTACGACGTGGATCATATCGTGCCCCAGTCTTTTCTCA
AAGATGATTCTATTGATAATAAAGTGTTGACAAGATCCGATAAAAATAGAGGGAAGAGTGATAACGTCCCCT
CAGAAGAAGTTGTCAAGAAAATGAAAAATTATTGGCGGCAGCTGCTGAACGCCAAACTGATCACACAACGGA
AGTTCGATAATCTGACTAAGGCTGAACGAGGTGGCCTGTCTGAGTTGGATAAAGCCGGCTTCATCAAAAGGCA
GCTTGTTGAGACACGCCAGATCACCAAGCACGTGGCCCAAATTCTCGATTCACGCATGAACACCAAGTACGAT
GAAAATGACAAACTGATTCGAGAGGTGAAAGTTATTACTCTGAAGTCTAAGCTGGTCTCAGATTTCAGAAAGG
ACTTTCAGTTTTATAAGGTGAGAGAGATCAACAATTACCACCATGCGCATGATGCCTACCTGAATGCAGTGGT
AGGCACTGCACTTATCAAAAAATATCCCAAGCTTGAATCTGAATTTGTTTACGGAGACTATAAAGTGTACGAT
GTTAGGAAAATGATCGCAAAGTCTGAGCAGGAAATAGGCAAGGCCACCGCTAAGTACTTCTTTTACAGCAAT
ATTATGAATTTTTTCAAGACCGAGATTACACTGGCCAATGGAGAGATTCGGAAGCGACCACTTATCGAAACAA
ACGGAGAAACAGGAGAAATCGTGTGGGACAAGGGTAGGGATTTCGCGACAGTCCGGAAGGTCCTGTCCATGC
CGCAGGTGAACATCGTTAAAAAGACCGAAGTACAGACCGGAGGCTTCTCCAAGGAAAGTATCCTCCCGAAAA
GGAACAGCGACAAGCTGATCGCACGCAAAAAAGATTGGGACCCCAAGAAATACGGCGGATTCGATTCTCCTA
CAGTCGCTTACAGTGTACTGGTTGTGGCCAAAGTGGAGAAAGGGAAGTCTAAAAAACTCAAAAGCGTCAAGG
AACTGCTGGGCATCACAATCATGGAGCGATCAAGCTTCGAAAAAAACCCCATCGACTTTCTCGAGGCGAAAG
GATATAAAGAGGTCAAAAAAGACCTCATCATTAAGCTTCCCAAGTACTCTCTCTTTGAGCTTGAAAACGGCCG
GAAACGAATGCTCGCTAGTGCGGGCGAGCTGCAGAAAGGTAACGAGCTGGCACTGCCCTCTAAATACGTTAA
TTTCTTGTATCTGGCCAGCCACTATGAAAAGCTCAAAGGGTCTCCCGAAGATAATGAGCAGAAGCAGCTGTTC
GTGGAACAACACAAACACTACCTTGATGAGATCATCGAGCAAATAAGCGAATTCTCCAAAAGAGTGATCCTC
GCCGACGCTAACCTCGATAAGGTGCTTTCTGCTTACAATAAGCACAGGGATAAGCCCATCAGGGAGCAGGCA
GAAAACATTATCCACTTGTTTACTCTGACCAACTTGGGCGCGCCTGCAGCCTTCAAGTACTTCGACACCACCAT
AGACAGAAAGCGGTACACCTCTACAAAGGAGGTCCTGGACGCCACACTGATTCATCAGTCAATTACGGGGCT
CTATGAAACAAGAATCGACCTCTCTCAGCTCGGTGGAGACAGCAGGGCTGACCCCAAGAAGAAGAGGAAGGT
GGGCGGAGGAGGGAGTGGCGGAGGCGGAAGCGGCGGCGGAGGCAGCGGCTCTAGCCTGGACGACGAGCACA
TCCTGAGCGCCCTGCTGCAGAGCGACGACGAACTGGTGGGCGAGGACAGCGACAGCGAGGTCAGCGACCACG
TGTCCGAGGACGACGTGCAGTCCGACACCGAGGAAGCCTTCATCGACGAGGTGCACGAAGTGCAGCCTACCA
GCAGCGGCTCCGAGATCCTGGACGAGCAGAACGTGATCGAGCAGCCTGGCAGCTCCCTGGCCAGCAACAGAA
TCCTGACCCTGCCCCAGAGAACCATCAGAGGCAAGAACAAGCACTGCTGGTCCACCTCCAAGAGCACCAGGC
GGAGCAGAGTGTCCGCCCTGAACATCGTGCGGAGCCAGAGGGGCCCCACCAGAATGTGCAGAAACATCTACG
 
ACCCCCTGCTGTGCTTCAAGCTGTTCTTCACCGACGAGATCATCAGCGAGATCGTGAAGTGGACCAACGCCGA
GATCAGCCTGAAGAGGCGGGAGAGCATGACCAGCGCCACCTTCAGAGACACCAACGAGGACGAGATCTACGC
CTTCTTCGGCATCCTGGTGATGACCGCCGTGAGAAAGGACAACCACATGAGCACCGACGACCTGTTCGACAGA
TCCCTGAGCATGGTGTACGTGTCCGTGATGAGCAGAGACAGATTCGACTTCCTGATCAGATGCCTGAGAATGG
ACGACAAGAGCATCAGACCCACCCTGCGGGAGAACGACGTGTTCACCCCCGTGCGGAAGATCTGGGACCTGT
TCATCCACCAGTGCATCCAGAACTACACCCCTGGCGCCCACCTGACCATCGATGAGCAGCTGCTGGGCTTCAG
AGGCAGATGCCCCTTCAGAGTGTACATCCCCAACAAGCCCAGCAAGTACGGCATCAAGATCCTGATGATGTGC
GACAGCGGCACCAAGTACATGATCAACGGCATGCCCTACCTGGGCAGAGGCACCCAGACAAACGGCGTGCCC
CTGGGCGAGTACTACGTGAAAGAACTGAGCAAGCCTGTGCATGGCAGCTGCAGGAACATCACCTGCGACAAC
TGGTTCACCAGCATCCCCCTGGCCAAGAACCTGCTGCAGGAACCCTACAAGCTGACCATCGTGGGCACCGTGC
GGAGCAACAAGCGGGAGATCCCAGAGGTGCTGAAGAACAGCAGATCCAGACCTGTGGGAACAAGCATGTTCT
GCTTCGACGGCCCCCTGACCCTGGTGTCCTACAAGCCCAAGCCCGCCAAGATGGTGTACCTGCTGTCCAGCTG
CGACGAGGACGCCAGCATCAACGAGAGCACCGGCAAGCCCCAGATGGTGATGTACTACAACCAGACCAAGGG
CGGCGTGGACACCCTGGACCAGATGTGCAGCGTGATGACCTGCAGCAGAAAGACCAACAGATGGCCCATGGC
CCTGCTGTACGGCATGATCAATATCGCCTGCATCAACAGCTTCATCATCTACAGCCACAACGTGTCCAGCAAG
GGCGAGAAGGTGCAGAGCCGGAAGAAATTCATGCGGAACCTGTACATGAGCCTGACCTCCAGCTTCATGAGA
AAGAGACTGGAAGCCCCCACCCTGAAGAGATACCTGCGGGACAACATCAGCAACATCCTGCCCAAGGAAGTG
CCAGGAACAAGCGACGACAGCACCGAGGAACCCGTGATGAAGAAGAGGACCTACTGCACCTACTGTCCCAGC
AAGATCAGAAGAAAGGCCAACGCCAGCTGCAAGAAATGCAAAAAAGTGATCTGCCGGGAGCACAACATCGA
CATGTGCCAGAGCTGTTTCTGA
 Note: The underlined part is the part encoding PBase.
Cas9-PBase N347A_R376E_D450N encoding DNA sequence(SEQ ID NO:135) 
ATGGACAAGAAGTACTCCATTGGGCTCGacATCGGCACAAACAGCGTCGGCTGGGCCGTCATTACGGACGAGT
ACAAGGTGCCGAGCAAAAAATTCAAAGTTCTGGGCAATACCGATCGCCACAGCATAAAGAAGAACCTCATTG
GCGCCCTCCTGTTCGACTCCGGGGAGACGGCCGAAGCCACGCGGCTCAAAAGAACAGCACGGCGCAGATATA
CCCGCAGAAAGAATCGGATCTGCTACCTGCAGGAGATCTTTAGTAATGAGATGGCTAAGGTGGATGACTCTTT
CTTCCATAGGCTGGAGGAGTCCTTTTTGGTGGAGGAGGATAAAAAGCACGAGCGCCACCCAATCTTTGGCAAT
ATCGTGGACGAGGTGGCGTACCATGAAAAGTACCCAACCATATATCATCTGAGGAAGAAGCTTGTAGACAGT
ACTGATAAGGCTGACTTGCGGTTGATCTATCTCGCGCTGGCGCATATGATCAAATTTCGGGGACACTTCCTCAT
CGAGGGGGACCTGAACCCAGACAACAGCGATGTCGACAAACTCTTTATCCAACTGGTTCAGACTTACAATCAG
CTTTTCGAAGAGAACCCGATCAACGCATCCGGAGTTGACGCCAAAGCAATCCTGAGCGCTAGGCTGTCCAAAT
CCCGGCGGCTCGAAAACCTCATCGCACAGCTCCCTGGGGAGAAGAAGAACGGCCTGTTTGGTAATCTTATCGC
CCTGTCACTCGGGCTGACCCCCAACTTTAAATCTAACTTCGACCTGGCCGAAGATGCCAAGCTTCAACTGAGC
AAAGACACCTACGATGATGATCTCGACAATCTGCTGGCCCAGATCGGCGACCAGTACGCAGACCTTTTTTTGG
CGGCAAAGAACCTGTCAGACGCCATTCTGCTGAGTGATATTCTGCGAGTGAACACGGAGATCACCAAAGCTCC
GCTGAGCGCTAGTATGATCAAGCGCTATGATGAGCACCACCAAGACTTGACTTTGCTGAAGGCCCTTGTCAGA
CAGCAACTGCCTGAGAAGTACAAGGAAATTTTCTTCGATCAGTCTAAAAATGGCTACGCCGGATACATTGACG
 
GCGGAGCAAGCCAGGAGGAATTTTACAAATTTATTAAGCCCATCTTGGAAAAAATGGACGGCACCGAGGAGC
TGCTGGTAAAGCTTAACAGAGAAGATCTGTTGCGCAAACAGCGCACTTTCGACAATGGAAGCATCCCCCACCA
GATTCACCTGGGCGAACTGCACGCTATCCTCAGGCGGCAAGAGGATTTCTACCCCTTTTTGAAAGATAACAGG
GAAAAGATTGAGAAAATCCTCACATTTCGGATACCCTACTATGTAGGCCCCCTCGCCCGGGGAAATTCCAGAT
TCGCGTGGATGACTCGCAAATCAGAAGAGACCATCACTCCCTGGAACTTCGAGGAAGTCGTGGATAAGGGGG
CCTCTGCCCAGTCCTTCATCGAAAGGATGACTAACTTTGATAAAAATCTGCCTAACGAAAAGGTGCTTCCTAA
ACACTCTCTGCTGTACGAGTACTTCACAGTTTATAACGAGCTCACCAAGGTCAAATACGTCACAGAAGGGATG
AGAAAGCCAGCATTCCTGTCTGGAGAGCAGAAGAAAGCTATCGTGGACCTCCTCTTCAAGACGAACCGGAAA
GTTACCGTGAAACAGCTCAAAGAAGACTATTTCAAAAAGATTGAATGTTTCGACTCTGTTGAAATCAGCGGAG
TGGAGGATCGCTTCAACGCATCCCTGGGAACGTATCACGATCTCCTGAAAATCATTAAAGACAAGGACTTCCT
GGACAATGAGGAGAACGAGGACATTCTTGAGGACATTGTCCTCACCCTTACGTTGTTTGAAGATAGGGAGATG
ATTGAAGAACGCTTGAAAACTTACGCTCATCTCTTCGACGACAAAGTCATGAAACAGCTCAAGAGGCGCCGAT
ATACAGGATGGGGGCGGCTGTCAAGAAAACTGATCAATGGGATCCGAGACAAGCAGAGTGGAAAGACAATC
CTGGATTTTCTTAAGTCCGATGGATTTGCCAACAGGAACTTCATGCAGTTGATCCATGATGACTCTCTCACCTT
TAAGGAGGACATCCAGAAAGCACAAGTTTCTGGCCAGGGGGACAGTCTTCACGAGCACATCGCTAATCTTGC
AGGTAGCCCAGCTATCAAAAAGGGAATACTGCAGACCGTTAAGGTCGTGGATGAACTCGTCAAAGTAATGGG
AAGGCATAAGCCCGAGAATATCGTTATCGAGATGGCCCGAGAGAACCAAACTACCCAGAAGGGACAGAAGA
ACAGTAGGGAAAGGATGAAGAGGATTGAAGAGGGTATAAAAGAACTGGGGTCCCAAATCCTTAAGGAACAC
CCAGTTGAAAACACCCAGCTTCAGAATGAGAAGCTCTACCTGTACTACCTGCAGAACGGCAGGGACATGTAC
GTGGATCAGGAACTGGACATCAATCGGCTCTCCGACTACGACGTGGATCATATCGTGCCCCAGTCTTTTCTCA
AAGATGATTCTATTGATAATAAAGTGTTGACAAGATCCGATAAAAATAGAGGGAAGAGTGATAACGTCCCCT
CAGAAGAAGTTGTCAAGAAAATGAAAAATTATTGGCGGCAGCTGCTGAACGCCAAACTGATCACACAACGGA
AGTTCGATAATCTGACTAAGGCTGAACGAGGTGGCCTGTCTGAGTTGGATAAAGCCGGCTTCATCAAAAGGCA
GCTTGTTGAGACACGCCAGATCACCAAGCACGTGGCCCAAATTCTCGATTCACGCATGAACACCAAGTACGAT
GAAAATGACAAACTGATTCGAGAGGTGAAAGTTATTACTCTGAAGTCTAAGCTGGTCTCAGATTTCAGAAAGG
ACTTTCAGTTTTATAAGGTGAGAGAGATCAACAATTACCACCATGCGCATGATGCCTACCTGAATGCAGTGGT
AGGCACTGCACTTATCAAAAAATATCCCAAGCTTGAATCTGAATTTGTTTACGGAGACTATAAAGTGTACGAT
GTTAGGAAAATGATCGCAAAGTCTGAGCAGGAAATAGGCAAGGCCACCGCTAAGTACTTCTTTTACAGCAAT
ATTATGAATTTTTTCAAGACCGAGATTACACTGGCCAATGGAGAGATTCGGAAGCGACCACTTATCGAAACAA
ACGGAGAAACAGGAGAAATCGTGTGGGACAAGGGTAGGGATTTCGCGACAGTCCGGAAGGTCCTGTCCATGC
CGCAGGTGAACATCGTTAAAAAGACCGAAGTACAGACCGGAGGCTTCTCCAAGGAAAGTATCCTCCCGAAAA
GGAACAGCGACAAGCTGATCGCACGCAAAAAAGATTGGGACCCCAAGAAATACGGCGGATTCGATTCTCCTA
CAGTCGCTTACAGTGTACTGGTTGTGGCCAAAGTGGAGAAAGGGAAGTCTAAAAAACTCAAAAGCGTCAAGG
AACTGCTGGGCATCACAATCATGGAGCGATCAAGCTTCGAAAAAAACCCCATCGACTTTCTCGAGGCGAAAG
GATATAAAGAGGTCAAAAAAGACCTCATCATTAAGCTTCCCAAGTACTCTCTCTTTGAGCTTGAAAACGGCCG
GAAACGAATGCTCGCTAGTGCGGGCGAGCTGCAGAAAGGTAACGAGCTGGCACTGCCCTCTAAATACGTTAA
TTTCTTGTATCTGGCCAGCCACTATGAAAAGCTCAAAGGGTCTCCCGAAGATAATGAGCAGAAGCAGCTGTTC
GTGGAACAACACAAACACTACCTTGATGAGATCATCGAGCAAATAAGCGAATTCTCCAAAAGAGTGATCCTC
 
GCCGACGCTAACCTCGATAAGGTGCTTTCTGCTTACAATAAGCACAGGGATAAGCCCATCAGGGAGCAGGCA
GAAAACATTATCCACTTGTTTACTCTGACCAACTTGGGCGCGCCTGCAGCCTTCAAGTACTTCGACACCACCAT
AGACAGAAAGCGGTACACCTCTACAAAGGAGGTCCTGGACGCCACACTGATTCATCAGTCAATTACGGGGCT
CTATGAAACAAGAATCGACCTCTCTCAGCTCGGTGGAGACAGCAGGGCTGACCCCAAGAAGAAGAGGAAGGT
GGGCGGAGGAGGGAGTGGCGGAGGCGGAAGCGGCGGCGGAGGCAGCGGCTCTAGCCTGGACGACGAGCACA
TCCTGAGCGCCCTGCTGCAGAGCGACGACGAACTGGTGGGCGAGGACAGCGACAGCGAGGTCAGCGACCACG
TGTCCGAGGACGACGTGCAGTCCGACACCGAGGAAGCCTTCATCGACGAGGTGCACGAAGTGCAGCCTACCA
GCAGCGGCTCCGAGATCCTGGACGAGCAGAACGTGATCGAGCAGCCTGGCAGCTCCCTGGCCAGCAACAGAA
TCCTGACCCTGCCCCAGAGAACCATCAGAGGCAAGAACAAGCACTGCTGGTCCACCTCCAAGAGCACCAGGC
GGAGCAGAGTGTCCGCCCTGAACATCGTGCGGAGCCAGAGGGGCCCCACCAGAATGTGCAGAAACATCTACG
ACCCCCTGCTGTGCTTCAAGCTGTTCTTCACCGACGAGATCATCAGCGAGATCGTGAAGTGGACCAACGCCGA
GATCAGCCTGAAGAGGCGGGAGAGCATGACCAGCGCCACCTTCAGAGACACCAACGAGGACGAGATCTACGC
CTTCTTCGGCATCCTGGTGATGACCGCCGTGAGAAAGGACAACCACATGAGCACCGACGACCTGTTCGACAGA
TCCCTGAGCATGGTGTACGTGTCCGTGATGAGCAGAGACAGATTCGACTTCCTGATCAGATGCCTGAGAATGG
ACGACAAGAGCATCAGACCCACCCTGCGGGAGAACGACGTGTTCACCCCCGTGCGGAAGATCTGGGACCTGT
TCATCCACCAGTGCATCCAGAACTACACCCCTGGCGCCCACCTGACCATCGATGAGCAGCTGCTGGGCTTCAG
AGGCAGATGCCCCTTCAGAGTGTACATCCCCAACAAGCCCAGCAAGTACGGCATCAAGATCCTGATGATGTGC
GACAGCGGCACCAAGTACATGATCAACGGCATGCCCTACCTGGGCAGAGGCACCCAGACAAACGGCGTGCCC
CTGGGCGAGTACTACGTGAAAGAACTGAGCAAGCCTGTGCATGGCAGCTGCAGGAACATCACCTGCGACgcCT
GGTTCACCAGCATCCCCCTGGCCAAGAACCTGCTGCAGGAACCCTACAAGCTGACCATCGTGGGCACCGTGCG
GAGCAACAAGgagGAGATCCCAGAGGTGCTGAAGAACAGCAGATCCAGACCTGTGGGAACAAGCATGTTCTGC
TTCGACGGCCCCCTGACCCTGGTGTCCTACAAGCCCAAGCCCGCCAAGATGGTGTACCTGCTGTCCAGCTGCG
ACGAGGACGCCAGCATCAACGAGAGCACCGGCAAGCCCCAGATGGTGATGTACTACAACCAGACCAAGGGCG
GCGTGGACACCCTGaACCAGATGTGCAGCGTGATGACCTGCAGCAGAAAGACCAACAGATGGCCCATGGCCCT
GCTGTACGGCATGATCAATATCGCCTGCATCAACAGCTTCATCATCTACAGCCACAACGTGTCCAGCAAGGGC
GAGAAGGTGCAGAGCCGGAAGAAATTCATGCGGAACCTGTACATGAGCCTGACCTCCAGCTTCATGAGAAAG
AGACTGGAAGCCCCCACCCTGAAGAGATACCTGCGGGACAACATCAGCAACATCCTGCCCAAGGAAGTGCCA
GGAACAAGCGACGACAGCACCGAGGAACCCGTGATGAAGAAGAGGACCTACTGCACCTACTGTCCCAGCAAG
ATCAGAAGAAAGGCCAACGCCAGCTGCAAGAAATGCAAAAAAGTGATCTGCCGGGAGCACAACATCGACAT
GTGCCAGAGCTGTTTCTGA
 Note: The underlined part is the part encoding PBase.
Cas9-PBase N347E_R376E_D450N encoding DNA sequence(SEQ ID NO:136) 
ATGGACAAGAAGTACTCCATTGGGCTCGacATCGGCACAAACAGCGTCGGCTGGGCCGTCATTACGGACGAGT
ACAAGGTGCCGAGCAAAAAATTCAAAGTTCTGGGCAATACCGATCGCCACAGCATAAAGAAGAACCTCATTG
GCGCCCTCCTGTTCGACTCCGGGGAGACGGCCGAAGCCACGCGGCTCAAAAGAACAGCACGGCGCAGATATA
CCCGCAGAAAGAATCGGATCTGCTACCTGCAGGAGATCTTTAGTAATGAGATGGCTAAGGTGGATGACTCTTT
CTTCCATAGGCTGGAGGAGTCCTTTTTGGTGGAGGAGGATAAAAAGCACGAGCGCCACCCAATCTTTGGCAAT
 
ATCGTGGACGAGGTGGCGTACCATGAAAAGTACCCAACCATATATCATCTGAGGAAGAAGCTTGTAGACAGT
ACTGATAAGGCTGACTTGCGGTTGATCTATCTCGCGCTGGCGCATATGATCAAATTTCGGGGACACTTCCTCAT
CGAGGGGGACCTGAACCCAGACAACAGCGATGTCGACAAACTCTTTATCCAACTGGTTCAGACTTACAATCAG
CTTTTCGAAGAGAACCCGATCAACGCATCCGGAGTTGACGCCAAAGCAATCCTGAGCGCTAGGCTGTCCAAAT
CCCGGCGGCTCGAAAACCTCATCGCACAGCTCCCTGGGGAGAAGAAGAACGGCCTGTTTGGTAATCTTATCGC
CCTGTCACTCGGGCTGACCCCCAACTTTAAATCTAACTTCGACCTGGCCGAAGATGCCAAGCTTCAACTGAGC
AAAGACACCTACGATGATGATCTCGACAATCTGCTGGCCCAGATCGGCGACCAGTACGCAGACCTTTTTTTGG
CGGCAAAGAACCTGTCAGACGCCATTCTGCTGAGTGATATTCTGCGAGTGAACACGGAGATCACCAAAGCTCC
GCTGAGCGCTAGTATGATCAAGCGCTATGATGAGCACCACCAAGACTTGACTTTGCTGAAGGCCCTTGTCAGA
CAGCAACTGCCTGAGAAGTACAAGGAAATTTTCTTCGATCAGTCTAAAAATGGCTACGCCGGATACATTGACG
GCGGAGCAAGCCAGGAGGAATTTTACAAATTTATTAAGCCCATCTTGGAAAAAATGGACGGCACCGAGGAGC
TGCTGGTAAAGCTTAACAGAGAAGATCTGTTGCGCAAACAGCGCACTTTCGACAATGGAAGCATCCCCCACCA
GATTCACCTGGGCGAACTGCACGCTATCCTCAGGCGGCAAGAGGATTTCTACCCCTTTTTGAAAGATAACAGG
GAAAAGATTGAGAAAATCCTCACATTTCGGATACCCTACTATGTAGGCCCCCTCGCCCGGGGAAATTCCAGAT
TCGCGTGGATGACTCGCAAATCAGAAGAGACCATCACTCCCTGGAACTTCGAGGAAGTCGTGGATAAGGGGG
CCTCTGCCCAGTCCTTCATCGAAAGGATGACTAACTTTGATAAAAATCTGCCTAACGAAAAGGTGCTTCCTAA
ACACTCTCTGCTGTACGAGTACTTCACAGTTTATAACGAGCTCACCAAGGTCAAATACGTCACAGAAGGGATG
AGAAAGCCAGCATTCCTGTCTGGAGAGCAGAAGAAAGCTATCGTGGACCTCCTCTTCAAGACGAACCGGAAA
GTTACCGTGAAACAGCTCAAAGAAGACTATTTCAAAAAGATTGAATGTTTCGACTCTGTTGAAATCAGCGGAG
TGGAGGATCGCTTCAACGCATCCCTGGGAACGTATCACGATCTCCTGAAAATCATTAAAGACAAGGACTTCCT
GGACAATGAGGAGAACGAGGACATTCTTGAGGACATTGTCCTCACCCTTACGTTGTTTGAAGATAGGGAGATG
ATTGAAGAACGCTTGAAAACTTACGCTCATCTCTTCGACGACAAAGTCATGAAACAGCTCAAGAGGCGCCGAT
ATACAGGATGGGGGCGGCTGTCAAGAAAACTGATCAATGGGATCCGAGACAAGCAGAGTGGAAAGACAATC
CTGGATTTTCTTAAGTCCGATGGATTTGCCAACAGGAACTTCATGCAGTTGATCCATGATGACTCTCTCACCTT
TAAGGAGGACATCCAGAAAGCACAAGTTTCTGGCCAGGGGGACAGTCTTCACGAGCACATCGCTAATCTTGC
AGGTAGCCCAGCTATCAAAAAGGGAATACTGCAGACCGTTAAGGTCGTGGATGAACTCGTCAAAGTAATGGG
AAGGCATAAGCCCGAGAATATCGTTATCGAGATGGCCCGAGAGAACCAAACTACCCAGAAGGGACAGAAGA
ACAGTAGGGAAAGGATGAAGAGGATTGAAGAGGGTATAAAAGAACTGGGGTCCCAAATCCTTAAGGAACAC
CCAGTTGAAAACACCCAGCTTCAGAATGAGAAGCTCTACCTGTACTACCTGCAGAACGGCAGGGACATGTAC
GTGGATCAGGAACTGGACATCAATCGGCTCTCCGACTACGACGTGGATCATATCGTGCCCCAGTCTTTTCTCA
AAGATGATTCTATTGATAATAAAGTGTTGACAAGATCCGATAAAAATAGAGGGAAGAGTGATAACGTCCCCT
CAGAAGAAGTTGTCAAGAAAATGAAAAATTATTGGCGGCAGCTGCTGAACGCCAAACTGATCACACAACGGA
AGTTCGATAATCTGACTAAGGCTGAACGAGGTGGCCTGTCTGAGTTGGATAAAGCCGGCTTCATCAAAAGGCA
GCTTGTTGAGACACGCCAGATCACCAAGCACGTGGCCCAAATTCTCGATTCACGCATGAACACCAAGTACGAT
GAAAATGACAAACTGATTCGAGAGGTGAAAGTTATTACTCTGAAGTCTAAGCTGGTCTCAGATTTCAGAAAGG
ACTTTCAGTTTTATAAGGTGAGAGAGATCAACAATTACCACCATGCGCATGATGCCTACCTGAATGCAGTGGT
AGGCACTGCACTTATCAAAAAATATCCCAAGCTTGAATCTGAATTTGTTTACGGAGACTATAAAGTGTACGAT
GTTAGGAAAATGATCGCAAAGTCTGAGCAGGAAATAGGCAAGGCCACCGCTAAGTACTTCTTTTACAGCAAT
 
ATTATGAATTTTTTCAAGACCGAGATTACACTGGCCAATGGAGAGATTCGGAAGCGACCACTTATCGAAACAA
ACGGAGAAACAGGAGAAATCGTGTGGGACAAGGGTAGGGATTTCGCGACAGTCCGGAAGGTCCTGTCCATGC
CGCAGGTGAACATCGTTAAAAAGACCGAAGTACAGACCGGAGGCTTCTCCAAGGAAAGTATCCTCCCGAAAA
GGAACAGCGACAAGCTGATCGCACGCAAAAAAGATTGGGACCCCAAGAAATACGGCGGATTCGATTCTCCTA
CAGTCGCTTACAGTGTACTGGTTGTGGCCAAAGTGGAGAAAGGGAAGTCTAAAAAACTCAAAAGCGTCAAGG
AACTGCTGGGCATCACAATCATGGAGCGATCAAGCTTCGAAAAAAACCCCATCGACTTTCTCGAGGCGAAAG
GATATAAAGAGGTCAAAAAAGACCTCATCATTAAGCTTCCCAAGTACTCTCTCTTTGAGCTTGAAAACGGCCG
GAAACGAATGCTCGCTAGTGCGGGCGAGCTGCAGAAAGGTAACGAGCTGGCACTGCCCTCTAAATACGTTAA
TTTCTTGTATCTGGCCAGCCACTATGAAAAGCTCAAAGGGTCTCCCGAAGATAATGAGCAGAAGCAGCTGTTC
GTGGAACAACACAAACACTACCTTGATGAGATCATCGAGCAAATAAGCGAATTCTCCAAAAGAGTGATCCTC
GCCGACGCTAACCTCGATAAGGTGCTTTCTGCTTACAATAAGCACAGGGATAAGCCCATCAGGGAGCAGGCA
GAAAACATTATCCACTTGTTTACTCTGACCAACTTGGGCGCGCCTGCAGCCTTCAAGTACTTCGACACCACCAT
AGACAGAAAGCGGTACACCTCTACAAAGGAGGTCCTGGACGCCACACTGATTCATCAGTCAATTACGGGGCT
CTATGAAACAAGAATCGACCTCTCTCAGCTCGGTGGAGACAGCAGGGCTGACCCCAAGAAGAAGAGGAAGGT
GGGCGGAGGAGGGAGTGGCGGAGGCGGAAGCGGCGGCGGAGGCAGCGGCTCTAGCCTGGACGACGAGCACA
TCCTGAGCGCCCTGCTGCAGAGCGACGACGAACTGGTGGGCGAGGACAGCGACAGCGAGGTCAGCGACCACG
TGTCCGAGGACGACGTGCAGTCCGACACCGAGGAAGCCTTCATCGACGAGGTGCACGAAGTGCAGCCTACCA
GCAGCGGCTCCGAGATCCTGGACGAGCAGAACGTGATCGAGCAGCCTGGCAGCTCCCTGGCCAGCAACAGAA
TCCTGACCCTGCCCCAGAGAACCATCAGAGGCAAGAACAAGCACTGCTGGTCCACCTCCAAGAGCACCAGGC
GGAGCAGAGTGTCCGCCCTGAACATCGTGCGGAGCCAGAGGGGCCCCACCAGAATGTGCAGAAACATCTACG
ACCCCCTGCTGTGCTTCAAGCTGTTCTTCACCGACGAGATCATCAGCGAGATCGTGAAGTGGACCAACGCCGA
GATCAGCCTGAAGAGGCGGGAGAGCATGACCAGCGCCACCTTCAGAGACACCAACGAGGACGAGATCTACGC
CTTCTTCGGCATCCTGGTGATGACCGCCGTGAGAAAGGACAACCACATGAGCACCGACGACCTGTTCGACAGA
TCCCTGAGCATGGTGTACGTGTCCGTGATGAGCAGAGACAGATTCGACTTCCTGATCAGATGCCTGAGAATGG
ACGACAAGAGCATCAGACCCACCCTGCGGGAGAACGACGTGTTCACCCCCGTGCGGAAGATCTGGGACCTGT
TCATCCACCAGTGCATCCAGAACTACACCCCTGGCGCCCACCTGACCATCGATGAGCAGCTGCTGGGCTTCAG
AGGCAGATGCCCCTTCAGAGTGTACATCCCCAACAAGCCCAGCAAGTACGGCATCAAGATCCTGATGATGTGC
GACAGCGGCACCAAGTACATGATCAACGGCATGCCCTACCTGGGCAGAGGCACCCAGACAAACGGCGTGCCC
CTGGGCGAGTACTACGTGAAAGAACTGAGCAAGCCTGTGCATGGCAGCTGCAGGAACATCACCTGCGACgagT
GGTTCACCAGCATCCCCCTGGCCAAGAACCTGCTGCAGGAACCCTACAAGCTGACCATCGTGGGCACCGTGCG
GAGCAACAAGgaGGAGATCCCAGAGGTGCTGAAGAACAGCAGATCCAGACCTGTGGGAACAAGCATGTTCTG
CTTCGACGGCCCCCTGACCCTGGTGTCCTACAAGCCCAAGCCCGCCAAGATGGTGTACCTGCTGTCCAGCTGC
GACGAGGACGCCAGCATCAACGAGAGCACCGGCAAGCCCCAGATGGTGATGTACTACAACCAGACCAAGGGC
GGCGTGGACACCCTGaACCAGATGTGCAGCGTGATGACCTGCAGCAGAAAGACCAACAGATGGCCCATGGCC
CTGCTGTACGGCATGATCAATATCGCCTGCATCAACAGCTTCATCATCTACAGCCACAACGTGTCCAGCAAGG
GCGAGAAGGTGCAGAGCCGGAAGAAATTCATGCGGAACCTGTACATGAGCCTGACCTCCAGCTTCATGAGAA
AGAGACTGGAAGCCCCCACCCTGAAGAGATACCTGCGGGACAACATCAGCAACATCCTGCCCAAGGAAGTGC
CAGGAACAAGCGACGACAGCACCGAGGAACCCGTGATGAAGAAGAGGACCTACTGCACCTACTGTCCCAGCA
 
AGATCAGAAGAAAGGCCAACGCCAGCTGCAAGAAATGCAAAAAAGTGATCTGCCGGGAGCACAACATCGAC
ATGTGCCAGAGCTGTTTCTGA
 Note: The underlined part is the part encoding PBase.
Cas9-PBase R315A_N347E_D450N encoding DNA sequence(SEQ ID NO:137) 
ATGGACAAGAAGTACTCCATTGGGCTCGacATCGGCACAAACAGCGTCGGCTGGGCCGTCATTACGGACGAGT
ACAAGGTGCCGAGCAAAAAATTCAAAGTTCTGGGCAATACCGATCGCCACAGCATAAAGAAGAACCTCATTG
GCGCCCTCCTGTTCGACTCCGGGGAGACGGCCGAAGCCACGCGGCTCAAAAGAACAGCACGGCGCAGATATA
CCCGCAGAAAGAATCGGATCTGCTACCTGCAGGAGATCTTTAGTAATGAGATGGCTAAGGTGGATGACTCTTT
CTTCCATAGGCTGGAGGAGTCCTTTTTGGTGGAGGAGGATAAAAAGCACGAGCGCCACCCAATCTTTGGCAAT
ATCGTGGACGAGGTGGCGTACCATGAAAAGTACCCAACCATATATCATCTGAGGAAGAAGCTTGTAGACAGT
ACTGATAAGGCTGACTTGCGGTTGATCTATCTCGCGCTGGCGCATATGATCAAATTTCGGGGACACTTCCTCAT
CGAGGGGGACCTGAACCCAGACAACAGCGATGTCGACAAACTCTTTATCCAACTGGTTCAGACTTACAATCAG
CTTTTCGAAGAGAACCCGATCAACGCATCCGGAGTTGACGCCAAAGCAATCCTGAGCGCTAGGCTGTCCAAAT
CCCGGCGGCTCGAAAACCTCATCGCACAGCTCCCTGGGGAGAAGAAGAACGGCCTGTTTGGTAATCTTATCGC
CCTGTCACTCGGGCTGACCCCCAACTTTAAATCTAACTTCGACCTGGCCGAAGATGCCAAGCTTCAACTGAGC
AAAGACACCTACGATGATGATCTCGACAATCTGCTGGCCCAGATCGGCGACCAGTACGCAGACCTTTTTTTGG
CGGCAAAGAACCTGTCAGACGCCATTCTGCTGAGTGATATTCTGCGAGTGAACACGGAGATCACCAAAGCTCC
GCTGAGCGCTAGTATGATCAAGCGCTATGATGAGCACCACCAAGACTTGACTTTGCTGAAGGCCCTTGTCAGA
CAGCAACTGCCTGAGAAGTACAAGGAAATTTTCTTCGATCAGTCTAAAAATGGCTACGCCGGATACATTGACG
GCGGAGCAAGCCAGGAGGAATTTTACAAATTTATTAAGCCCATCTTGGAAAAAATGGACGGCACCGAGGAGC
TGCTGGTAAAGCTTAACAGAGAAGATCTGTTGCGCAAACAGCGCACTTTCGACAATGGAAGCATCCCCCACCA
GATTCACCTGGGCGAACTGCACGCTATCCTCAGGCGGCAAGAGGATTTCTACCCCTTTTTGAAAGATAACAGG
GAAAAGATTGAGAAAATCCTCACATTTCGGATACCCTACTATGTAGGCCCCCTCGCCCGGGGAAATTCCAGAT
TCGCGTGGATGACTCGCAAATCAGAAGAGACCATCACTCCCTGGAACTTCGAGGAAGTCGTGGATAAGGGGG
CCTCTGCCCAGTCCTTCATCGAAAGGATGACTAACTTTGATAAAAATCTGCCTAACGAAAAGGTGCTTCCTAA
ACACTCTCTGCTGTACGAGTACTTCACAGTTTATAACGAGCTCACCAAGGTCAAATACGTCACAGAAGGGATG
AGAAAGCCAGCATTCCTGTCTGGAGAGCAGAAGAAAGCTATCGTGGACCTCCTCTTCAAGACGAACCGGAAA
GTTACCGTGAAACAGCTCAAAGAAGACTATTTCAAAAAGATTGAATGTTTCGACTCTGTTGAAATCAGCGGAG
TGGAGGATCGCTTCAACGCATCCCTGGGAACGTATCACGATCTCCTGAAAATCATTAAAGACAAGGACTTCCT
GGACAATGAGGAGAACGAGGACATTCTTGAGGACATTGTCCTCACCCTTACGTTGTTTGAAGATAGGGAGATG
ATTGAAGAACGCTTGAAAACTTACGCTCATCTCTTCGACGACAAAGTCATGAAACAGCTCAAGAGGCGCCGAT
ATACAGGATGGGGGCGGCTGTCAAGAAAACTGATCAATGGGATCCGAGACAAGCAGAGTGGAAAGACAATC
CTGGATTTTCTTAAGTCCGATGGATTTGCCAACAGGAACTTCATGCAGTTGATCCATGATGACTCTCTCACCTT
TAAGGAGGACATCCAGAAAGCACAAGTTTCTGGCCAGGGGGACAGTCTTCACGAGCACATCGCTAATCTTGC
AGGTAGCCCAGCTATCAAAAAGGGAATACTGCAGACCGTTAAGGTCGTGGATGAACTCGTCAAAGTAATGGG
AAGGCATAAGCCCGAGAATATCGTTATCGAGATGGCCCGAGAGAACCAAACTACCCAGAAGGGACAGAAGA
ACAGTAGGGAAAGGATGAAGAGGATTGAAGAGGGTATAAAAGAACTGGGGTCCCAAATCCTTAAGGAACAC
 
CCAGTTGAAAACACCCAGCTTCAGAATGAGAAGCTCTACCTGTACTACCTGCAGAACGGCAGGGACATGTAC
GTGGATCAGGAACTGGACATCAATCGGCTCTCCGACTACGACGTGGATCATATCGTGCCCCAGTCTTTTCTCA
AAGATGATTCTATTGATAATAAAGTGTTGACAAGATCCGATAAAAATAGAGGGAAGAGTGATAACGTCCCCT
CAGAAGAAGTTGTCAAGAAAATGAAAAATTATTGGCGGCAGCTGCTGAACGCCAAACTGATCACACAACGGA
AGTTCGATAATCTGACTAAGGCTGAACGAGGTGGCCTGTCTGAGTTGGATAAAGCCGGCTTCATCAAAAGGCA
GCTTGTTGAGACACGCCAGATCACCAAGCACGTGGCCCAAATTCTCGATTCACGCATGAACACCAAGTACGAT
GAAAATGACAAACTGATTCGAGAGGTGAAAGTTATTACTCTGAAGTCTAAGCTGGTCTCAGATTTCAGAAAGG
ACTTTCAGTTTTATAAGGTGAGAGAGATCAACAATTACCACCATGCGCATGATGCCTACCTGAATGCAGTGGT
AGGCACTGCACTTATCAAAAAATATCCCAAGCTTGAATCTGAATTTGTTTACGGAGACTATAAAGTGTACGAT
GTTAGGAAAATGATCGCAAAGTCTGAGCAGGAAATAGGCAAGGCCACCGCTAAGTACTTCTTTTACAGCAAT
ATTATGAATTTTTTCAAGACCGAGATTACACTGGCCAATGGAGAGATTCGGAAGCGACCACTTATCGAAACAA
ACGGAGAAACAGGAGAAATCGTGTGGGACAAGGGTAGGGATTTCGCGACAGTCCGGAAGGTCCTGTCCATGC
CGCAGGTGAACATCGTTAAAAAGACCGAAGTACAGACCGGAGGCTTCTCCAAGGAAAGTATCCTCCCGAAAA
GGAACAGCGACAAGCTGATCGCACGCAAAAAAGATTGGGACCCCAAGAAATACGGCGGATTCGATTCTCCTA
CAGTCGCTTACAGTGTACTGGTTGTGGCCAAAGTGGAGAAAGGGAAGTCTAAAAAACTCAAAAGCGTCAAGG
AACTGCTGGGCATCACAATCATGGAGCGATCAAGCTTCGAAAAAAACCCCATCGACTTTCTCGAGGCGAAAG
GATATAAAGAGGTCAAAAAAGACCTCATCATTAAGCTTCCCAAGTACTCTCTCTTTGAGCTTGAAAACGGCCG
GAAACGAATGCTCGCTAGTGCGGGCGAGCTGCAGAAAGGTAACGAGCTGGCACTGCCCTCTAAATACGTTAA
TTTCTTGTATCTGGCCAGCCACTATGAAAAGCTCAAAGGGTCTCCCGAAGATAATGAGCAGAAGCAGCTGTTC
GTGGAACAACACAAACACTACCTTGATGAGATCATCGAGCAAATAAGCGAATTCTCCAAAAGAGTGATCCTC
GCCGACGCTAACCTCGATAAGGTGCTTTCTGCTTACAATAAGCACAGGGATAAGCCCATCAGGGAGCAGGCA
GAAAACATTATCCACTTGTTTACTCTGACCAACTTGGGCGCGCCTGCAGCCTTCAAGTACTTCGACACCACCAT
AGACAGAAAGCGGTACACCTCTACAAAGGAGGTCCTGGACGCCACACTGATTCATCAGTCAATTACGGGGCT
CTATGAAACAAGAATCGACCTCTCTCAGCTCGGTGGAGACAGCAGGGCTGACCCCAAGAAGAAGAGGAAGGT
GGGCGGAGGAGGGAGTGGCGGAGGCGGAAGCGGCGGCGGAGGCAGCGGCTCTAGCCTGGACGACGAGCACA
TCCTGAGCGCCCTGCTGCAGAGCGACGACGAACTGGTGGGCGAGGACAGCGACAGCGAGGTCAGCGACCACG
TGTCCGAGGACGACGTGCAGTCCGACACCGAGGAAGCCTTCATCGACGAGGTGCACGAAGTGCAGCCTACCA
GCAGCGGCTCCGAGATCCTGGACGAGCAGAACGTGATCGAGCAGCCTGGCAGCTCCCTGGCCAGCAACAGAA
TCCTGACCCTGCCCCAGAGAACCATCAGAGGCAAGAACAAGCACTGCTGGTCCACCTCCAAGAGCACCAGGC
GGAGCAGAGTGTCCGCCCTGAACATCGTGCGGAGCCAGAGGGGCCCCACCAGAATGTGCAGAAACATCTACG
ACCCCCTGCTGTGCTTCAAGCTGTTCTTCACCGACGAGATCATCAGCGAGATCGTGAAGTGGACCAACGCCGA
GATCAGCCTGAAGAGGCGGGAGAGCATGACCAGCGCCACCTTCAGAGACACCAACGAGGACGAGATCTACGC
CTTCTTCGGCATCCTGGTGATGACCGCCGTGAGAAAGGACAACCACATGAGCACCGACGACCTGTTCGACAGA
TCCCTGAGCATGGTGTACGTGTCCGTGATGAGCAGAGACAGATTCGACTTCCTGATCAGATGCCTGAGAATGG
ACGACAAGAGCATCAGACCCACCCTGCGGGAGAACGACGTGTTCACCCCCGTGCGGAAGATCTGGGACCTGT
TCATCCACCAGTGCATCCAGAACTACACCCCTGGCGCCCACCTGACCATCGATGAGCAGCTGCTGGGCTTCAG
AGGCAGATGCCCCTTCAGAGTGTACATCCCCAACAAGCCCAGCAAGTACGGCATCAAGATCCTGATGATGTGC
GACAGCGGCACCAAGTACATGATCAACGGCATGCCCTACCTGGGCgccGGCACCCAGACAAACGGCGTGCCCC
 
TGGGCGAGTACTACGTGAAAGAACTGAGCAAGCCTGTGCATGGCAGCTGCAGGAACATCACCTGCGACgagTG
GTTCACCAGCATCCCCCTGGCCAAGAACCTGCTGCAGGAACCCTACAAGCTGACCATCGTGGGCACCGTGCGG
AGCAACAAGCGGGAGATCCCAGAGGTGCTGAAGAACAGCAGATCCAGACCTGTGGGAACAAGCATGTTCTGC
TTCGACGGCCCCCTGACCCTGGTGTCCTACAAGCCCAAGCCCGCCAAGATGGTGTACCTGCTGTCCAGCTGCG
ACGAGGACGCCAGCATCAACGAGAGCACCGGCAAGCCCCAGATGGTGATGTACTACAACCAGACCAAGGGCG
GCGTGGACACCCTGaACCAGATGTGCAGCGTGATGACCTGCAGCAGAAAGACCAACAGATGGCCCATGGCCCT
GCTGTACGGCATGATCAATATCGCCTGCATCAACAGCTTCATCATCTACAGCCACAACGTGTCCAGCAAGGGC
GAGAAGGTGCAGAGCCGGAAGAAATTCATGCGGAACCTGTACATGAGCCTGACCTCCAGCTTCATGAGAAAG
AGACTGGAAGCCCCCACCCTGAAGAGATACCTGCGGGACAACATCAGCAACATCCTGCCCAAGGAAGTGCCA
GGAACAAGCGACGACAGCACCGAGGAACCCGTGATGAAGAAGAGGACCTACTGCACCTACTGTCCCAGCAAG
ATCAGAAGAAAGGCCAACGCCAGCTGCAAGAAATGCAAAAAAGTGATCTGCCGGGAGCACAACATCGACAT
GTGCCAGAGCTGTTTCTGA
 Note: The underlined part is the part encoding PBase.
AAVS1-PB reporter-BSD-tdTomato plasmid sequence(SEQ ID NO:15) 
ctagctagtcctgcaggtttaaacgaattcgccctttgctttctctgaccagcattctctcccctgggcctgtgccgctttctgtctgcagcttgtggcctgggtcacctctacggctggccc
agatccttccctgccgcctccttcaggttccgtcttcctccactccctcttccccttgctctctgctgtgttgctgcccaaggatgctctttccggagcacttccttctcggcgctgcaccacg
tgatgtcctctgagcggatcctccccgtgtctgggtcctctccgggcatctctcctccctcacccaaccccatgccgtcttcactcgctgggttcccttttccttctccttctggggcctgtg
ccatctctcgtttcttaggatggccttctccgacggatgtctcccttgcgtcccgcctccccttcttgtaggcctgcatcatcaccgtttttctggacaaccccaaagtaccccgtctccctg
gctttagccacctctccatcctcttgctttctttgcctggacaccccgttctcctgtggattcgggtcacctctcactcctttcatttgggcagctcccctaccccccttacctctctagtctgtg
ctagctcttccagccccctgtcatggcatcttccaggggtccgagagctcagctagtcttcttcctccaacccgggcccctatgtccacttcaggacagcatgtttgctgcctccagggat
cctgtgtccccgagctgggaccaccttatattcccagggccggttaatgtggctctggttctgggtacttttatctgtcccctccaccccacagtggggcaagcttctgacctcttctcttcc
tcccacagggcctcgagagatctggcagcggagagggcagaggaagtcttctaacatgcggtgacgtggaggagaatcccggccctaggctcgagatgaccgagtacaagccca
cggtgcgcctcgccacccgcgacgacgtccccagggccgtacgcaccctcgccgccgcgttcgccgactaccccgccacgcgccacaccgtcgatccggaccgccacatcgag
cgggtcaccgagctgcaagaactcttcctcacgcgcgtcgggctcgacatcggcaaggtgtgggtcgcggacgacggcgccgcggtggcggtctggaccacgccggagagcgt
cgaagcgggggcggtgttcgccgagatcggcccgcgcatggccgagttgagcggttcccggctggccgcgcagcaacagatggaaggcctcctggcgccgcaccggcccaag
gagcccgcgtggttcctggccaccgtcggcgtctcgcccgaccaccagggcaagggtctgggcagcgccgtcgtgctccccggagtggaggcggccgagcgcgccggggtgc
ccgccttcctggagacctccgcgccccgcaacctccccttctacgagcggctcggcttcaccgtcaccgccgacgtcgaggtgcccgaaggaccgcgcacctggtgcatgacccg
caagcccggtgcctgatctagagggcccgtttaaacccgctgatcagcctcgactgtgccttctagttgccagccatctgttgtttgcccctcccccgtgccttccttgaccctggaaggt
gccactcccactgtcctttcctaataaaatgaggaaattgcatcgcattgtctgagtaggtgtcattctattctggggggtggggtggggcaggacagcaagggggaggattgggaag
acaatagcaggcatgctggggatgcggtgggctctatgggtctcgacattgattattgactagtTATTAATAGTAATCAATTACGGGGTCATTAGTTC
ATAGCCCATATATGGAGTTCCGCGTTACATAACTTACGGTAAATGGCCCGCCTGGCTGACCGCCCAACGACCC
CCGCCCATTGACGTCAATAATGACGTATGTTCCCATAGTAACGCCAATAGGGACTTTCCATTGACGTCAATGG
GTGGACTATTTACGGTAAACTGCCCACTTGGCAGTACATCAAGTGTATCATATGCCAAGTACGCCCCCTATTG
ACGTCAATGACGGTAAATGGCCCGCCTGGCATTATGCCCAGTACATGACCTTATGGGACTTTCCTACTTGGCA
GTACATCTACGTATTAGTCATCGCTATTACCATGGGTCGAGGTGAGCCCCACGTTCTGCTTCACTCTCCCCATC
TCCCCCCCCTCCCCACCCCCAATTTTGTATTTATTTATTTTTTAATTATTTTGTGCAGCGATGGGGGCGGGGGG
GGGGGGGGCGCGCGCCAGGCGGGGCGGGGCGGGGCGAGGGGCGGGGCGGGGCGAGGCGGAGAGGTGCGGCG
 
GCAGCCAATCAGAGCGGCGCGCTCCGAAAGTTTCCTTTTATGGCGAGGCGGCGGCGGCGGCCCTATAAA
AAGCGAAGCGCGCGGCGGGCGGGAGTCGCTGCGTTGCCTTCGCCCCGTGCCCCGCTCCGCGCCGCCTCGCGCC
GCCCGCCCCGGCTCTGACTGACCGCGTTACTCCCACAGGTGAGCGGGCGGGACGGCCCTTCTCCTCCGGGCTG
TAATTAGCGCTTGGTTTAATGACGGCTCGTTTCTTTTCTGTGGCTGCGTGAAAGCCTTAAAGGGCTCCGGGAGG
GCCCTTTGTGCGGGGGGGAGCGGCTCGGGGGGTGCGTGCGTGTGTGTGTGCGTGGGGAGCGCCGCGTGCGGC
CCGCGCTGCCCGGCGGCTGTGAGCGCTGCGGGCGCGGCGCGGGGCTTTGTGCGCTCCGCGTGTGCGCGAGGG
GAGCGCGGCCGGGGGCGGTGCCCCGCGGTGCGGGGGGGCTGCGAGGGGAACAAAGGCTGCGTGCGGGGTGT
GTGCGTGGGGGGGTGAGCAGGGGGTGTGGGCGCGGCGGTCGGGCTGTAACCCCCCCCTGCACCCCCCTCCCC
GAGTTGCTGAGCACGGCCCGGCTTCGGGTGCGGGGCTCCGTGCGGGGCGTGGCGCGGGGCTCGCCGTGCCGG
GCGGGGGGTGGCGGCAGGTGGGGGTGCCGGGCGGGGCGGGGCCGCCTCGGGCCGGGGAGGGCTCGGGGGAG
GGGCGCGGCGGCCCCGGAGCGCCGGCGGCTGTCGAGGCGCGGCGAGCCGCAGCCATTGCCTTTTATGGTAAT
CGTGCGAGAGGGCGCAGGGACTTCCTTTGTCCCAAATCTGGCGGAGCCGAAATCTGGGAGGCGCCGCCGCAC
CCCCTCTAGCGGGCGCGGGCGAAGCGGTGCGGCGCCGGCAGGAAGGAAATGGGCGGGGAGGGCCTTCGTGCG
TCGCCGCGCCGCCGTCCCCTTCTCCATCTCCAGCCTCGGGGCTGCCGCAGGGGGACGGCTGCCTTCGGGGGGG
ACGGGGCAGGGCGGGGTTCGGCTTCTGGCGTGTGACCGGCGGCTCTAGAGCCTCTGCTAACCATGTTCATGCC
TTCTTCTTTTTCCTACAGCTCCTGGGCAACGTGCTGGTTGTTGTGCTGTCTCATCATTTTGGCAAAGAATTCGTC
GACctagaCTGCAGAACACGCAGCTAGATTAACCCTAGAAAGATAATCATATTGTGACGTACGTTAAAGATAATC
ATGCGTAAAATTGACGCATGTGTTTTATCGGTCTGTATATCGAGGTTTATTTATTAATTTGAATAGATATTAAG
TTTTATTATATTTACACTTACATACTAATAATAAATTCAACAAACAATTTATTTATGTTTATTTATTTATTAAAA
AAAAACAAAAACTCAAAATTTCTTCTATAAAGTAACAAAACTTTTATGAGGGACAGCCCCCCCCCAAAGCCCC
CAGGGATGTAATTACGTCCCTCCCCCGCTAGTGGGCAGCAGCGAGCCGCCCGGGGCTCCGCTCCGGTCCGGCG
CTCCCCCCGCATCCCCGAGCCGGCAGCGTGCGGGGACAGCCCGGGCACGGGGAAGGTGGCACGGGATCGCTT
TCCTCTGAACGCTTCTCGCTGCTCTTTGAGCCTGCAGACACCTGGGGGGATACGGGGAAAAGGCCTCCACGGC
CAGACTAGTAATGTGTGTCAGTTAGGGTGTGGAAAGTCCCCAGGCTCCCCAGCAGGCAGAAGTATGCAAAGC
ATGCATCTCAATTAGTCAGCAACCAGGTGTGGAAAGTCCCCAGGCTCCCCAGCAGGCAGAAGTATGCAAAGC
ATGCATCTCAATTAGTCAGCAACCATAGTCCCGCCCCTAACTCCGCCCATCCCGCCCCTAACTCCGCCCAGTTC
CGCCCATTCTCCGCCCCATGGCTGACTAATTTTTTTTATTTATGCAGAGGCCGAGGCCGCCTCTGCCTCTGAGC
TATTCCAGAAGTAGTGAGGAGGCTTTTTTGGAGGCCTAGGCTTTTGCAAAAAGCTCCCGGGAGCTTGTATATC
CATTTTCGGATCTGATCAGCACGTGTTGACAATTAATCATCGGCATAGTATATCGGCATAGTATAATACGACA
AGGTGAGGAACTAAACCATGGCCAAGCCTTTGTCTCAAGAAGAATCCACCCTCATTGAAAGAGCAACGGCTA
CAATCAACAGCATCCCCATCTCTGAAGACTACAGCGTCGCCAGCGCAGCTCTCTCTAGCGACGGCCGCATCTT
CACTGGTGTCAATGTATATCATTTTACTGGGGGACCTTGTGCAGAACTCGTGGTGCTGGGCACTGCTGCTGCTG
CGGCAGCTGGCAACCTGACTTGTATCGTCGCGATCGGAAATGAGAACAGGGGCATCTTGAGCCCCTGCGGAC
GGTGCCGACAGGTGCTTCTCGATCTGCATCCTGGGATCAAAGCCATAGTGAAGGACAGTGATGGACAGCCGA
CGGCAGTTGGGATTCGTGAATTGCTGCCCTCTGGTTATGTGTGGGAGGGCTAAGCACTTCGTGGCCGAGGAGC
AGGACTGACACGTGCTACGAGATTTCGATTCCACCGCCGCCTTCTATGAAAGGTTGGGCTTCGGAATCGTTTTC
CGGGACGCCGGCTGGATGATCCTCCAGCGCGGGGATCTCATGCTGGAGTTCTTCGCCCACCCCAACTTGTTTAT
TGCAGCTTATAATGGTTACAAATAAAGCAATAGCATCACAAATTTCACAAATAAAGCATTTTTTTCACTGCATT
 
CTAGTTGTGGTTTGTCCAAACTCATCAATGTATCTTATCATGTCTGGCGGCCATCTATAACAAGAAAATATATA
TATAATAAGTTATCACGTAAGTAGAACATGAAATAACAATATAATTATCGTATGAGTTAAATCTTAAAAGTCA
CGTAAAAGATAATCATGCGTCATTTTGACTCACGCGGTCGTTATAGTTCAAAATCAGTGACACTTACCGCATTG
ACAAGCACGCCTCACGGGAGCTCCAAGCGGCGACTGAGATGTCCTAAATGCACAGCGACGGATTCGCGCTAT
TTAGAAAGAGAGAGCAATATTTCAAGAATGCATGCGTCAATTTTACGCAGACTATCTTTCTAGGGTTAATCTA
GCTGCATCAGGATCATATCGTCGtctagacgccaccATGGTGTCCAAGGGCGAGGAGGTGATCAAGGAGTTCATGCG
CTTCAAGGTGCGCATGGAGGGCTCCATGAACGGCCACGAGTTCGAGATCGAGGGCGAGGGCGAGGGCCGCCC
CTACGAGGGCACCCAGACCGCCAAGCTGAAGGTGACCAAGGGCGGCCCCCTGCCCTTCGCCTGGGACATCCT
GTCCCCCCAGTTCATGTACGGCTCCAAGGCCTACGTGAAGCACCCCGCCGACATCCCCGACTACAAGAAGCTG
TCCTTCCCCGAGGGCTTCAAGTGGGAGCGCGTGATGAACTTCGAGGACGGCGGCCTGGTGACCGTGACCCAGG
ACTCCTCCCTGCAGGACGGCACCCTGATCTACAAGGTGAAGATGCGCGGCACCAACTTCCCCCCCGACGGCCC
CGTGATGCAGAAGAAGACCATGGGCTGGGAGGCCTCCACCGAGCGCCTGTACCCCCGCGACGGCGTGCTGAA
GGGCGAGATCCACCAGGCCCTGAAGCTGAAGGACGGCGGCCACTACCTGGTGGAGTTCAAGACCATCTACAT
GGCCAAGAAGCCCGTGCAGCTGCCCGGCTACTACTACGTGGACACCAAGCTGGACATCACCTCCCACAACGA
GGACTACACCATCGTGGAGCAGTACGAGCGCTCCGAGGGCCGCCACCACCTGTTCCTGtaaacgcgtgaattcactcctcagg
tgcaggctgcctatcagaaggtggtggctggtgtggccaatgccctggctcacaaataccactgagatctttttccctctgccaaaaattatggggacatcatgaagccccttgagcatc
tgacttctggctaataaaggaaatttattttcattgcaatagtgtgttggaattttttgtgtctctcactcggaaggacatatgggagggcaaatcatttaaaacatcagaatgagtatttggttt
agagtttggcaacatatgcccatatgctggctgccatgaacaaaggttggctataaagaggtcatcagtatatgaaacagccccctgctgtccattccttattccatagaaaagccttgac
ttgaggttagattttttttatattttgttttgtgttatttttttctttaacatccctaaaattttccttacatgttttactagccagatttttcctcctctcctgactactcccagtcatagctgtccctcttctc
ttatggagatccctcgacctgcagcccaagcttggcgtaatcatggtcatagctgtttcctgtgtgaaattgttatccgctcacaattccacacaacatacgagccggaagcataaagtgt
aaagcctggggtgcctaatgagtgagctaactcacattaattgcgttgcgctcactgcccgctttccagtcgggaaacctgtcgtgccagcggatcgacagtactaagctttactaggg
acaggattggtgacagaaaagccccatccttaggcctcctccttcctagtctcctgatattgggtctaacccccacctcctgttaggcagattccttatctggtgacacacccccatttcct
ggagccatctctctccttgccagaacctctaaggtttgcttacgatggagccagagaggatcctgggagggagagcttggcagggggtgggagggaagggggggatgcgtgacct
gcccggttctcagtggccaccctgcgctaccctctcccagaacctgagctgctctgacgcggctgtctggtgcgtttcactgatcctggtgctgcagcttccttacacttcccaagagga
gaagcagtttggaaaaacaaaatcagaataagttggtcctgagttctaactttggctcttcacctttctagtccccaatttatattgttcctccgtgcgtcagttttacctgtgagataaggcca
gtagccagccccgtcctggcagggctgtggtgaggaggggggtgtccgtgtggaaaactccctttgtgagaatggtgcgtcctaggtgttcaccaggtcgtggccgcctctactccct
ttctctttctccatccttctttccttaaagagtccccagtgctatctgggacatattcctccgcccagagcagggtcccgcttccctaaggccctgctctgggcttctgggtttgagtccttgg
caagcccaggagaggcgctcaggcttccctgtcccccttcctcgtccaccatctcatgcccctggctctcctgccccttccctacaggggttcctggctctgctctaagggcaagggcg
aattcgcggccgctaaattcaattcgccctatagtgagtcgtattacaattcactggccgtcgttttacaacgtcgtgactgggaaaaccctggcgttacccaacttaatcgccttgcagca
catccccctttcgccagctggcgtaatagcgaagaggcccgcaccgatcgcccttcccaacagttgcgcagcctatacgtacggcagtttaaggtttacacctataaaagagagagcc
gttatcgtctgtttgtggatgtacagagtgatattattgacacgccggggcgacggatggtgatccccctggccagtgcacgtctgctgtcagataaagtctcccgtgaactttacccggt
ggtgcatatcggggatgaaagctggcgcatgatgaccaccgatatggccagtgtgccggtctccgttatcggggaagaagtggctgatctcagccaccgcgaaaatgacatcaaaa
acgccattaacctgatgttctggggaatataaatgtcaggcatgagattatcaaaaaggatcttcacctagatccttttcacgtagaaagccagtccgcagaaacggtgctgaccccgga
tgaatgtcagctactgggctatctggacaagggaaaacgcaagcgcaaagagaaagcaggtagcttgcagtgggcttacatggcgatagctagactgggcggttttatggacagca
agcgaaccggaattgccagctggggcgccctctggtaaggttgggaagccctgcaaagtaaactggatggctttcttgccgccaaggatctgatggcgcaggggatcaagctctgat
caagagacaggatgaggatcgtttcgcatgattgaacaagatggattgcacgcaggttctccggccgcttgggtggagaggctattcggctatgactgggcacaacagacaatcggc
tgctctgatgccgccgtgttccggctgtcagcgcaggggcgcccggttctttttgtcaagaccgacctgtccggtgccctgaatgaactgcaagacgaggcagcgcggctatcgtgg
ctggccacgacgggcgttccttgcgcagctgtgctcgacgttgtcactgaagcgggaagggactggctgctattgggcgaagtgccggggcaggatctcctgtcatctcaccttgctc
 
ctgccgagaaagtatccatcatggctgatgcaatgcggcggctgcatacgcttgatccggctacctgcccattcgaccaccaagcgaaacatcgcatcgagcgagcacgtactcgga
tggaagccggtcttgtcgatcaggatgatctggacgaagagcatcaggggctcgcgccagccgaactgttcgccaggctcaaggcgagcatgcccgacggcgaggatctcgtcgt
gacccatgcatggcgatgcctgcttgccgaatatcatggtggaaaatggccgcttttctggattcatcgactgtggccggctgggtgtggcggaccgctatcaggacatagcgttggct
acccgtgatattgctgaagagcttggcggcgaatgggctgaccgcttcctcgtgctttacggtatcgccgctcccgattcgcagcgcatcgccttctatcgccttcttgacgagttcttctg
aattattaacgcttacaatttcctgatgcggtattttctccttacgcatctgtgcggtatttcacaccgcatcaggtggcacttttcggggaaatgtgcgcggaacccctatttgtttatttttcta
aatacattcaaatatgtatccgctcatgagattatcaaaaaggatcttcacctagatccttttaaattaaaaatgaagttttaaatcaatctaaagtatatatgagtaaacttggtctgacagtta
ccaatgcttaatcagtgaggcacctatctcagcgatctgtctatttcgttcatccatagttgcctgactccccgtcgtgtagataactacgatacgggagggcttaccatctggccccagtg
ctgcaatgataccgcgagacccacgctcaccggctccagatttatcagcaataaaccagccagccggaagggccgagcgcagaagtggtcctgcaactttatccgcctccatccagt
ctattaattgttgccgggaagctagagtaagtagttcgccagttaatagtttgcgcaacgttgttgccattgctacaggcatcgtggtgtcacgctcgtcgtttggtatggcttcattcagctc
cggttcccaacgatcaaggcgagttacatgatcccccatgttgtgcaaaaaagcggttagctccttcggtcctccgatcgttgtcagaagtaagttggccgcagtgttatcactcatggtt
atggcagcactgcataattctcttactgtcatgccatccgtaagatgcttttctgtgactggtgagtactcaaccaagtcattctgagaatagtgtatgcggcgaccgagttgctcttgcccg
gcgtcaatacgggataataccgcgccacatagcagaactttaaaagtgctcatcattggaaaacgttcttcggggcgaaaactctcaaggatcttaccgctgttgagatccagttcgatg
taacccactcgtgcacccaactgatcttcagcatcttttactttcaccagcgtttctgggtgagcaaaaacaggaaggcaaaatgccgcaaaaaagggaataagggcgacacggaaat
gttgaatactcatactcttcctttttcaatattattgaagcatttatcagggttattgtctcatgaccaaaatcccttaacgtgagttttcgttccactgagcgtcagaccccgtagaaaagatca
aaggatcttcttgagatcctttttttctgcgcgtaatctgctgcttgcaaacaaaaaaaccaccgctaccagcggtggtttgtttgccggatcaagagctaccaactctttttccgaaggtaa
ctggcttcagcagagcgcagataccaaatactgttcttctagtgtagccgtagttaggccaccacttcaagaactctgtagcaccgcctacatacctcgctctgctaatcctgttaccagt
ggctgctgccagtggcgataagtcgtgtcttaccgggttggactcaagacgatagttaccggataaggcgcagcggtcgggctgaacggggggttcgtgcacacagcccagcttgg
agcgaacgacctacaccgaactgagatacctacagcgtgagctatgagaaagcgccacgcttcccgaagggagaaaggcggacaggtatccggtaagcggcagggtcggaaca
ggagagcgcacgagggagcttccagggggaaacgcctggtatctttatagtcctgtcgggtttcgccacctctgacttgagcgtcgatttttgtgatgctcgtcaggggggcggagcc
tatggaaaaacgccagcaacgcggcctttttacggttcctggccttttgctggccttttgctcacatgttctttcctgcgttatcccctgattctgtggataaccgtattaccgcctttgagtga
gctgataccgctcgccgcagccgaacgaccgagcgcagcgagtcagtgagcgaggaagcggaagagcgcccaatacgcaaaccgcctctccccgcgcgttggccgattcatta
atgcagctggcacgacaggtttcccgactggaaagcgggcagtgagcgcaacgcaattaatgtgagttagctcactcattaggcaccccaggctttacactttatgcttccggctcgta
tgttgtgtggaattgtgagcggataacaatttcacacaggaaacagctatgaccatgattacgccaagctcagaattaaccctcactaaaggga
Receiving plasmid sequence(SEQ ID NO:16) 
TCGCGCGTTTCGGTGATGACGGTGAAAACCTCTGACACATGCAGCTCCCGGAGACGGTCACAGCTTGTCTGTA
AGCGGATGCCGGGAGCAGACAAGCCCGTCAGGGCGCGTCAGCGGGTGTTGGCGGGTGTCGGGGCTGGCTTAA
CTATGCGGCATCAGAGCAGATTGTACTGAGAGTGCACCATATGCGGTGTGAAATACCGCACAGATGCGTAAG
GAGAAAATACCGCATCAGGCGCCATTCGCCATTCAGGCTGCGCAACTGTTGGGAAGGGCGATCGGTGCGGGC
CTCTTCGCTATTACGCCAGCTGGCGAAAGGGGGATGTGCTGCAAGGCGATTAAGTTGGGTAACGCCAGGGTTT
TCCCAGTCACGACGTTGTAAAACGACGGCCAGTGAATTGGAGATCGGTACTTCGCATAACTTCGTATAGCATA
CATTATACGAAGTTATGAATGCGTCGAGATACGCGTCGCGGCCGCTTgcTTGGCGCGccgcagGCCAGTGTGTGAG
ACCGGGTTGGgacTAGGGATAACAGGGTAATCggctGTCGAGTCGCTTCTCGATTATGGTAGCCGGGCTATttacttttgt
aaaactttatggtttgtggaaaacaaatgtttttgaacatttaaaaagttcagatgttaaaaagttgaaaggttaatgtaaaacaatcaatattaaagaattttgatgccaaaactattagataa
aaggttaatctacatccctactagaattctcatacttaactggttggttatgtggaagaaacatactttcacaataaagagctttaggatatgatgccattttatatcactagtaggcagacca
gcagacttttttttattgtgatatgggataacctaggcatactgcactgtacactctgacatatgaagtgctctagtcaagtttaactggtgtccacagaggacatggtttaactggaattcgt
caagcctctggttctaatttctcatttgcagGGGACgctactTACggcaaactgacactgaaatttatttgcactaccggcaaactgcccgtaccgtggcccacactggtgacaac
attgacatacggagtgCAGTGCttcgccagatatcccgatcacatgaaacagcacgatttctttaagagcgccatgcctgagggttacgtgCAAgagcgaacaattttcttcaaa
 
gacgatggcaattacaaaactcGAGCAGAAGTGAAGTTTGAAGGGgacaccctggtgaaccgcatcgagctgaagggcatcgacttcaaggaggacggcaa
catcctggggcacaagctggagtacaactacaacagccacaaggtctatatcaccgccgacaagcagaagaacggcatcaaggtgaacttcaagacccgccacaacatcgagga
cggcagcgtgcagctcgccgaccactaccagcagaacacccccatcggcgacggccccgtgctgctgcccgacaaccactacctgagcacccagtccgccctgagcaaagacc
ccaacgagaagcgcgatcacatggtcctgctggagttcgtgaccgccgccgggatcactctcggcatggacgagctgtacaagtaaGTATGATTTAaagctcgctttcttgc
tgtccaatttctattaaaggttcctttgttccctaagtccaactactaaactgggggatattatgaagggccttgagcatctggattctgcctaataaaaaacatttattttcattgcaatgatgta
tttaaattatttctgaatattttactaaaaagggaatgtgggaggtcagtgcatttaaaacataaagaaatgaagagctagttcaaaccttgggaaaatacactatatcttaaactccatgaaa
gaaggtgaggctgcaaacagctaatgcacattggcaacagcccctgatgcctatgccttattcatccctcagaaaaggattcaagtagaggcttgatttggaggttaaagttttgctatgc
tgtattttacGATATCGATATCTATAACAAGAAAATATATATATAATAAGTTATCACGTAAGTAGAACATGAAATAAC
AATATAATTATCGTATGAGTTAAATCTTAAAAGTCACGTAAAAGATAATCATGCGTCATTTTGACTCACGCGG
TCGTTATAGTTCAAAATCAGTGACACTTACCGCATTGACAAGCACGCCTCACGGGAGCTCCAAGCGGCGACTG
AGATGTCCTAAATGCACAGCGACGGATTCGCGCTATTTAGAAAGAGAGAGCAATATTTCAAGAATGAcGCgTgg
aTTCTCGAGTaggTTGGTACCTTTTTTggccGGCTTGTCGACGACGGCGGTCTCCGTCGTCAGGATCATccgggccaccgc
caccatggccaagcctttgtctcaagaagaatccaccctcattgaaagagcaacggctacaatcaacagcatccccatctctgaagactacagcgtcgccagcgcagctctctctagc
gacggccgcatcttcactggtgtcaatgtatatcattttactgggggaccttgtgcagaactcgtggtgctgggcactgctgctgctgcggcagctggcaacctgacttgtatcgtcgcg
atcggaaatgagaacaggggcatcttgagcccctgcggacggtgccgacaggtgcttctcgatctgcatcctgggatcaaagccatagtgaaggacagtgatggacagccgacgg
cagttgggattcgtgaattgctgccctctggttatgtgtgggagggctaaGTTTAAACCCGCTGATCAGCCTCGACTGTGCCTTCTAGTTGCCA
GCCATCTGTTGTTTGCCCCTCCCCCGTGCCTTCCTTGACCCTGGAAGGTGCCACTCCCACTGTCCTTTCCTAATA
AAATGAGGAAATTGCATCGCATTGTCTGAGTAGGTGTCATTCTATTCTGGGGGGTGGGGTGGGGCAGGACAGC
AAGGGGGAGGATTGGGAAGACAATAGCAGGCATGCTGGGGATGCGGTGGGCTCTATGGCTTCATCGGATGCC
GGGACCGACGAGTGCAGAGGCGTGCAAATAACTTCGTATAGCATACATTATACGAAGTTATGCGAGCTTGGC
GTAATCATGGTCATAGCTGTTTCCTGTGTGAAATTGTTATCCGCTCACAATTCCACACAACATACGAGCCGGAA
GCATAAAGTGTAAAGCCTGGGGTGCCTAATGAGTGAGCTAACTCACATTAATTGCGTTGCGCTCACTGCCCGC
TTTCCAGTCGGGAAACCTGTCGTGCCAGCTGCATTAATGAATCGGCCAACGCGCGGGGAGAGGCGGTTTGCGT
ATTGGGCGCTCTTCCGCTTCCTCGCTCACTGACTCGCTGCGCTCGGTCGTTCGGCTGCGGCGAGCGGTATCAGC
TCACTCAAAGGCGGTAATACGGTTATCCACAGAATCAGGGGATAACGCAGGAAAGAACATGTGAGCAAAAGG
CCAGCAAAAGGCCAGGAACCGTAAAAAGGCCGCGTTGCTGGCGTTTTTCCATAGGCTCCGCCCCCCTGACGAG
CATCACAAAAATCGACGCTCAAGTCAGAGGTGGCGAAACCCGACAGGACTATAAAGATACCAGGCGTTTCCC
CCTGGAAGCTCCCTCGTGCGCTCTCCTGTTCCGACCCTGCCGCTTACCGGATACCTGTCCGCCTTTCTCCCTTCG
GGAAGCGTGGCGCTTTCTCATAGCTCACGCTGTAGGTATCTCAGTTCGGTGTAGGTCGTTCGCTCCAAGCTGGG
CTGTGTGCACGAACCCCCCGTTCAGCCCGACCGCTGCGCCTTATCCGGTAACTATCGTCTTGAGTCCAACCCGG
TAAGACACGACTTATCGCCACTGGCAGCAGCCACTGGTAACAGGATTAGCAGAGCGAGGTATGTAGGCGGTG
CTACAGAGTTCTTGAAGTGGTGGCCTAACTACGGCTACACTAGAAGAACAGTATTTGGTATCTGCGCTCTGCT
GAAGCCAGTTACCTTCGGAAAAAGAGTTGGTAGCTCTTGATCCGGCAAACAAACCACCGCTGGTAGCGGTGGT
TTTTTTGTTTGCAAGCAGCAGATTACGCGCAGAAAAAAAGGATCTCAAGAAGATCCTTTGATCTTTTCTACGG
GGTCTGACGCTCAGTGGAACGAAAACTCACGTTAAGGGATTTTGGTCATGAGATTATCAAAAAGGATCTTCAC
CTAGATCCTTTTAAATTAAAAATGAAGTTTTAAATCAATCTAAAGTATATATGAGTAAACTTGGTCTGACAGTT
ACCAATGCTTAATCAGTGAGGCACCTATCTCAGCGATCTGTCTATTTCGTTCATCCATAGTTGCCTGACTCCCC
GTCGTGTAGATAACTACGATACGGGAGGGCTTACCATCTGGCCCCAGTGCTGCAATGATACCGCGAGACCCAC
 
GCTCACCGGCTCCAGATTTATCAGCAATAAACCAGCCAGCCGGAAGGGCCGAGCGCAGAAGTGGTCCTGCAA
CTTTATCCGCCTCCATCCAGTCTATTAATTGTTGCCGGGAAGCTAGAGTAAGTAGTTCGCCAGTTAATAGTTTG
CGCAACGTTGTTGCCATTGCTACAGGCATCGTGGTGTCACGCTCGTCGTTTGGTATGGCTTCATTCAGCTCCGG
TTCCCAACGATCAAGGCGAGTTACATGATCCCCCATGTTGTGCAAAAAAGCGGTTAGCTCCTTCGGTCCTCCG
ATCGTTGTCAGAAGTAAGTTGGCCGCAGTGTTATCACTCATGGTTATGGCAGCACTGCATAATTCTCTTACTGT
CATGCCATCCGTAAGATGCTTTTCTGTGACTGGTGAGTACTCAACCAAGTCATTCTGAGAATAGTGTATGCGGC
GACCGAGTTGCTCTTGCCCGGCGTCAATACGGGATAATACCGCGCCACATAGCAGAACTTTAAAAGTGCTCAT
CATTGGAAAACGTTCTTCGGGGCGAAAACTCTCAAGGATCTTACCGCTGTTGAGATCCAGTTCGATGTAACCC
ACTCGTGCACCCAACTGATCTTCAGCATCTTTTACTTTCACCAGCGTTTCTGGGTGAGCAAAAACAGGAAGGC
AAAATGCCGCAAAAAAGGGAATAAGGGCGACACGGAAATGTTGAATACTCATACTCTTCCTTTTTCAATATTA
TTGAAGCATTTATCAGGGTTATTGTCTCATGAGCGGATACATATTTGAATGTATTTAGAAAAATAAACAAATA
GGGGTTCCGCGCACATTTCCCCGAAAAGTGCCACCTGACGTCTAAGAAACCATTATTATCATGACATTAACCT
ATAAAAATAGGCGTATCACGAGGCCCTTTCGTC
N GFP transposon donor plasmid sequence(SEQ ID NO:17) 
CTGCAGAACACGCAGCTAGcggccgcATTAACCCTAGAAAGATAATCATATTGTGACGTACGTTAAAGATAATCA
TGCGTAAAATTGACGCATGTGTTTTATCGGTCTGTATATCGAGGTTTATTTATTAATTTGAATAGATATTAAGT
TTTATTATATTTACACTTACATACTAATAATAAATTCAACAAACAATTTATTTATGTTTATTTATTTATTAAAAA
AAAACAAAAACTCAAAATTTCTTCTATAAAGTAACAAAACTTTTATGAGGGACAGCCCCCCCCCAAAGCCCCC
AGGGATGTAATTACGTCCCTCCCCCGCTAGGGGGCAGCAGCGAGCCGCCCGGGGCTCCGCTCCGGTCCGGCGC
TCCCCCCGCATCCCCGAGCCGGCAGCGTGCGGGGACAGCCCGGGCACGGGGAAGGTGGCACGGGATCGCTTT
CCTCTGAACGCTTCTCGCTGCTCTTTGAGCCTGCAGACACCTGGGGGGATACGGGGAAAAGGCCTCCACGGCC
AGActagtAATGTGTGTCAGTTAGGGTGTGGAAAGTCCCCAGGCTCCCCAGCAGGCAGAAGTATGCAAAGCATG
CATCTCAATTAGTCAGCAACCAGGTGTGGAAAGTCCCCAGGCTCCCCAGCAGGCAGAAGTATGCAAAGCATG
CATCTCAATTAGTCAGCAACCATAGTCCCGCCCCTAACTCCGCCCATCCCGCCCCTAACTCCGCCCAGTTCCGC
CCATTCTCCGCCCCATGGCTGACTAATTTTTTTTATTTATGCAGAGGCCGAGGCCGCCTCTGCCTCTGAGCTATT
CCAGAAGTAGTGAGGAGGCTTTTTTGGAGGCCTAGGCTTTTGCAAAAAGCTCCCGGGAGCTTGTATATCCATT
TTCGGATCTGATCAGCACGTGTTGACAATTAATCATCGGCATAGTATATCGGCATAGTATAATACGACAAGGT
GAGGAAtcgccACCatggttagtaagggcgaggaattgattaaggagaacatgcatatgaagctgtacatggaaggcactgtggataaccaccactttaaatgcaccagtga
aggcgaaggcaagccatatgagggaacacagactatgcggattaaagtggtggagggtggcccattgccattcgcttttgacattctcgctaccagttttctttatgggtccaagacctt
cataaatcatactcaggggatcccagacttcttcaaacaatcttttccggaagggttcacctgggagcgggtgacaacttacgaggatgggggcgtgctgaccgcaactcaggatacc
agcctccaggacggatgcttgatctataacgttaagatccgcggcgtgaattttacctcaaatgggccggtgatgcagaaaaagaccctgggatgggaggctttcactgaaacgctct
accccgctgatggcggcctggagggcagaaatgatatggccctgaagctggtgggagggagccaccttatagctaacgccaagaccacttaccgctccaaaaagccagcaaaga
atctcaagatgcccggggtgtactacgttgattaccgcctggagaggatcaaggaagccaataacgagacctacgtcgagcagcacgaggtggccgttgcccggtactgcgatcttc
caagtaaacttggccataagctgaatGGTTCCGGTgagggcagaggaagtcttctaacatgcggtgacgtggaggagaatcccggccctGGTTCCGGTatgaccga
gtacaagcccacggtgcgcctcgccacccgcgacgacgtccccagggccgtacgcaccctcgccgccgcgttcgccgactaccccgccacgcgccacaccgtcgatccggacc
gccacatcgagcgggtcaccgagctgcaagaactcttcctcacgcgcgtcgggctcgacatcggcaaggtgtgggtcgcggacgacggcgccgcggtggcggtctggaccacg
ccggagagcgtcgaagcgggggcggtgttcgccgagatcggcccgcgcatggccgagttgagcggttcccggctggccgcgcagcaacagatggaaggcctcctggcgccgc
 
accggcccaaggagcccgcgtggttcctggccaccgtcggcgtctcgcccgaccaccagggcaagggtctgggcagcgccgtcgtgctccccggagtggaggcggccgagcg
cgccggggtgcccgccttcctggagacctccgcgccccgcaacctccccttctacgagcggctcggcttcaccgtcaccgccgacgtcgaggtgcccgaaggaccgcgcacctg
gtgcatgacccgcaagcccggtgcctgacgaCACCCCAACTTGTTTATTGCAGCTTATAATGGTTACAAATAAAGCAATAGCAT
CACAAATTTCACAAATAAAGCATTTTTTTCACTGCATTCTAGTTGTGGTTTGTCCAAACTCATCAATGTATCTTA
TCATGTCTGGGCGAGCTCACGGGGACAGCCCCCCCCCAAAGCCCCCAGGGATGTAATTACGTCCCTCCCCCGC
TAGGGGGCAGCAGCGAGCCGCCCGGGGCTCCGCTCCGGTCCGGCGCTCCCCCCGCATCCCCGAGCCGGCAGC
GTGCGGGGACAGCCCGGGCACGGGGAAGGTGGCACGGGATCGCTTTCCTCTGAACGCTTCTCGCTGCTCTTTG
AGCCTGCAGACACCTGGGGGGATACGGGGAAAAAGCTTTAGGCTGAAAGAGAGATTTAGAATGACAGAATCA
TAGAACGGCCTGGGTTGCAAAGGAGCACAGTGCTCATCCAGATCCAACCCCCTGCTATGTGCAGGGTCATCAA
CCAGCAGCCCAGGCTGCCCAGAGCCACATCCAGCCTGGCCTTGAATGCCTGCAGGGATGGGGCATCCACAGC
CTCCTTGGGCAACCTGTTCAGTGCGTCACCACCCTCTGGGGGAAAAACTGCCTCCTCATATCCAACCCAAACCT
CCCCTGTCTCAGTGTAAAGCCATTCCCCCTTGTCCTATCAAGGGGGAGTTTGCTGTGACATTGTTGGTCTGGGG
TGACACATGTTTGCCAATTCAGTGCATCACGGAGAGGCAGATCTTGGGGATAAGGAAGTGCAGGACAGCATG
GACGTGGGACATGCAGGTGTTGAGGGCTCTGGGACACTCTCCAAGTCACAGCGTTCAGAACAGCCTTAAGGAT
AAGAAGATAGGATAGAAGGACAAAGAGCAAGTTAAAACCCAGCATGGAGAGGAGCACAAAAAGGCCACAGA
CACTGCTGGTCCCTGTGTCTGAGCCTGCATGTTTGATGGTGTCTGGATGCAAGCAGAAGGGGTGGAAGAGCTT
GCCTGGAGAGATACAGCTGGGTCAGTAGGACTGGGACAGGCAGCTGGAGAATTGCCATGTAGATGTTCATAC
AATCGTCAAATCATGAAGGCTGGAAAAGCCCTCCAAGATCCCCAAGACCAACCCCAACCCACCCACCGTGCC
CACTGGCCATGTCCCTCAGTGCCACATCCCCACAGTTCTTCATCACCTCCAGGGACGGTGACCCCCCCACCTCC
GTGGGCAGCTGTGCCACTGCAGCACCGCTCTTTGGAGAAGGTAAATCTTGCTAAATCCAGCCCGACCCTCCCC
TGGCACAACGTAAGGCCATTATCTCTCATCCAACTCCAGGACGGAGTCAGTGAGAATATTgcGCtcttgaaaggagtggg
aattcgttacataacttacggtaaatggcccgcctggctgaccgcccaacgacccccgcccattgacgtcaataatgacgtatgttcccatagtaacgccaatagggactttccattgac
gtcaatgggtggagtatttacggtaaactgcccacttggcagtacatcaagtgtatcatatgccaagtacgccccctattgacgtcaatgacggtaaatggcccgcctggcattatgccc
agtacatgaccttatgggactttcctacttggcagtacatctacgtattagtcatcgctattaccatggtcgaggtgagccccacgttctgcttcactctccccatctcccccccctccccac
ccccaattttgtatttatttattttttaattattttgtgcagcgatgggggcggggggggggggggggcgcgcgccaggcggggcggggcggggcgaggggcggggcggggcga
ggcggagaggtgcggcggcagccaatcagagcggcgcgctccgaaagtttccttttatggcgaggcggcggcggcggcggccctataaaaagcgaagcgcgcggcgggcgg
gagtcgctgcgcgctgccttcgccccgtgccccgctccgccgccgcctcgcgccgcccgccccggctctgactgaccgcgttactcccacaggtgagcgggcgggacggccctt
ctcctccgggctgtaattagcgcttggtttaatgacggcttgtttcttttctgtggctgcgtgaaagccttgaggggctccgggagggccctttgtgcggggggagcggctcggggggt
gcgtgcgtgtgtgtgtgcgtggggagcgccgcgtgcggctccgcgctgcccggcggctgtgagcgctgcgggcgcggcgcggggctttgtgcgctccgcagtgtgcgcgaggg
gagcgcggccgggggcggtgccccgcggtgcggggggggctgcgaggggaacaaaggctgcgtgcggggtgtgtgcgtgggggggtgagcagggggtgtgggcgcgtcg
gtcgggctgcaaccccccctgcacccccctccccgagttgctgagcacggcccggcttcgggtgcggggctccgtacggggcgtggcgcggggctcgccgtgccgggcgggg
ggtggcggcaggtgggggtgccgggcggggcggggccgcctcgggccggggagggctcgggggaggggcgcggcggcccccggagcgccggcggctgtcgaggcgcg
gcgagccgcagccattgccttttatggtaatcgtgcgagagggcgcagggacttcctttgtcccaaatctgtgcggagccgaaatctgggaggcgccgccgcaccccctctagcgg
gcgcggggcgaagcggtgcggcgccggcaggaaggaaatgggcggggagggccttcgtgcgtcgccgcgccgccgtccccttctccctctccagcctcggggctgtccgcgg
ggggacggctgccttcgggggggacggggcagggcggggttcggcttctggcgtgtgaccggcggctctagagcctctgctaaccatgttcatgccttcttctttttcctacagctcct
gggcaacgtgctggttattgtgctgtctcatcattttggcaaagaattgatttgataccgcgggcGCCACCatggtgtctaagggcgaagagctctttactggcgtggtgcccatcC
TGGTTGAATTGGACGGAGATGTTAACGGACACAAATTTAGCGTATCTGGAGAGGGCGAAGGTAAGTAatcggttca
agtgattctcctgcctcagcctcccaagtagctgggattagaggtccccaccaccatgcctggctaattttttgtactttcagtagaaatggggttttgccatgttggccaggctgttctcga
 
actcctgagctcaggtgatccaactgtctcggcctcccaaagtgctgggattacaggcgtgagccactgtgcctagcctgagccaccacgccggcctaatttttaaattttttgtagaga
cagggtctcattatgttgcccagggtggtgtcaagctccaggtctcaagtgatccccctacctccgcctcccaaagttgtgggattgtaggcatgagccactgcaagaaaaccttaactg
cagcctaataattgttttctttgggataacttttaaagtacattaaaagactatcaacttaatttctgatcatattttgttgaataaaataagtaaaatgtcttgtgaaacaaaatgctttttaacatc
catataaagctatCTATATATAGCTATCTATGTCTaccCATGCGTCAATTTTACGCAGACTATCTTTCTAGGGTTAATCGG
CGCGccTTAATCTAGCTGCATCAGGATCATATCGTCGGGTCTTTTTTCCGGCTCAGTCATCGCCCAAGCTGGCGC
TATCTGGGCATCGGGGAGGAAGAAGCCCGTGCCTTTTCCCGCGAGGTTGAAGCGGCATGGAAAGAGTTTGCC
GAGGATGACTGCTGCTGCATTGACGTTGAGCGAAAACGCACGTTTACCATGATGATTCGGGAAGGTGTGGCCA
TGCACGCCTTTAACGGTGAACTGTTCGTTCAGGCCACCTGGGATACCAGTTCGTCGCGGCTTTTCCGGACACAG
TTCCGGATGGTCAGCCCGAAGCGCATCAGCAACCCGAACAATACCGGCGACAGCCGGAACTGCCGTGCCGGT
GTGCAGATTAATGACAGCGGTGCGGCGCTGGGATATTACGTCAGCGAGGACGGGTATCCTGGCTGGATGCCG
CAGAAATGGACATGGATACCCCGTGAGTTACCCGGCGGGCGCGCTTGGCGTAATCATGGTCATAGCTGTTTCC
TGTGTGAAATTGTTATCCGCTCACAATTCCACACAACATACGAGCCGGAAGCATAAAGTGTAAAGCCTGGGGT
GCCTAATGAGTGAGCTAACTCACATTAATTGCGTTGCGCTCACTGCCCGCTTTCCAGTCGGGAAACCTGTCGTG
CCAGCTGCATTAATGAATCGGCCAACGCGCGGGGAGAGGCGGTTTGCGTATTGGGCGCTCTTCCGCTTCCTCG
CTCACTGACTCGCTGCGCTCGGTCGTTCGGCTGCGGCGAGCGGTATCAGCTCACTCAAAGGCGGTAATACGGT
TATCCACAGAATCAGGGGATAACGCAGGAAAGAACATGTGAGCAAAAGGCCAGCAAAAGGCCAGGAACCGT
AAAAAGGCCGCGTTGCTGGCGTTTTTCCATAGGCTCCGCCCCCCTGACGAGCATCACAAAAATCGACGCTCAA
GTCAGAGGTGGCGAAACCCGACAGGACTATAAAGATACCAGGCGTTTCCCCCTGGAAGCTCCCTCGTGCGCTC
TCCTGTTCCGACCCTGCCGCTTACCGGATACCTGTCCGCCTTTCTCCCTTCGGGAAGCGTGGCGCTTTCTCATA
GCTCACGCTGTAGGTATCTCAGTTCGGTGTAGGTCGTTCGCTCCAAGCTGGGCTGTGTGCACGAACCCCCCGTT
CAGCCCGACCGCTGCGCCTTATCCGGTAACTATCGTCTTGAGTCCAACCCGGTAAGACACGACTTATCGCCAC
TGGCAGCAGCCACTGGTAACAGGATTAGCAGAGCGAGGTATGTAGGCGGTGCTACAGAGTTCTTGAAGTGGT
GGCCTAACTACGGCTACACTAGAAGAACAGTATTTGGTATCTGCGCTCTGCTGAAGCCAGTTACCTTCGGAAA
AAGAGTTGGTAGCTCTTGATCCGGCAAACAAACCACCGCTGGTAGCGGTGGTTTTTTTGTTTGCAAGCAGCAG
ATTACGCGCAGAAAAAAAGGATCTCAAGAAGATCCTTTGATCTTTTCTACGGGGTCTGACGCTCAGTGGAACG
AAAACTCACGTTAAGGGATTTTGGTCATGAgtgcgcggaacccctatttgtttatttttctaaatacattcaaatatgtatccgctcatgagacaataaccctg
ataaatgcttcaataatattgaaaaaggaagagtATGAGGGAAGCGGTGATCGCCGAAGTATCGACTCAACTATCAGAGGTAGTTG
GCGTCATCGAGCGCCATCTCGAACCGACGTTGCTGGCCGTACATTTGTACGGCTCCGCAGTGGATGGCGGCCT
GAAGCCACACAGTGATATTGATTTGCTGGTTACGGTGACCGTAAGGCTTGATGAAACAACGCGGCGAGCTTTG
ATCAACGACCTTTTGGAAACTTCGGCTTCCCCTGGAGAGAGCGAGATTCTCCGCGCTGTAGAAGTCACCATTG
TTGTGCACGACGACATCATTCCGTGGCGTTATCCAGCTAAGCGCGAACTGCAATTTGGAGAATGGCAGCGCAA
TGACATTCTTGCAGGTATCTTCGAGCCAGCCACGATCGACATTGATCTGGCTATCTTGCTGACAAAAGCAAGA
GAACATAGCGTTGCCTTGGTAGGTCCAGCGGCGGAGGAACTCTTTGATCCGGTTCCTGAACAGGATCTATTTG
AGGCGCTAAATGAAACCTTAACGCTATGGAACTCGCCGCCCGACTGGGCTGGCGATGAGCGAAATGTAGTGC
TTACGTTGTCCCGCATTTGGTACAGCGCAGTAACCGGCAAAATCGCGCCGAAGGATGTCGCTGCCGACTGGGC
AATGGAGCGCCTGCCGGCCCAGTATCAGCCCGTCATACTTGAAGCTAGACAGGCTTATCTTGGACAAGAAGAA
GATCGCTTGGCCTCGCGCGCAGATCAGTTGGAAGAATTTGTCCACTACGTGAAAGGCGAGATCACCAAGGTAG
TCGGCAAATAACCCTCGAGCCACCCATGACCAAAATCCCTTAACGTGAGTTA
Cas9-PBase N347A_R376E_D450N-ROSA gRNA plasmid sequence(SEQ ID NO:18) 
GTGATGCGGTTTTGGCAGTACATCAATGGGCGTGGATAGCGGTTTGACTCACGGGGATTTCCAAGTCTCCACC
CCATTGACGTCAATGGGAGTTTGTTTTGGCACCAAAATCAACGGGACTTTCCAAAATGTCGTAACAACTCCGC
CCCATTGACGCAAATGGGCGGTAGGCGTGTACGGTGGGAGGTCTATATAAGCAGAGCTCGTTTAGTGAACCGT
CAGATCGCCTGGAGACGCCATCCACGCTGTTTTGACCTCCATAGAAGACACCGGGACCGATCCAGCCTCCGGA
CTCTAGAGGATCGAACCCTTGCCACCATGGACAAGAAGTACTCCATTGGGCTCGacATCGGCACAAACAGCGT
CGGCTGGGCCGTCATTACGGACGAGTACAAGGTGCCGAGCAAAAAATTCAAAGTTCTGGGCAATACCGATCG
CCACAGCATAAAGAAGAACCTCATTGGCGCCCTCCTGTTCGACTCCGGGGAGACGGCCGAAGCCACGCGGCT
CAAAAGAACAGCACGGCGCAGATATACCCGCAGAAAGAATCGGATCTGCTACCTGCAGGAGATCTTTAGTAA
TGAGATGGCTAAGGTGGATGACTCTTTCTTCCATAGGCTGGAGGAGTCCTTTTTGGTGGAGGAGGATAAAAAG
CACGAGCGCCACCCAATCTTTGGCAATATCGTGGACGAGGTGGCGTACCATGAAAAGTACCCAACCATATATC
ATCTGAGGAAGAAGCTTGTAGACAGTACTGATAAGGCTGACTTGCGGTTGATCTATCTCGCGCTGGCGCATAT
GATCAAATTTCGGGGACACTTCCTCATCGAGGGGGACCTGAACCCAGACAACAGCGATGTCGACAAACTCTTT
ATCCAACTGGTTCAGACTTACAATCAGCTTTTCGAAGAGAACCCGATCAACGCATCCGGAGTTGACGCCAAAG
CAATCCTGAGCGCTAGGCTGTCCAAATCCCGGCGGCTCGAAAACCTCATCGCACAGCTCCCTGGGGAGAAGA
AGAACGGCCTGTTTGGTAATCTTATCGCCCTGTCACTCGGGCTGACCCCCAACTTTAAATCTAACTTCGACCTG
GCCGAAGATGCCAAGCTTCAACTGAGCAAAGACACCTACGATGATGATCTCGACAATCTGCTGGCCCAGATCG
GCGACCAGTACGCAGACCTTTTTTTGGCGGCAAAGAACCTGTCAGACGCCATTCTGCTGAGTGATATTCTGCG
AGTGAACACGGAGATCACCAAAGCTCCGCTGAGCGCTAGTATGATCAAGCGCTATGATGAGCACCACCAAGA
CTTGACTTTGCTGAAGGCCCTTGTCAGACAGCAACTGCCTGAGAAGTACAAGGAAATTTTCTTCGATCAGTCT
AAAAATGGCTACGCCGGATACATTGACGGCGGAGCAAGCCAGGAGGAATTTTACAAATTTATTAAGCCCATCT
TGGAAAAAATGGACGGCACCGAGGAGCTGCTGGTAAAGCTTAACAGAGAAGATCTGTTGCGCAAACAGCGCA
CTTTCGACAATGGAAGCATCCCCCACCAGATTCACCTGGGCGAACTGCACGCTATCCTCAGGCGGCAAGAGGA
TTTCTACCCCTTTTTGAAAGATAACAGGGAAAAGATTGAGAAAATCCTCACATTTCGGATACCCTACTATGTA
GGCCCCCTCGCCCGGGGAAATTCCAGATTCGCGTGGATGACTCGCAAATCAGAAGAGACCATCACTCCCTGGA
ACTTCGAGGAAGTCGTGGATAAGGGGGCCTCTGCCCAGTCCTTCATCGAAAGGATGACTAACTTTGATAAAAA
TCTGCCTAACGAAAAGGTGCTTCCTAAACACTCTCTGCTGTACGAGTACTTCACAGTTTATAACGAGCTCACCA
AGGTCAAATACGTCACAGAAGGGATGAGAAAGCCAGCATTCCTGTCTGGAGAGCAGAAGAAAGCTATCGTGG
ACCTCCTCTTCAAGACGAACCGGAAAGTTACCGTGAAACAGCTCAAAGAAGACTATTTCAAAAAGATTGAAT
GTTTCGACTCTGTTGAAATCAGCGGAGTGGAGGATCGCTTCAACGCATCCCTGGGAACGTATCACGATCTCCT
GAAAATCATTAAAGACAAGGACTTCCTGGACAATGAGGAGAACGAGGACATTCTTGAGGACATTGTCCTCAC
CCTTACGTTGTTTGAAGATAGGGAGATGATTGAAGAACGCTTGAAAACTTACGCTCATCTCTTCGACGACAAA
GTCATGAAACAGCTCAAGAGGCGCCGATATACAGGATGGGGGCGGCTGTCAAGAAAACTGATCAATGGGATC
CGAGACAAGCAGAGTGGAAAGACAATCCTGGATTTTCTTAAGTCCGATGGATTTGCCAACAGGAACTTCATGC
AGTTGATCCATGATGACTCTCTCACCTTTAAGGAGGACATCCAGAAAGCACAAGTTTCTGGCCAGGGGGACAG
TCTTCACGAGCACATCGCTAATCTTGCAGGTAGCCCAGCTATCAAAAAGGGAATACTGCAGACCGTTAAGGTC
GTGGATGAACTCGTCAAAGTAATGGGAAGGCATAAGCCCGAGAATATCGTTATCGAGATGGCCCGAGAGAAC
 
CAAACTACCCAGAAGGGACAGAAGAACAGTAGGGAAAGGATGAAGAGGATTGAAGAGGGTATAAAAGAACT
GGGGTCCCAAATCCTTAAGGAACACCCAGTTGAAAACACCCAGCTTCAGAATGAGAAGCTCTACCTGTACTAC
CTGCAGAACGGCAGGGACATGTACGTGGATCAGGAACTGGACATCAATCGGCTCTCCGACTACGACGTGGAT
CATATCGTGCCCCAGTCTTTTCTCAAAGATGATTCTATTGATAATAAAGTGTTGACAAGATCCGATAAAAATA
GAGGGAAGAGTGATAACGTCCCCTCAGAAGAAGTTGTCAAGAAAATGAAAAATTATTGGCGGCAGCTGCTGA
ACGCCAAACTGATCACACAACGGAAGTTCGATAATCTGACTAAGGCTGAACGAGGTGGCCTGTCTGAGTTGG
ATAAAGCCGGCTTCATCAAAAGGCAGCTTGTTGAGACACGCCAGATCACCAAGCACGTGGCCCAAATTCTCGA
TTCACGCATGAACACCAAGTACGATGAAAATGACAAACTGATTCGAGAGGTGAAAGTTATTACTCTGAAGTCT
AAGCTGGTCTCAGATTTCAGAAAGGACTTTCAGTTTTATAAGGTGAGAGAGATCAACAATTACCACCATGCGC
ATGATGCCTACCTGAATGCAGTGGTAGGCACTGCACTTATCAAAAAATATCCCAAGCTTGAATCTGAATTTGT
TTACGGAGACTATAAAGTGTACGATGTTAGGAAAATGATCGCAAAGTCTGAGCAGGAAATAGGCAAGGCCAC
CGCTAAGTACTTCTTTTACAGCAATATTATGAATTTTTTCAAGACCGAGATTACACTGGCCAATGGAGAGATTC
GGAAGCGACCACTTATCGAAACAAACGGAGAAACAGGAGAAATCGTGTGGGACAAGGGTAGGGATTTCGCG
ACAGTCCGGAAGGTCCTGTCCATGCCGCAGGTGAACATCGTTAAAAAGACCGAAGTACAGACCGGAGGCTTC
TCCAAGGAAAGTATCCTCCCGAAAAGGAACAGCGACAAGCTGATCGCACGCAAAAAAGATTGGGACCCCAAG
AAATACGGCGGATTCGATTCTCCTACAGTCGCTTACAGTGTACTGGTTGTGGCCAAAGTGGAGAAAGGGAAGT
CTAAAAAACTCAAAAGCGTCAAGGAACTGCTGGGCATCACAATCATGGAGCGATCAAGCTTCGAAAAAAACC
CCATCGACTTTCTCGAGGCGAAAGGATATAAAGAGGTCAAAAAAGACCTCATCATTAAGCTTCCCAAGTACTC
TCTCTTTGAGCTTGAAAACGGCCGGAAACGAATGCTCGCTAGTGCGGGCGAGCTGCAGAAAGGTAACGAGCT
GGCACTGCCCTCTAAATACGTTAATTTCTTGTATCTGGCCAGCCACTATGAAAAGCTCAAAGGGTCTCCCGAA
GATAATGAGCAGAAGCAGCTGTTCGTGGAACAACACAAACACTACCTTGATGAGATCATCGAGCAAATAAGC
GAATTCTCCAAAAGAGTGATCCTCGCCGACGCTAACCTCGATAAGGTGCTTTCTGCTTACAATAAGCACAGGG
ATAAGCCCATCAGGGAGCAGGCAGAAAACATTATCCACTTGTTTACTCTGACCAACTTGGGCGCGCCTGCAGC
CTTCAAGTACTTCGACACCACCATAGACAGAAAGCGGTACACCTCTACAAAGGAGGTCCTGGACGCCACACTG
ATTCATCAGTCAATTACGGGGCTCTATGAAACAAGAATCGACCTCTCTCAGCTCGGTGGAGACAGCAGGGCTG
ACCCCAAGAAGAAGAGGAAGGTGGGCGGAGGAGGGAGTGGCGGAGGCGGAAGCGGCGGCGGAGGCAGCGG
CTCTAGCCTGGACGACGAGCACATCCTGAGCGCCCTGCTGCAGAGCGACGACGAACTGGTGGGCGAGGACAG
CGACAGCGAGGTCAGCGACCACGTGTCCGAGGACGACGTGCAGTCCGACACCGAGGAAGCCTTCATCGACGA
GGTGCACGAAGTGCAGCCTACCAGCAGCGGCTCCGAGATCCTGGACGAGCAGAACGTGATCGAGCAGCCTGG
CAGCTCCCTGGCCAGCAACAGAATCCTGACCCTGCCCCAGAGAACCATCAGAGGCAAGAACAAGCACTGCTG
GTCCACCTCCAAGAGCACCAGGCGGAGCAGAGTGTCCGCCCTGAACATCGTGCGGAGCCAGAGGGGCCCCAC
CAGAATGTGCAGAAACATCTACGACCCCCTGCTGTGCTTCAAGCTGTTCTTCACCGACGAGATCATCAGCGAG
ATCGTGAAGTGGACCAACGCCGAGATCAGCCTGAAGAGGCGGGAGAGCATGACCAGCGCCACCTTCAGAGAC
ACCAACGAGGACGAGATCTACGCCTTCTTCGGCATCCTGGTGATGACCGCCGTGAGAAAGGACAACCACATG
AGCACCGACGACCTGTTCGACAGATCCCTGAGCATGGTGTACGTGTCCGTGATGAGCAGAGACAGATTCGACT
TCCTGATCAGATGCCTGAGAATGGACGACAAGAGCATCAGACCCACCCTGCGGGAGAACGACGTGTTCACCC
CCGTGCGGAAGATCTGGGACCTGTTCATCCACCAGTGCATCCAGAACTACACCCCTGGCGCCCACCTGACCAT
CGATGAGCAGCTGCTGGGCTTCAGAGGCAGATGCCCCTTCAGAGTGTACATCCCCAACAAGCCCAGCAAGTAC
 
GGCATCAAGATCCTGATGATGTGCGACAGCGGCACCAAGTACATGATCAACGGCATGCCCTACCTGGGCAGA
GGCACCCAGACAAACGGCGTGCCCCTGGGCGAGTACTACGTGAAAGAACTGAGCAAGCCTGTGCATGGCAGC
TGCAGGAACATCACCTGCGACgcCTGGTTCACCAGCATCCCCCTGGCCAAGAACCTGCTGCAGGAACCCTACA
AGCTGACCATCGTGGGCACCGTGCGGAGCAACAAGgagGAGATCCCAGAGGTGCTGAAGAACAGCAGATCCAG
ACCTGTGGGAACAAGCATGTTCTGCTTCGACGGCCCCCTGACCCTGGTGTCCTACAAGCCCAAGCCCGCCAAG
ATGGTGTACCTGCTGTCCAGCTGCGACGAGGACGCCAGCATCAACGAGAGCACCGGCAAGCCCCAGATGGTG
ATGTACTACAACCAGACCAAGGGCGGCGTGGACACCCTGaACCAGATGTGCAGCGTGATGACCTGCAGCAGA
AAGACCAACAGATGGCCCATGGCCCTGCTGTACGGCATGATCAATATCGCCTGCATCAACAGCTTCATCATCT
ACAGCCACAACGTGTCCAGCAAGGGCGAGAAGGTGCAGAGCCGGAAGAAATTCATGCGGAACCTGTACATGA
GCCTGACCTCCAGCTTCATGAGAAAGAGACTGGAAGCCCCCACCCTGAAGAGATACCTGCGGGACAACATCA
GCAACATCCTGCCCAAGGAAGTGCCAGGAACAAGCGACGACAGCACCGAGGAACCCGTGATGAAGAAGAGG
ACCTACTGCACCTACTGTCCCAGCAAGATCAGAAGAAAGGCCAACGCCAGCTGCAAGAAATGCAAAAAAGTG
ATCTGCCGGGAGCACAACATCGACATGTGCCAGAGCTGTTTCTGAAAGGGTTCGATCCCTACCGGTTAGTAAT
GAGTTTAAACGGGGGAGGCTAACTGAAACACGGAAGGAGACAATACCGGAAGGAACCCGCGCTATGACGGC
AATAAAAAGACAGAATAAAACGCACGGGTGTTGGGTCGTTTGTTCATAAACGCGGGGTTCGGTCCCAGGGCT
GGCACTCTGTCGATACCCCACCGAGACCCCATTGGGGCCAATACGCCCGCGTTTCTTCCTTTTCCCCACCCCAC
CCCCCAAGTTCGGGTGAAGGCCCAGGGCTCGCAGCCAACGTCGGGGCGGCAGGCCCTGCCATAGCAGATCTG
CGCAGCTGGGGCTCTAGGGGGTATCCCCACGCGCCCTGTAGCGGCGCATTAAGCGCGGCGGGTGTGGTGGTTA
CGCGCAGCGTGACCGCTACACTTGCCAGCGCCCTAGCGCCCGCTCCTTTCGCTTTCTTCCCTTCCTTTCTCGCCA
CGTTCGCCGGCTTTCCCCGTCAAGCTCTAAATCGGGGGCTCCCTTTAGGGTTCCGATTTAGTGCTTTACGGCAC
CTCGACCCCAAAAAACTTGATTAGGGTGATGGTTCACGTAGTGGGCCATCGCCCTGATAGACGGTTTTTCGCC
CTTTGACGTTGGAGTCCACGTTCTTTAATAGTGGACTCTTGTTCCAAACTGGAACAACACTCAACCCTATCTCG
GTCTATTCTTTTGATTTATAAGGGATTTTGCCGATTTCGGCCTATTGGTTAAAAAATGAGCTGATTTAACAAAA
ATTTAACGCGAATTAATTCTGTGGAATGTGTGTCAGTTAGGGTGTGGAAAGTCCCCAGGCTCCCCAGCAGGCA
GAAGTATGCAAAGCATGCATCTCAATTAGTCAGCAACCAGGTGTGGAAAGTCCCCAGGCTCCCCAGCAGGCA
GAAGTATGCAAAGCATGCATCTCAATTAGTCAGCAACCATAGTCCCGCCCCTAACTCCGCCCATCCCGCCCCT
AACTCCGCCCAGTTCCGCCCATTCTCCGCCCCATGGCTGACTAATTTTTTTTATTTATGCAGAGGCCGAGGCCG
CCTCTGCCTCTGAGCTATTCCAGAAGTAGTGAGGAGGCTTTTTTGGAGGCCTAGGCTTTTGCAAAAAGCTCCCG
GGAGCTTGTATATCCATTTTCGGATCTGATCAAGAGACAGGATGAGGATCGTTTCGCATGATTGAACAAGATG
GATTGCACGCAGGTTCTCCGGCCGCTTGGGTGGAGAGGCTATTCGGCTATGACTGGGCACAACAGACAATCGG
CTGCTCTGATGCCGCCGTGTTCCGGCTGTCAGCGCAGGGGCGCCCGGTTCTTTTTGTCAAGACCGACCTGTCCG
GTGCCCTGAATGAACTGCAGGACGAGGCAGCGCGGCTATCGTGGCTGGCCACGACGGGCGTTCCTTGCGCAG
CTGTGCTCGACGTTGTCACTGAAGCGGGAAGGGACTGGCTGCTATTGGGCGAAGTGCCGGGGCAGGATCTCCT
GTCATCTCACCTTGCTCCTGCCGAGAAAGTATCCATCATGGCTGATGCAATGCGGCGGCTGCATACGCTTGATC
CGGCTACCTGCCCATTCGACCACCAAGCGAAACATCGCATCGAGCGAGCACGTACTCGGATGGAAGCCGGTCT
TGTCGATCAGGATGATCTGGACGAAGAGCATCAGGGGCTCGCGCCAGCCGAACTGTTCGCCAGGCTCAAGGC
GCGCATGCCCGACGGCGAGGATCTCGTCGTGACCCATGGCGATGCCTGCTTGCCGAATATCATGGTGGAAAAT
GGCCGCTTTTCTGGATTCATCGACTGTGGCCGGCTGGGTGTGGCGGACCGCTATCAGGACATAGCGTTGGCTA
 
CCCGTGATATTGCTGAAGAGCTTGGCGGCGAATGGGCTGACCGCTTCCTCGTGCTTTACGGTATCGCCGCTCCC
GATTCGCAGCGCATCGCCTTCTATCGCCTTCTTGACGAGTTCTTCTGAGCGGGACTCTGGGGTTCGCGAAATGA
CCGACCAAGCGACGCCCAACCTGCCATCACGAGATTTCGATTCCACCGCCGCCTTCTATGAAAGGTTGGGCTT
CGGAATCGTTTTCCGGGACGCCGGCTGGATGATCCTCCAGCGCGGGGATCTCATGCTGGAGTTCTTCGCCCAC
CCCAACTTGTTTATTGCAGCTTATAATGGTTACAAATAAAGCAATAGCATCACAAATTTCACAAATAAAGCAT
TTTTTTCACTGCATTCTAGTTGTGGTTTGTCCAAACTCATCAATGTATCTTATCATGTCTGTATACCGTCGACCT
CTAGCTAGAGCTTGGCGTAATCATGGTCATAGCTGTTTCCTGTGTGAAATTGTTATCCGCTCACAATTCCACAC
AACATACGAGCCGGAAGCATAAAGTGTAAAGCCTGGGGTGCCTAATGAGTGAGCTAACTCACATTAATTGCG
TTGCGCTCACTGCCCGCTTTCCAGTCGGGAAACCTGTCGTGCCAGCTGcattaatgaatcggccaacgcgcggggagaggcggtttgc
gtattgggcgctCTTCCGCTTCCTCGCTCACTGACTCGCTGCGCTCGGTCGTTCGGCTGCGGCGAGCGGTATCAGCTCA
CTCAAAGGCGGTAATACGGTTATCCACAGAATCAGGGGATAACGCAGGAAAGAACATGTGAGCAAAAGGCCA
GCAAAAGGCCAGGAACCGTAAAAAGGCCGCGTTGCTGGCGTTTTTCCATAGGCTCCGCCCCCCTGACGAGCAT
CACAAAAATCGACGCTCAAGTCAGAGGTGGCGAAACCCGACAGGACTATAAAGATACCAGGCGTTTCCCCCT
GGAAGCTCCCTCGTGCGCTCTCCTGTTCCGACCCTGCCGCTTACCGGATACCTGTCCGCCTTTCTCCCTTCGGG
AAGCGTGGCGCTTTCTCATAGCTCACGCTGTAGGTATCTCAGTTCGGTGTAGGTCGTTCGCTCCAAGCTGGGCT
GTGTGCACGAACCCCCCGTTCAGCCCGACCGCTGCGCCTTATCCGGTAACTATCGTCTTGAGTCCAACCCGGTA
AGACACGACTTATCGCCACTGGCAGCAGCCACTGGTAACAGGATTAGCAGAGCGAGGTATGTAGGCGGTGCT
ACAGAGTTCTTGAAGTGGTGGCCTAACTACGGCTACACTAGAAGAACAGTATTTGGTATCTGCGCTCTGCTGA
AGCCAGTTACCTTCGGAAAAAGAGTTGGTAGCTCTTGATCCGGCAAACAAACCACCGCTGGTAGCGGTGGTTT
TTTTGTTTGCAAGCAGCAGATTACGCGCAGAAAAAAAGGATCTCAAGAAGATCCTTTGATCTTTTCTACGGGG
TCTGACGCTCAGTGGAACGAAAACTCACGTTAAGGGATTTTGGTCATGAGATTATCAAAAAGGATCTTCACCT
AGATCCTTTTAAATTAAAAATGAAGTTTTAAATCAATCTAAAGTATATATGAGTAAACTTGGTCTGACAGTTAC
CAATGCTTAATCAGTGAGGCACCTATCTCAGCGATCTGTCTATTTCGTTCATCCATAGTTGCCTGACTCCCCGT
CGTGTAGATAACTACGATACGGGAGGGCTTACCATCTGGCCCCAGTGCTGCAATGATACCGCGAGACCCACGC
TCACCGGCTCCAGATTTATCAGCAATAAACCAGCCAGCCGGAAGGGCCGAGCGCAGAAGTGGTCCTGCAACT
TTATCCGCCTCCATCCAGTCTATTAATTGTTGCCGGGAAGCTAGAGTAAGTAGTTCGCCAGTTAATAGTTTGCG
CAACGTTGTTGCCATTGCTACAGGCATCGTGGTGTCACGCTCGTCGTTTGGTATGGCTTCATTCAGCTCCGGTT
CCCAACGATCAAGGCGAGTTACATGATCCCCCATGTTGTGCAAAAAAGCGGTTAGCTCCTTCGGTCCTCCGAT
CGTTGTCAGAAGTAAGTTGGCCGCAGTGTTATCACTCATGGTTATGGCAGCACTGCATAATTCTCTTACTGTCA
TGCCATCCGTAAGATGCTTTTCTGTGACTGGTGAGTACTCAACCAAGTCATTCTGAGAATAGTGTATGCGGCG
ACCGAGTTGCTCTTGCCCGGCGTCAATACGGGATAATACCGCGCCACATAGCAGAACTTTAAAAGTGCTCATC
ATTGGAAAACGTTCTTCGGGGCGAAAACTCTCAAGGATCTTACCGCTGTTGAGATCCAGTTCGATGTAACCCA
CTCGTGCACCCAACTGATCTTCAGCATCTTTTACTTTCACCAGCGTTTCTGGGTGAGCAAAAACAGGAAGGCA
AAATGCCGCAAAAAAGGGAATAAGGGCGACACGGAAATGTTGAATACTCATACTCTTCCTTTTTCAATATTAT
TGAAGCATTTATCAGGGTTATTGTCTCATGAGCGGATACATATTTGAATGTATTTAGAAAAATAAACAAATAG
GGGTTCCGCGCACATTTCCCCGAAAAGTGCCACCTGACGTCGACGGATCGGGAGATCTCCCGATCCCCTATGG
TGCACTCTCAGTACAATCTGCTCTGATGCCGCATAGTTAAGCCAGTATCTGCTCCCTGCTTGTGTGTTGGAGGT
CGCTGAGTAGTGCGCGAGCAAAATTTAAGCTACAACAAGGCAAGGCTTGACCGACAATTGCATGAAGAATCT
 
GCTTAGGGTTAGGCGTTTTGCGCTGCTTCGCGATGTACGGGCCAGAGGGCCTATTTCCCATGATTCCTTCATAT
TTGCATATACGATACAAGGCTGTTAGAGAGATAATTGGAATTAATTTGACTGTAAACACAAAGATATTAGTAC
AAAATACGTGACGTAGAAAGTAATAATTTCTTGGGTAGTTTGCAGTTTTAAAATTATGTTTTAAAATGGACTAT
CATATGCTTACCGTAACTTGAAAGTATTTCGATTTCTTGGCTTTATATATCTTGTGGAAAGGACGAAACACCGT
CGAGTCGCTTCTCGATTAGTTTTAGAGCTAGAAATAGCAAGTTAAAATAAGGCTAGTCCGTTATCAACTTGAA
AAAGTGGCACCGAGTCGGTGCTTTTTTGTTTTAGAGCTAGAAATAGCAAGTTAAAATAAGGCTAGTCCGTTTTT
AGCGCGTGCGCCAATTCTGCAGACAAATGATATACGCGTTGACATTGATTATTGACTAGTTATTAATAGTAAT
CAATTACGGGGTCATTAGTTCATAGCCCATATATGGAGTTCCGCGTTACATAACTTACGGTAAATGGCCCGCCT
GGCTGACCGCCCAACGACCCCCGCCCATTGACGTCAATAATGACGTATGTTCCCATAGTAACGCCAATAGGGA
CTTTCCATTGACGTCAATGGGTGGAGTATTTACGGTAAACTGCCCACTTGGCAGTACATCAAGTGTATCATATG
CCAAGTACGCCCCCTATTGACGTCAATGACGGTAAATGGCCCGCCTGGCATTATGCCCAGTACATGACCTTAT
GGGACTTTCCTACTTGGCAGTACATCTACGTATTAGTCATCGCTATTACCATG
 Note: The underlined part is the part encoding PBase.
Cas9-PBase N347A_R376E_D450N-GSH1gRNA plasmid sequence(SEQ ID NO:19) 
GTGATGCGGTTTTGGCAGTACATCAATGGGCGTGGATAGCGGTTTGACTCACGGGGATTTCCAAGTCTCCACC
CCATTGACGTCAATGGGAGTTTGTTTTGGCACCAAAATCAACGGGACTTTCCAAAATGTCGTAACAACTCCGC
CCCATTGACGCAAATGGGCGGTAGGCGTGTACGGTGGGAGGTCTATATAAGCAGAGCTCGTTTAGTGAACCGT
CAGATCGCCTGGAGACGCCATCCACGCTGTTTTGACCTCCATAGAAGACACCGGGACCGATCCAGCCTCCGGA
CTCTAGAGGATCGAACCCTTGCCACCATGGACAAGAAGTACTCCATTGGGCTCGacATCGGCACAAACAGCGT
CGGCTGGGCCGTCATTACGGACGAGTACAAGGTGCCGAGCAAAAAATTCAAAGTTCTGGGCAATACCGATCG
CCACAGCATAAAGAAGAACCTCATTGGCGCCCTCCTGTTCGACTCCGGGGAGACGGCCGAAGCCACGCGGCT
CAAAAGAACAGCACGGCGCAGATATACCCGCAGAAAGAATCGGATCTGCTACCTGCAGGAGATCTTTAGTAA
TGAGATGGCTAAGGTGGATGACTCTTTCTTCCATAGGCTGGAGGAGTCCTTTTTGGTGGAGGAGGATAAAAAG
CACGAGCGCCACCCAATCTTTGGCAATATCGTGGACGAGGTGGCGTACCATGAAAAGTACCCAACCATATATC
ATCTGAGGAAGAAGCTTGTAGACAGTACTGATAAGGCTGACTTGCGGTTGATCTATCTCGCGCTGGCGCATAT
GATCAAATTTCGGGGACACTTCCTCATCGAGGGGGACCTGAACCCAGACAACAGCGATGTCGACAAACTCTTT
ATCCAACTGGTTCAGACTTACAATCAGCTTTTCGAAGAGAACCCGATCAACGCATCCGGAGTTGACGCCAAAG
CAATCCTGAGCGCTAGGCTGTCCAAATCCCGGCGGCTCGAAAACCTCATCGCACAGCTCCCTGGGGAGAAGA
AGAACGGCCTGTTTGGTAATCTTATCGCCCTGTCACTCGGGCTGACCCCCAACTTTAAATCTAACTTCGACCTG
GCCGAAGATGCCAAGCTTCAACTGAGCAAAGACACCTACGATGATGATCTCGACAATCTGCTGGCCCAGATCG
GCGACCAGTACGCAGACCTTTTTTTGGCGGCAAAGAACCTGTCAGACGCCATTCTGCTGAGTGATATTCTGCG
AGTGAACACGGAGATCACCAAAGCTCCGCTGAGCGCTAGTATGATCAAGCGCTATGATGAGCACCACCAAGA
CTTGACTTTGCTGAAGGCCCTTGTCAGACAGCAACTGCCTGAGAAGTACAAGGAAATTTTCTTCGATCAGTCT
AAAAATGGCTACGCCGGATACATTGACGGCGGAGCAAGCCAGGAGGAATTTTACAAATTTATTAAGCCCATCT
TGGAAAAAATGGACGGCACCGAGGAGCTGCTGGTAAAGCTTAACAGAGAAGATCTGTTGCGCAAACAGCGCA
CTTTCGACAATGGAAGCATCCCCCACCAGATTCACCTGGGCGAACTGCACGCTATCCTCAGGCGGCAAGAGGA
TTTCTACCCCTTTTTGAAAGATAACAGGGAAAAGATTGAGAAAATCCTCACATTTCGGATACCCTACTATGTA
 
GGCCCCCTCGCCCGGGGAAATTCCAGATTCGCGTGGATGACTCGCAAATCAGAAGAGACCATCACTCCCTGGA
ACTTCGAGGAAGTCGTGGATAAGGGGGCCTCTGCCCAGTCCTTCATCGAAAGGATGACTAACTTTGATAAAAA
TCTGCCTAACGAAAAGGTGCTTCCTAAACACTCTCTGCTGTACGAGTACTTCACAGTTTATAACGAGCTCACCA
AGGTCAAATACGTCACAGAAGGGATGAGAAAGCCAGCATTCCTGTCTGGAGAGCAGAAGAAAGCTATCGTGG
ACCTCCTCTTCAAGACGAACCGGAAAGTTACCGTGAAACAGCTCAAAGAAGACTATTTCAAAAAGATTGAAT
GTTTCGACTCTGTTGAAATCAGCGGAGTGGAGGATCGCTTCAACGCATCCCTGGGAACGTATCACGATCTCCT
GAAAATCATTAAAGACAAGGACTTCCTGGACAATGAGGAGAACGAGGACATTCTTGAGGACATTGTCCTCAC
CCTTACGTTGTTTGAAGATAGGGAGATGATTGAAGAACGCTTGAAAACTTACGCTCATCTCTTCGACGACAAA
GTCATGAAACAGCTCAAGAGGCGCCGATATACAGGATGGGGGCGGCTGTCAAGAAAACTGATCAATGGGATC
CGAGACAAGCAGAGTGGAAAGACAATCCTGGATTTTCTTAAGTCCGATGGATTTGCCAACAGGAACTTCATGC
AGTTGATCCATGATGACTCTCTCACCTTTAAGGAGGACATCCAGAAAGCACAAGTTTCTGGCCAGGGGGACAG
TCTTCACGAGCACATCGCTAATCTTGCAGGTAGCCCAGCTATCAAAAAGGGAATACTGCAGACCGTTAAGGTC
GTGGATGAACTCGTCAAAGTAATGGGAAGGCATAAGCCCGAGAATATCGTTATCGAGATGGCCCGAGAGAAC
CAAACTACCCAGAAGGGACAGAAGAACAGTAGGGAAAGGATGAAGAGGATTGAAGAGGGTATAAAAGAACT
GGGGTCCCAAATCCTTAAGGAACACCCAGTTGAAAACACCCAGCTTCAGAATGAGAAGCTCTACCTGTACTAC
CTGCAGAACGGCAGGGACATGTACGTGGATCAGGAACTGGACATCAATCGGCTCTCCGACTACGACGTGGAT
CATATCGTGCCCCAGTCTTTTCTCAAAGATGATTCTATTGATAATAAAGTGTTGACAAGATCCGATAAAAATA
GAGGGAAGAGTGATAACGTCCCCTCAGAAGAAGTTGTCAAGAAAATGAAAAATTATTGGCGGCAGCTGCTGA
ACGCCAAACTGATCACACAACGGAAGTTCGATAATCTGACTAAGGCTGAACGAGGTGGCCTGTCTGAGTTGG
ATAAAGCCGGCTTCATCAAAAGGCAGCTTGTTGAGACACGCCAGATCACCAAGCACGTGGCCCAAATTCTCGA
TTCACGCATGAACACCAAGTACGATGAAAATGACAAACTGATTCGAGAGGTGAAAGTTATTACTCTGAAGTCT
AAGCTGGTCTCAGATTTCAGAAAGGACTTTCAGTTTTATAAGGTGAGAGAGATCAACAATTACCACCATGCGC
ATGATGCCTACCTGAATGCAGTGGTAGGCACTGCACTTATCAAAAAATATCCCAAGCTTGAATCTGAATTTGT
TTACGGAGACTATAAAGTGTACGATGTTAGGAAAATGATCGCAAAGTCTGAGCAGGAAATAGGCAAGGCCAC
CGCTAAGTACTTCTTTTACAGCAATATTATGAATTTTTTCAAGACCGAGATTACACTGGCCAATGGAGAGATTC
GGAAGCGACCACTTATCGAAACAAACGGAGAAACAGGAGAAATCGTGTGGGACAAGGGTAGGGATTTCGCG
ACAGTCCGGAAGGTCCTGTCCATGCCGCAGGTGAACATCGTTAAAAAGACCGAAGTACAGACCGGAGGCTTC
TCCAAGGAAAGTATCCTCCCGAAAAGGAACAGCGACAAGCTGATCGCACGCAAAAAAGATTGGGACCCCAAG
AAATACGGCGGATTCGATTCTCCTACAGTCGCTTACAGTGTACTGGTTGTGGCCAAAGTGGAGAAAGGGAAGT
CTAAAAAACTCAAAAGCGTCAAGGAACTGCTGGGCATCACAATCATGGAGCGATCAAGCTTCGAAAAAAACC
CCATCGACTTTCTCGAGGCGAAAGGATATAAAGAGGTCAAAAAAGACCTCATCATTAAGCTTCCCAAGTACTC
TCTCTTTGAGCTTGAAAACGGCCGGAAACGAATGCTCGCTAGTGCGGGCGAGCTGCAGAAAGGTAACGAGCT
GGCACTGCCCTCTAAATACGTTAATTTCTTGTATCTGGCCAGCCACTATGAAAAGCTCAAAGGGTCTCCCGAA
GATAATGAGCAGAAGCAGCTGTTCGTGGAACAACACAAACACTACCTTGATGAGATCATCGAGCAAATAAGC
GAATTCTCCAAAAGAGTGATCCTCGCCGACGCTAACCTCGATAAGGTGCTTTCTGCTTACAATAAGCACAGGG
ATAAGCCCATCAGGGAGCAGGCAGAAAACATTATCCACTTGTTTACTCTGACCAACTTGGGCGCGCCTGCAGC
CTTCAAGTACTTCGACACCACCATAGACAGAAAGCGGTACACCTCTACAAAGGAGGTCCTGGACGCCACACTG
ATTCATCAGTCAATTACGGGGCTCTATGAAACAAGAATCGACCTCTCTCAGCTCGGTGGAGACAGCAGGGCTG
 
ACCCCAAGAAGAAGAGGAAGGTGGGCGGAGGAGGGAGTGGCGGAGGCGGAAGCGGCGGCGGAGGCAGCGG
CTCTAGCCTGGACGACGAGCACATCCTGAGCGCCCTGCTGCAGAGCGACGACGAACTGGTGGGCGAGGACAG
CGACAGCGAGGTCAGCGACCACGTGTCCGAGGACGACGTGCAGTCCGACACCGAGGAAGCCTTCATCGACGA
GGTGCACGAAGTGCAGCCTACCAGCAGCGGCTCCGAGATCCTGGACGAGCAGAACGTGATCGAGCAGCCTGG
CAGCTCCCTGGCCAGCAACAGAATCCTGACCCTGCCCCAGAGAACCATCAGAGGCAAGAACAAGCACTGCTG
GTCCACCTCCAAGAGCACCAGGCGGAGCAGAGTGTCCGCCCTGAACATCGTGCGGAGCCAGAGGGGCCCCAC
CAGAATGTGCAGAAACATCTACGACCCCCTGCTGTGCTTCAAGCTGTTCTTCACCGACGAGATCATCAGCGAG
ATCGTGAAGTGGACCAACGCCGAGATCAGCCTGAAGAGGCGGGAGAGCATGACCAGCGCCACCTTCAGAGAC
ACCAACGAGGACGAGATCTACGCCTTCTTCGGCATCCTGGTGATGACCGCCGTGAGAAAGGACAACCACATG
AGCACCGACGACCTGTTCGACAGATCCCTGAGCATGGTGTACGTGTCCGTGATGAGCAGAGACAGATTCGACT
TCCTGATCAGATGCCTGAGAATGGACGACAAGAGCATCAGACCCACCCTGCGGGAGAACGACGTGTTCACCC
CCGTGCGGAAGATCTGGGACCTGTTCATCCACCAGTGCATCCAGAACTACACCCCTGGCGCCCACCTGACCAT
CGATGAGCAGCTGCTGGGCTTCAGAGGCAGATGCCCCTTCAGAGTGTACATCCCCAACAAGCCCAGCAAGTAC
GGCATCAAGATCCTGATGATGTGCGACAGCGGCACCAAGTACATGATCAACGGCATGCCCTACCTGGGCAGA
GGCACCCAGACAAACGGCGTGCCCCTGGGCGAGTACTACGTGAAAGAACTGAGCAAGCCTGTGCATGGCAGC
TGCAGGAACATCACCTGCGACgcCTGGTTCACCAGCATCCCCCTGGCCAAGAACCTGCTGCAGGAACCCTACA
AGCTGACCATCGTGGGCACCGTGCGGAGCAACAAGgagGAGATCCCAGAGGTGCTGAAGAACAGCAGATCCAG
ACCTGTGGGAACAAGCATGTTCTGCTTCGACGGCCCCCTGACCCTGGTGTCCTACAAGCCCAAGCCCGCCAAG
ATGGTGTACCTGCTGTCCAGCTGCGACGAGGACGCCAGCATCAACGAGAGCACCGGCAAGCCCCAGATGGTG
ATGTACTACAACCAGACCAAGGGCGGCGTGGACACCCTGaACCAGATGTGCAGCGTGATGACCTGCAGCAGA
AAGACCAACAGATGGCCCATGGCCCTGCTGTACGGCATGATCAATATCGCCTGCATCAACAGCTTCATCATCT
ACAGCCACAACGTGTCCAGCAAGGGCGAGAAGGTGCAGAGCCGGAAGAAATTCATGCGGAACCTGTACATGA
GCCTGACCTCCAGCTTCATGAGAAAGAGACTGGAAGCCCCCACCCTGAAGAGATACCTGCGGGACAACATCA
GCAACATCCTGCCCAAGGAAGTGCCAGGAACAAGCGACGACAGCACCGAGGAACCCGTGATGAAGAAGAGG
ACCTACTGCACCTACTGTCCCAGCAAGATCAGAAGAAAGGCCAACGCCAGCTGCAAGAAATGCAAAAAAGTG
ATCTGCCGGGAGCACAACATCGACATGTGCCAGAGCTGTTTCTGAAAGGGTTCGATCCCTACCGGTTAGTAAT
GAGTTTAAACGGGGGAGGCTAACTGAAACACGGAAGGAGACAATACCGGAAGGAACCCGCGCTATGACGGC
AATAAAAAGACAGAATAAAACGCACGGGTGTTGGGTCGTTTGTTCATAAACGCGGGGTTCGGTCCCAGGGCT
GGCACTCTGTCGATACCCCACCGAGACCCCATTGGGGCCAATACGCCCGCGTTTCTTCCTTTTCCCCACCCCAC
CCCCCAAGTTCGGGTGAAGGCCCAGGGCTCGCAGCCAACGTCGGGGCGGCAGGCCCTGCCATAGCAGATCTG
CGCAGCTGGGGCTCTAGGGGGTATCCCCACGCGCCCTGTAGCGGCGCATTAAGCGCGGCGGGTGTGGTGGTTA
CGCGCAGCGTGACCGCTACACTTGCCAGCGCCCTAGCGCCCGCTCCTTTCGCTTTCTTCCCTTCCTTTCTCGCCA
CGTTCGCCGGCTTTCCCCGTCAAGCTCTAAATCGGGGGCTCCCTTTAGGGTTCCGATTTAGTGCTTTACGGCAC
CTCGACCCCAAAAAACTTGATTAGGGTGATGGTTCACGTAGTGGGCCATCGCCCTGATAGACGGTTTTTCGCC
CTTTGACGTTGGAGTCCACGTTCTTTAATAGTGGACTCTTGTTCCAAACTGGAACAACACTCAACCCTATCTCG
GTCTATTCTTTTGATTTATAAGGGATTTTGCCGATTTCGGCCTATTGGTTAAAAAATGAGCTGATTTAACAAAA
ATTTAACGCGAATTAATTCTGTGGAATGTGTGTCAGTTAGGGTGTGGAAAGTCCCCAGGCTCCCCAGCAGGCA
GAAGTATGCAAAGCATGCATCTCAATTAGTCAGCAACCAGGTGTGGAAAGTCCCCAGGCTCCCCAGCAGGCA
 
GAAGTATGCAAAGCATGCATCTCAATTAGTCAGCAACCATAGTCCCGCCCCTAACTCCGCCCATCCCGCCCCT
AACTCCGCCCAGTTCCGCCCATTCTCCGCCCCATGGCTGACTAATTTTTTTTATTTATGCAGAGGCCGAGGCCG
CCTCTGCCTCTGAGCTATTCCAGAAGTAGTGAGGAGGCTTTTTTGGAGGCCTAGGCTTTTGCAAAAAGCTCCCG
GGAGCTTGTATATCCATTTTCGGATCTGATCAAGAGACAGGATGAGGATCGTTTCGCATGATTGAACAAGATG
GATTGCACGCAGGTTCTCCGGCCGCTTGGGTGGAGAGGCTATTCGGCTATGACTGGGCACAACAGACAATCGG
CTGCTCTGATGCCGCCGTGTTCCGGCTGTCAGCGCAGGGGCGCCCGGTTCTTTTTGTCAAGACCGACCTGTCCG
GTGCCCTGAATGAACTGCAGGACGAGGCAGCGCGGCTATCGTGGCTGGCCACGACGGGCGTTCCTTGCGCAG
CTGTGCTCGACGTTGTCACTGAAGCGGGAAGGGACTGGCTGCTATTGGGCGAAGTGCCGGGGCAGGATCTCCT
GTCATCTCACCTTGCTCCTGCCGAGAAAGTATCCATCATGGCTGATGCAATGCGGCGGCTGCATACGCTTGATC
CGGCTACCTGCCCATTCGACCACCAAGCGAAACATCGCATCGAGCGAGCACGTACTCGGATGGAAGCCGGTCT
TGTCGATCAGGATGATCTGGACGAAGAGCATCAGGGGCTCGCGCCAGCCGAACTGTTCGCCAGGCTCAAGGC
GCGCATGCCCGACGGCGAGGATCTCGTCGTGACCCATGGCGATGCCTGCTTGCCGAATATCATGGTGGAAAAT
GGCCGCTTTTCTGGATTCATCGACTGTGGCCGGCTGGGTGTGGCGGACCGCTATCAGGACATAGCGTTGGCTA
CCCGTGATATTGCTGAAGAGCTTGGCGGCGAATGGGCTGACCGCTTCCTCGTGCTTTACGGTATCGCCGCTCCC
GATTCGCAGCGCATCGCCTTCTATCGCCTTCTTGACGAGTTCTTCTGAGCGGGACTCTGGGGTTCGCGAAATGA
CCGACCAAGCGACGCCCAACCTGCCATCACGAGATTTCGATTCCACCGCCGCCTTCTATGAAAGGTTGGGCTT
CGGAATCGTTTTCCGGGACGCCGGCTGGATGATCCTCCAGCGCGGGGATCTCATGCTGGAGTTCTTCGCCCAC
CCCAACTTGTTTATTGCAGCTTATAATGGTTACAAATAAAGCAATAGCATCACAAATTTCACAAATAAAGCAT
TTTTTTCACTGCATTCTAGTTGTGGTTTGTCCAAACTCATCAATGTATCTTATCATGTCTGTATACCGTCGACCT
CTAGCTAGAGCTTGGCGTAATCATGGTCATAGCTGTTTCCTGTGTGAAATTGTTATCCGCTCACAATTCCACAC
AACATACGAGCCGGAAGCATAAAGTGTAAAGCCTGGGGTGCCTAATGAGTGAGCTAACTCACATTAATTGCG
TTGCGCTCACTGCCCGCTTTCCAGTCGGGAAACCTGTCGTGCCAGCTGcattaatgaatcggccaacgcgcggggagaggcggtttgc
gtattgggcgctCTTCCGCTTCCTCGCTCACTGACTCGCTGCGCTCGGTCGTTCGGCTGCGGCGAGCGGTATCAGCTCA
CTCAAAGGCGGTAATACGGTTATCCACAGAATCAGGGGATAACGCAGGAAAGAACATGTGAGCAAAAGGCCA
GCAAAAGGCCAGGAACCGTAAAAAGGCCGCGTTGCTGGCGTTTTTCCATAGGCTCCGCCCCCCTGACGAGCAT
CACAAAAATCGACGCTCAAGTCAGAGGTGGCGAAACCCGACAGGACTATAAAGATACCAGGCGTTTCCCCCT
GGAAGCTCCCTCGTGCGCTCTCCTGTTCCGACCCTGCCGCTTACCGGATACCTGTCCGCCTTTCTCCCTTCGGG
AAGCGTGGCGCTTTCTCATAGCTCACGCTGTAGGTATCTCAGTTCGGTGTAGGTCGTTCGCTCCAAGCTGGGCT
GTGTGCACGAACCCCCCGTTCAGCCCGACCGCTGCGCCTTATCCGGTAACTATCGTCTTGAGTCCAACCCGGTA
AGACACGACTTATCGCCACTGGCAGCAGCCACTGGTAACAGGATTAGCAGAGCGAGGTATGTAGGCGGTGCT
ACAGAGTTCTTGAAGTGGTGGCCTAACTACGGCTACACTAGAAGAACAGTATTTGGTATCTGCGCTCTGCTGA
AGCCAGTTACCTTCGGAAAAAGAGTTGGTAGCTCTTGATCCGGCAAACAAACCACCGCTGGTAGCGGTGGTTT
TTTTGTTTGCAAGCAGCAGATTACGCGCAGAAAAAAAGGATCTCAAGAAGATCCTTTGATCTTTTCTACGGGG
TCTGACGCTCAGTGGAACGAAAACTCACGTTAAGGGATTTTGGTCATGAGATTATCAAAAAGGATCTTCACCT
AGATCCTTTTAAATTAAAAATGAAGTTTTAAATCAATCTAAAGTATATATGAGTAAACTTGGTCTGACAGTTAC
CAATGCTTAATCAGTGAGGCACCTATCTCAGCGATCTGTCTATTTCGTTCATCCATAGTTGCCTGACTCCCCGT
CGTGTAGATAACTACGATACGGGAGGGCTTACCATCTGGCCCCAGTGCTGCAATGATACCGCGAGACCCACGC
TCACCGGCTCCAGATTTATCAGCAATAAACCAGCCAGCCGGAAGGGCCGAGCGCAGAAGTGGTCCTGCAACT
 
TTATCCGCCTCCATCCAGTCTATTAATTGTTGCCGGGAAGCTAGAGTAAGTAGTTCGCCAGTTAATAGTTTGCG
CAACGTTGTTGCCATTGCTACAGGCATCGTGGTGTCACGCTCGTCGTTTGGTATGGCTTCATTCAGCTCCGGTT
CCCAACGATCAAGGCGAGTTACATGATCCCCCATGTTGTGCAAAAAAGCGGTTAGCTCCTTCGGTCCTCCGAT
CGTTGTCAGAAGTAAGTTGGCCGCAGTGTTATCACTCATGGTTATGGCAGCACTGCATAATTCTCTTACTGTCA
TGCCATCCGTAAGATGCTTTTCTGTGACTGGTGAGTACTCAACCAAGTCATTCTGAGAATAGTGTATGCGGCG
ACCGAGTTGCTCTTGCCCGGCGTCAATACGGGATAATACCGCGCCACATAGCAGAACTTTAAAAGTGCTCATC
ATTGGAAAACGTTCTTCGGGGCGAAAACTCTCAAGGATCTTACCGCTGTTGAGATCCAGTTCGATGTAACCCA
CTCGTGCACCCAACTGATCTTCAGCATCTTTTACTTTCACCAGCGTTTCTGGGTGAGCAAAAACAGGAAGGCA
AAATGCCGCAAAAAAGGGAATAAGGGCGACACGGAAATGTTGAATACTCATACTCTTCCTTTTTCAATATTAT
TGAAGCATTTATCAGGGTTATTGTCTCATGAGCGGATACATATTTGAATGTATTTAGAAAAATAAACAAATAG
GGGTTCCGCGCACATTTCCCCGAAAAGTGCCACCTGACGTCGACGGATCGGGAGATCTCCCGATCCCCTATGG
TGCACTCTCAGTACAATCTGCTCTGATGCCGCATAGTTAAGCCAGTATCTGCTCCCTGCTTGTGTGTTGGAGGT
CGCTGAGTAGTGCGCGAGCAAAATTTAAGCTACAACAAGGCAAGGCTTGACCGACAATTGCATGAAGAATCT
GCTTAGGGTTAGGCGTTTTGCGCTGCTTCGCGATGTACGGGCCAGAGGGCCTATTTCCCATGATTCCTTCATAT
TTGCATATACGATACAAGGCTGTTAGAGAGATAATTGGAATTAATTTGACTGTAAACACAAAGATATTAGTAC
AAAATACGTGACGTAGAAAGTAATAATTTCTTGGGTAGTTTGCAGTTTTAAAATTATGTTTTAAAATGGACTAT
CATATGCTTACCGTAACTTGAAAGTATTTCGATTTCTTGGCTTTATATATCTTGTGGAAAGGACGAAACACCTT
AGTCCTAGTGCCATGAAGGTTTTAGAGCTAGAAATAGCAAGTTAAAATAAGGCTAGTCCGTTATCAACTTGAA
AAAGTGGCACCGAGTCGGTGCTTTTTTGTTTTAGAGCTAGAAATAGCAAGTTAAAATAAGGCTAGTCCGTTTTT
AGCGCGTGCGCCAATTCTGCAGACAAATGATATACGCGTTGACATTGATTATTGACTAGTTATTAATAGTAAT
CAATTACGGGGTCATTAGTTCATAGCCCATATATGGAGTTCCGCGTTACATAACTTACGGTAAATGGCCCGCCT
GGCTGACCGCCCAACGACCCCCGCCCATTGACGTCAATAATGACGTATGTTCCCATAGTAACGCCAATAGGGA
CTTTCCATTGACGTCAATGGGTGGAGTATTTACGGTAAACTGCCCACTTGGCAGTACATCAAGTGTATCATATG
CCAAGTACGCCCCCTATTGACGTCAATGACGGTAAATGGCCCGCCTGGCATTATGCCCAGTACATGACCTTAT
GGGACTTTCCTACTTGGCAGTACATCTACGTATTAGTCATCGCTATTACCATG
 Note: The underlined part is the part encoding PBase.
Cas9-PBase N347A_R376E_D450N-GSH2gRNA plasmid sequence(SEQ ID NO:20) 
GTGATGCGGTTTTGGCAGTACATCAATGGGCGTGGATAGCGGTTTGACTCACGGGGATTTCCAAGTCTCCACC
CCATTGACGTCAATGGGAGTTTGTTTTGGCACCAAAATCAACGGGACTTTCCAAAATGTCGTAACAACTCCGC
CCCATTGACGCAAATGGGCGGTAGGCGTGTACGGTGGGAGGTCTATATAAGCAGAGCTCGTTTAGTGAACCGT
CAGATCGCCTGGAGACGCCATCCACGCTGTTTTGACCTCCATAGAAGACACCGGGACCGATCCAGCCTCCGGA
CTCTAGAGGATCGAACCCTTGCCACCATGGACAAGAAGTACTCCATTGGGCTCGacATCGGCACAAACAGCGT
CGGCTGGGCCGTCATTACGGACGAGTACAAGGTGCCGAGCAAAAAATTCAAAGTTCTGGGCAATACCGATCG
CCACAGCATAAAGAAGAACCTCATTGGCGCCCTCCTGTTCGACTCCGGGGAGACGGCCGAAGCCACGCGGCT
CAAAAGAACAGCACGGCGCAGATATACCCGCAGAAAGAATCGGATCTGCTACCTGCAGGAGATCTTTAGTAA
TGAGATGGCTAAGGTGGATGACTCTTTCTTCCATAGGCTGGAGGAGTCCTTTTTGGTGGAGGAGGATAAAAAG
CACGAGCGCCACCCAATCTTTGGCAATATCGTGGACGAGGTGGCGTACCATGAAAAGTACCCAACCATATATC
 
ATCTGAGGAAGAAGCTTGTAGACAGTACTGATAAGGCTGACTTGCGGTTGATCTATCTCGCGCTGGCGCATAT
GATCAAATTTCGGGGACACTTCCTCATCGAGGGGGACCTGAACCCAGACAACAGCGATGTCGACAAACTCTTT
ATCCAACTGGTTCAGACTTACAATCAGCTTTTCGAAGAGAACCCGATCAACGCATCCGGAGTTGACGCCAAAG
CAATCCTGAGCGCTAGGCTGTCCAAATCCCGGCGGCTCGAAAACCTCATCGCACAGCTCCCTGGGGAGAAGA
AGAACGGCCTGTTTGGTAATCTTATCGCCCTGTCACTCGGGCTGACCCCCAACTTTAAATCTAACTTCGACCTG
GCCGAAGATGCCAAGCTTCAACTGAGCAAAGACACCTACGATGATGATCTCGACAATCTGCTGGCCCAGATCG
GCGACCAGTACGCAGACCTTTTTTTGGCGGCAAAGAACCTGTCAGACGCCATTCTGCTGAGTGATATTCTGCG
AGTGAACACGGAGATCACCAAAGCTCCGCTGAGCGCTAGTATGATCAAGCGCTATGATGAGCACCACCAAGA
CTTGACTTTGCTGAAGGCCCTTGTCAGACAGCAACTGCCTGAGAAGTACAAGGAAATTTTCTTCGATCAGTCT
AAAAATGGCTACGCCGGATACATTGACGGCGGAGCAAGCCAGGAGGAATTTTACAAATTTATTAAGCCCATCT
TGGAAAAAATGGACGGCACCGAGGAGCTGCTGGTAAAGCTTAACAGAGAAGATCTGTTGCGCAAACAGCGCA
CTTTCGACAATGGAAGCATCCCCCACCAGATTCACCTGGGCGAACTGCACGCTATCCTCAGGCGGCAAGAGGA
TTTCTACCCCTTTTTGAAAGATAACAGGGAAAAGATTGAGAAAATCCTCACATTTCGGATACCCTACTATGTA
GGCCCCCTCGCCCGGGGAAATTCCAGATTCGCGTGGATGACTCGCAAATCAGAAGAGACCATCACTCCCTGGA
ACTTCGAGGAAGTCGTGGATAAGGGGGCCTCTGCCCAGTCCTTCATCGAAAGGATGACTAACTTTGATAAAAA
TCTGCCTAACGAAAAGGTGCTTCCTAAACACTCTCTGCTGTACGAGTACTTCACAGTTTATAACGAGCTCACCA
AGGTCAAATACGTCACAGAAGGGATGAGAAAGCCAGCATTCCTGTCTGGAGAGCAGAAGAAAGCTATCGTGG
ACCTCCTCTTCAAGACGAACCGGAAAGTTACCGTGAAACAGCTCAAAGAAGACTATTTCAAAAAGATTGAAT
GTTTCGACTCTGTTGAAATCAGCGGAGTGGAGGATCGCTTCAACGCATCCCTGGGAACGTATCACGATCTCCT
GAAAATCATTAAAGACAAGGACTTCCTGGACAATGAGGAGAACGAGGACATTCTTGAGGACATTGTCCTCAC
CCTTACGTTGTTTGAAGATAGGGAGATGATTGAAGAACGCTTGAAAACTTACGCTCATCTCTTCGACGACAAA
GTCATGAAACAGCTCAAGAGGCGCCGATATACAGGATGGGGGCGGCTGTCAAGAAAACTGATCAATGGGATC
CGAGACAAGCAGAGTGGAAAGACAATCCTGGATTTTCTTAAGTCCGATGGATTTGCCAACAGGAACTTCATGC
AGTTGATCCATGATGACTCTCTCACCTTTAAGGAGGACATCCAGAAAGCACAAGTTTCTGGCCAGGGGGACAG
TCTTCACGAGCACATCGCTAATCTTGCAGGTAGCCCAGCTATCAAAAAGGGAATACTGCAGACCGTTAAGGTC
GTGGATGAACTCGTCAAAGTAATGGGAAGGCATAAGCCCGAGAATATCGTTATCGAGATGGCCCGAGAGAAC
CAAACTACCCAGAAGGGACAGAAGAACAGTAGGGAAAGGATGAAGAGGATTGAAGAGGGTATAAAAGAACT
GGGGTCCCAAATCCTTAAGGAACACCCAGTTGAAAACACCCAGCTTCAGAATGAGAAGCTCTACCTGTACTAC
CTGCAGAACGGCAGGGACATGTACGTGGATCAGGAACTGGACATCAATCGGCTCTCCGACTACGACGTGGAT
CATATCGTGCCCCAGTCTTTTCTCAAAGATGATTCTATTGATAATAAAGTGTTGACAAGATCCGATAAAAATA
GAGGGAAGAGTGATAACGTCCCCTCAGAAGAAGTTGTCAAGAAAATGAAAAATTATTGGCGGCAGCTGCTGA
ACGCCAAACTGATCACACAACGGAAGTTCGATAATCTGACTAAGGCTGAACGAGGTGGCCTGTCTGAGTTGG
ATAAAGCCGGCTTCATCAAAAGGCAGCTTGTTGAGACACGCCAGATCACCAAGCACGTGGCCCAAATTCTCGA
TTCACGCATGAACACCAAGTACGATGAAAATGACAAACTGATTCGAGAGGTGAAAGTTATTACTCTGAAGTCT
AAGCTGGTCTCAGATTTCAGAAAGGACTTTCAGTTTTATAAGGTGAGAGAGATCAACAATTACCACCATGCGC
ATGATGCCTACCTGAATGCAGTGGTAGGCACTGCACTTATCAAAAAATATCCCAAGCTTGAATCTGAATTTGT
TTACGGAGACTATAAAGTGTACGATGTTAGGAAAATGATCGCAAAGTCTGAGCAGGAAATAGGCAAGGCCAC
CGCTAAGTACTTCTTTTACAGCAATATTATGAATTTTTTCAAGACCGAGATTACACTGGCCAATGGAGAGATTC
 
GGAAGCGACCACTTATCGAAACAAACGGAGAAACAGGAGAAATCGTGTGGGACAAGGGTAGGGATTTCGCG
ACAGTCCGGAAGGTCCTGTCCATGCCGCAGGTGAACATCGTTAAAAAGACCGAAGTACAGACCGGAGGCTTC
TCCAAGGAAAGTATCCTCCCGAAAAGGAACAGCGACAAGCTGATCGCACGCAAAAAAGATTGGGACCCCAAG
AAATACGGCGGATTCGATTCTCCTACAGTCGCTTACAGTGTACTGGTTGTGGCCAAAGTGGAGAAAGGGAAGT
CTAAAAAACTCAAAAGCGTCAAGGAACTGCTGGGCATCACAATCATGGAGCGATCAAGCTTCGAAAAAAACC
CCATCGACTTTCTCGAGGCGAAAGGATATAAAGAGGTCAAAAAAGACCTCATCATTAAGCTTCCCAAGTACTC
TCTCTTTGAGCTTGAAAACGGCCGGAAACGAATGCTCGCTAGTGCGGGCGAGCTGCAGAAAGGTAACGAGCT
GGCACTGCCCTCTAAATACGTTAATTTCTTGTATCTGGCCAGCCACTATGAAAAGCTCAAAGGGTCTCCCGAA
GATAATGAGCAGAAGCAGCTGTTCGTGGAACAACACAAACACTACCTTGATGAGATCATCGAGCAAATAAGC
GAATTCTCCAAAAGAGTGATCCTCGCCGACGCTAACCTCGATAAGGTGCTTTCTGCTTACAATAAGCACAGGG
ATAAGCCCATCAGGGAGCAGGCAGAAAACATTATCCACTTGTTTACTCTGACCAACTTGGGCGCGCCTGCAGC
CTTCAAGTACTTCGACACCACCATAGACAGAAAGCGGTACACCTCTACAAAGGAGGTCCTGGACGCCACACTG
ATTCATCAGTCAATTACGGGGCTCTATGAAACAAGAATCGACCTCTCTCAGCTCGGTGGAGACAGCAGGGCTG
ACCCCAAGAAGAAGAGGAAGGTGGGCGGAGGAGGGAGTGGCGGAGGCGGAAGCGGCGGCGGAGGCAGCGG
CTCTAGCCTGGACGACGAGCACATCCTGAGCGCCCTGCTGCAGAGCGACGACGAACTGGTGGGCGAGGACAG
CGACAGCGAGGTCAGCGACCACGTGTCCGAGGACGACGTGCAGTCCGACACCGAGGAAGCCTTCATCGACGA
GGTGCACGAAGTGCAGCCTACCAGCAGCGGCTCCGAGATCCTGGACGAGCAGAACGTGATCGAGCAGCCTGG
CAGCTCCCTGGCCAGCAACAGAATCCTGACCCTGCCCCAGAGAACCATCAGAGGCAAGAACAAGCACTGCTG
GTCCACCTCCAAGAGCACCAGGCGGAGCAGAGTGTCCGCCCTGAACATCGTGCGGAGCCAGAGGGGCCCCAC
CAGAATGTGCAGAAACATCTACGACCCCCTGCTGTGCTTCAAGCTGTTCTTCACCGACGAGATCATCAGCGAG
ATCGTGAAGTGGACCAACGCCGAGATCAGCCTGAAGAGGCGGGAGAGCATGACCAGCGCCACCTTCAGAGAC
ACCAACGAGGACGAGATCTACGCCTTCTTCGGCATCCTGGTGATGACCGCCGTGAGAAAGGACAACCACATG
AGCACCGACGACCTGTTCGACAGATCCCTGAGCATGGTGTACGTGTCCGTGATGAGCAGAGACAGATTCGACT
TCCTGATCAGATGCCTGAGAATGGACGACAAGAGCATCAGACCCACCCTGCGGGAGAACGACGTGTTCACCC
CCGTGCGGAAGATCTGGGACCTGTTCATCCACCAGTGCATCCAGAACTACACCCCTGGCGCCCACCTGACCAT
CGATGAGCAGCTGCTGGGCTTCAGAGGCAGATGCCCCTTCAGAGTGTACATCCCCAACAAGCCCAGCAAGTAC
GGCATCAAGATCCTGATGATGTGCGACAGCGGCACCAAGTACATGATCAACGGCATGCCCTACCTGGGCAGA
GGCACCCAGACAAACGGCGTGCCCCTGGGCGAGTACTACGTGAAAGAACTGAGCAAGCCTGTGCATGGCAGC
TGCAGGAACATCACCTGCGACgcCTGGTTCACCAGCATCCCCCTGGCCAAGAACCTGCTGCAGGAACCCTACA
AGCTGACCATCGTGGGCACCGTGCGGAGCAACAAGgagGAGATCCCAGAGGTGCTGAAGAACAGCAGATCCAG
ACCTGTGGGAACAAGCATGTTCTGCTTCGACGGCCCCCTGACCCTGGTGTCCTACAAGCCCAAGCCCGCCAAG
ATGGTGTACCTGCTGTCCAGCTGCGACGAGGACGCCAGCATCAACGAGAGCACCGGCAAGCCCCAGATGGTG
ATGTACTACAACCAGACCAAGGGCGGCGTGGACACCCTGaACCAGATGTGCAGCGTGATGACCTGCAGCAGA
AAGACCAACAGATGGCCCATGGCCCTGCTGTACGGCATGATCAATATCGCCTGCATCAACAGCTTCATCATCT
ACAGCCACAACGTGTCCAGCAAGGGCGAGAAGGTGCAGAGCCGGAAGAAATTCATGCGGAACCTGTACATGA
GCCTGACCTCCAGCTTCATGAGAAAGAGACTGGAAGCCCCCACCCTGAAGAGATACCTGCGGGACAACATCA
GCAACATCCTGCCCAAGGAAGTGCCAGGAACAAGCGACGACAGCACCGAGGAACCCGTGATGAAGAAGAGG
ACCTACTGCACCTACTGTCCCAGCAAGATCAGAAGAAAGGCCAACGCCAGCTGCAAGAAATGCAAAAAAGTG
 
ATCTGCCGGGAGCACAACATCGACATGTGCCAGAGCTGTTTCTGAAAGGGTTCGATCCCTACCGGTTAGTAAT
GAGTTTAAACGGGGGAGGCTAACTGAAACACGGAAGGAGACAATACCGGAAGGAACCCGCGCTATGACGGC
AATAAAAAGACAGAATAAAACGCACGGGTGTTGGGTCGTTTGTTCATAAACGCGGGGTTCGGTCCCAGGGCT
GGCACTCTGTCGATACCCCACCGAGACCCCATTGGGGCCAATACGCCCGCGTTTCTTCCTTTTCCCCACCCCAC
CCCCCAAGTTCGGGTGAAGGCCCAGGGCTCGCAGCCAACGTCGGGGCGGCAGGCCCTGCCATAGCAGATCTG
CGCAGCTGGGGCTCTAGGGGGTATCCCCACGCGCCCTGTAGCGGCGCATTAAGCGCGGCGGGTGTGGTGGTTA
CGCGCAGCGTGACCGCTACACTTGCCAGCGCCCTAGCGCCCGCTCCTTTCGCTTTCTTCCCTTCCTTTCTCGCCA
CGTTCGCCGGCTTTCCCCGTCAAGCTCTAAATCGGGGGCTCCCTTTAGGGTTCCGATTTAGTGCTTTACGGCAC
CTCGACCCCAAAAAACTTGATTAGGGTGATGGTTCACGTAGTGGGCCATCGCCCTGATAGACGGTTTTTCGCC
CTTTGACGTTGGAGTCCACGTTCTTTAATAGTGGACTCTTGTTCCAAACTGGAACAACACTCAACCCTATCTCG
GTCTATTCTTTTGATTTATAAGGGATTTTGCCGATTTCGGCCTATTGGTTAAAAAATGAGCTGATTTAACAAAA
ATTTAACGCGAATTAATTCTGTGGAATGTGTGTCAGTTAGGGTGTGGAAAGTCCCCAGGCTCCCCAGCAGGCA
GAAGTATGCAAAGCATGCATCTCAATTAGTCAGCAACCAGGTGTGGAAAGTCCCCAGGCTCCCCAGCAGGCA
GAAGTATGCAAAGCATGCATCTCAATTAGTCAGCAACCATAGTCCCGCCCCTAACTCCGCCCATCCCGCCCCT
AACTCCGCCCAGTTCCGCCCATTCTCCGCCCCATGGCTGACTAATTTTTTTTATTTATGCAGAGGCCGAGGCCG
CCTCTGCCTCTGAGCTATTCCAGAAGTAGTGAGGAGGCTTTTTTGGAGGCCTAGGCTTTTGCAAAAAGCTCCCG
GGAGCTTGTATATCCATTTTCGGATCTGATCAAGAGACAGGATGAGGATCGTTTCGCATGATTGAACAAGATG
GATTGCACGCAGGTTCTCCGGCCGCTTGGGTGGAGAGGCTATTCGGCTATGACTGGGCACAACAGACAATCGG
CTGCTCTGATGCCGCCGTGTTCCGGCTGTCAGCGCAGGGGCGCCCGGTTCTTTTTGTCAAGACCGACCTGTCCG
GTGCCCTGAATGAACTGCAGGACGAGGCAGCGCGGCTATCGTGGCTGGCCACGACGGGCGTTCCTTGCGCAG
CTGTGCTCGACGTTGTCACTGAAGCGGGAAGGGACTGGCTGCTATTGGGCGAAGTGCCGGGGCAGGATCTCCT
GTCATCTCACCTTGCTCCTGCCGAGAAAGTATCCATCATGGCTGATGCAATGCGGCGGCTGCATACGCTTGATC
CGGCTACCTGCCCATTCGACCACCAAGCGAAACATCGCATCGAGCGAGCACGTACTCGGATGGAAGCCGGTCT
TGTCGATCAGGATGATCTGGACGAAGAGCATCAGGGGCTCGCGCCAGCCGAACTGTTCGCCAGGCTCAAGGC
GCGCATGCCCGACGGCGAGGATCTCGTCGTGACCCATGGCGATGCCTGCTTGCCGAATATCATGGTGGAAAAT
GGCCGCTTTTCTGGATTCATCGACTGTGGCCGGCTGGGTGTGGCGGACCGCTATCAGGACATAGCGTTGGCTA
CCCGTGATATTGCTGAAGAGCTTGGCGGCGAATGGGCTGACCGCTTCCTCGTGCTTTACGGTATCGCCGCTCCC
GATTCGCAGCGCATCGCCTTCTATCGCCTTCTTGACGAGTTCTTCTGAGCGGGACTCTGGGGTTCGCGAAATGA
CCGACCAAGCGACGCCCAACCTGCCATCACGAGATTTCGATTCCACCGCCGCCTTCTATGAAAGGTTGGGCTT
CGGAATCGTTTTCCGGGACGCCGGCTGGATGATCCTCCAGCGCGGGGATCTCATGCTGGAGTTCTTCGCCCAC
CCCAACTTGTTTATTGCAGCTTATAATGGTTACAAATAAAGCAATAGCATCACAAATTTCACAAATAAAGCAT
TTTTTTCACTGCATTCTAGTTGTGGTTTGTCCAAACTCATCAATGTATCTTATCATGTCTGTATACCGTCGACCT
CTAGCTAGAGCTTGGCGTAATCATGGTCATAGCTGTTTCCTGTGTGAAATTGTTATCCGCTCACAATTCCACAC
AACATACGAGCCGGAAGCATAAAGTGTAAAGCCTGGGGTGCCTAATGAGTGAGCTAACTCACATTAATTGCG
TTGCGCTCACTGCCCGCTTTCCAGTCGGGAAACCTGTCGTGCCAGCTGcattaatgaatcggccaacgcgcggggagaggcggtttgc
gtattgggcgctCTTCCGCTTCCTCGCTCACTGACTCGCTGCGCTCGGTCGTTCGGCTGCGGCGAGCGGTATCAGCTCA
CTCAAAGGCGGTAATACGGTTATCCACAGAATCAGGGGATAACGCAGGAAAGAACATGTGAGCAAAAGGCCA
GCAAAAGGCCAGGAACCGTAAAAAGGCCGCGTTGCTGGCGTTTTTCCATAGGCTCCGCCCCCCTGACGAGCAT
 
CACAAAAATCGACGCTCAAGTCAGAGGTGGCGAAACCCGACAGGACTATAAAGATACCAGGCGTTTCCCCCT
GGAAGCTCCCTCGTGCGCTCTCCTGTTCCGACCCTGCCGCTTACCGGATACCTGTCCGCCTTTCTCCCTTCGGG
AAGCGTGGCGCTTTCTCATAGCTCACGCTGTAGGTATCTCAGTTCGGTGTAGGTCGTTCGCTCCAAGCTGGGCT
GTGTGCACGAACCCCCCGTTCAGCCCGACCGCTGCGCCTTATCCGGTAACTATCGTCTTGAGTCCAACCCGGTA
AGACACGACTTATCGCCACTGGCAGCAGCCACTGGTAACAGGATTAGCAGAGCGAGGTATGTAGGCGGTGCT
ACAGAGTTCTTGAAGTGGTGGCCTAACTACGGCTACACTAGAAGAACAGTATTTGGTATCTGCGCTCTGCTGA
AGCCAGTTACCTTCGGAAAAAGAGTTGGTAGCTCTTGATCCGGCAAACAAACCACCGCTGGTAGCGGTGGTTT
TTTTGTTTGCAAGCAGCAGATTACGCGCAGAAAAAAAGGATCTCAAGAAGATCCTTTGATCTTTTCTACGGGG
TCTGACGCTCAGTGGAACGAAAACTCACGTTAAGGGATTTTGGTCATGAGATTATCAAAAAGGATCTTCACCT
AGATCCTTTTAAATTAAAAATGAAGTTTTAAATCAATCTAAAGTATATATGAGTAAACTTGGTCTGACAGTTAC
CAATGCTTAATCAGTGAGGCACCTATCTCAGCGATCTGTCTATTTCGTTCATCCATAGTTGCCTGACTCCCCGT
CGTGTAGATAACTACGATACGGGAGGGCTTACCATCTGGCCCCAGTGCTGCAATGATACCGCGAGACCCACGC
TCACCGGCTCCAGATTTATCAGCAATAAACCAGCCAGCCGGAAGGGCCGAGCGCAGAAGTGGTCCTGCAACT
TTATCCGCCTCCATCCAGTCTATTAATTGTTGCCGGGAAGCTAGAGTAAGTAGTTCGCCAGTTAATAGTTTGCG
CAACGTTGTTGCCATTGCTACAGGCATCGTGGTGTCACGCTCGTCGTTTGGTATGGCTTCATTCAGCTCCGGTT
CCCAACGATCAAGGCGAGTTACATGATCCCCCATGTTGTGCAAAAAAGCGGTTAGCTCCTTCGGTCCTCCGAT
CGTTGTCAGAAGTAAGTTGGCCGCAGTGTTATCACTCATGGTTATGGCAGCACTGCATAATTCTCTTACTGTCA
TGCCATCCGTAAGATGCTTTTCTGTGACTGGTGAGTACTCAACCAAGTCATTCTGAGAATAGTGTATGCGGCG
ACCGAGTTGCTCTTGCCCGGCGTCAATACGGGATAATACCGCGCCACATAGCAGAACTTTAAAAGTGCTCATC
ATTGGAAAACGTTCTTCGGGGCGAAAACTCTCAAGGATCTTACCGCTGTTGAGATCCAGTTCGATGTAACCCA
CTCGTGCACCCAACTGATCTTCAGCATCTTTTACTTTCACCAGCGTTTCTGGGTGAGCAAAAACAGGAAGGCA
AAATGCCGCAAAAAAGGGAATAAGGGCGACACGGAAATGTTGAATACTCATACTCTTCCTTTTTCAATATTAT
TGAAGCATTTATCAGGGTTATTGTCTCATGAGCGGATACATATTTGAATGTATTTAGAAAAATAAACAAATAG
GGGTTCCGCGCACATTTCCCCGAAAAGTGCCACCTGACGTCGACGGATCGGGAGATCTCCCGATCCCCTATGG
TGCACTCTCAGTACAATCTGCTCTGATGCCGCATAGTTAAGCCAGTATCTGCTCCCTGCTTGTGTGTTGGAGGT
CGCTGAGTAGTGCGCGAGCAAAATTTAAGCTACAACAAGGCAAGGCTTGACCGACAATTGCATGAAGAATCT
GCTTAGGGTTAGGCGTTTTGCGCTGCTTCGCGATGTACGGGCCAGAGGGCCTATTTCCCATGATTCCTTCATAT
TTGCATATACGATACAAGGCTGTTAGAGAGATAATTGGAATTAATTTGACTGTAAACACAAAGATATTAGTAC
AAAATACGTGACGTAGAAAGTAATAATTTCTTGGGTAGTTTGCAGTTTTAAAATTATGTTTTAAAATGGACTAT
CATATGCTTACCGTAACTTGAAAGTATTTCGATTTCTTGGCTTTATATATCTTGTGGAAAGGACGAAACACCCA
TCAGACTTGATAGCACTGGTTTTAGAGCTAGAAATAGCAAGTTAAAATAAGGCTAGTCCGTTATCAACTTGAA
AAAGTGGCACCGAGTCGGTGCTTTTTTGTTTTAGAGCTAGAAATAGCAAGTTAAAATAAGGCTAGTCCGTTTTT
AGCGCGTGCGCCAATTCTGCAGACAAATGATATACGCGTTGACATTGATTATTGACTAGTTATTAATAGTAAT
CAATTACGGGGTCATTAGTTCATAGCCCATATATGGAGTTCCGCGTTACATAACTTACGGTAAATGGCCCGCCT
GGCTGACCGCCCAACGACCCCCGCCCATTGACGTCAATAATGACGTATGTTCCCATAGTAACGCCAATAGGGA
CTTTCCATTGACGTCAATGGGTGGAGTATTTACGGTAAACTGCCCACTTGGCAGTACATCAAGTGTATCATATG
CCAAGTACGCCCCCTATTGACGTCAATGACGGTAAATGGCCCGCCTGGCATTATGCCCAGTACATGACCTTAT
GGGACTTTCCTACTTGGCAGTACATCTACGTATTAGTCATCGCTATTACCATG
 Note: The underlined part is the part encoding PBase.
NeoR-GFP transposon donor plasmid sequence(SEQ ID NO:21) 
CTGCAGAACACGCAGCTAGATTAACCCTAGAAAGATAATCATATTGTGACGTACGTTAAAGATAATCATGCGT
AAAATTGACGCATGTGTTTTATCGGTCTGTATATCGAGGTTTATTTATTAATTTGAATAGATATTAAGTTTTATT
ATATTTACACTTACATACTAATAATAAATTCAACAAACAATTTATTTATGTTTATTTATTTATTAAAAAAAAAC
AAAAACTCAAAATTTCTTCTATAAAGTAACAAAACTTTTATGAGGGACAGCCCCCCCCCAAAGCCCCCAGGGA
TGTAATTACGTCCCTCCCCCGCTAGGGGGCAGCAGCGAGCCGCCCGGGGCTCCGCTCCGGTCCGGCGCTCCCC
CCGCATCCCCGAGCCGGCAGCGTGCGGGGACAGCCCGGGCACGGGGAAGGTGGCACGGGATCGCTTTCCTCT
GAACGCTTCTCGCTGCTCTTTGAGCCTGCAGACACCTGGGGGGATACGGGGAAAAGGCCTCCACGGCCAGActa
gtATAACTTCGTATAGCATACATTATACGAAGTTATAATGTGTGTCAGTTAGGGTGTGGAAAGTCCCCAGGCTC
CCCAGCAGGCAGAAGTATGCAAAGCATGCATCTCAATTAGTCAGCAACCAGGTGTGGAAAGTCCCCAGGCTC
CCCAGCAGGCAGAAGTATGCAAAGCATGCATCTCAATTAGTCAGCAACCATAGTCCCGCCCCTAACTCCGCCC
ATCCCGCCCCTAACTCCGCCCAGTTCCGCCCATTCTCCGCCCCATGGCTGACTAATTTTTTTTATTTATGCAGAG
GCCGAGGCCGCCTCTGCCTCTGAGCTATTCCAGAAGTAGTGAGGAGGCTTTTTTGGAGGCCTAGGCTTTTGCA
AAAAGCTCCCGGGAGCTTGTATATCCATTTTCGGATCTGATCAGCACGTGTTGACAATTAATCATCGGCATAGT
ATATCGGCATAGTATAATACGACAAGGTGAGGAAtcgccACCATGATTGAACAAGATGGATTGCACGCAGGTTC
TCCGGCCGCTTGGGTGGAGAGGCTATTCGGCTATGACTGGGCACAACAGACAATCGGCTGCTCTGATGCCGCC
GTGTTCCGGCTGTCAGCGCAGGGGCGCCCGGTTCTTTTTGTCAAGACCGACCTGTCCGGTGCCCTGAATGAACT
GCAAGACGAGGCAGCGCGGCTATCGTGGCTGGCCACGACGGGCGTTCCTTGCGCAGCTGTGCTCGACGTTGTC
ACTGAAGCGGGAAGGGACTGGCTGCTATTGGGCGAAGTGCCGGGGCAGGATCTCCTGTCATCTCACCTTGCTC
CTGCCGAGAAAGTATCCATCATGGCTGATGCAATGCGGCGGCTGCATACGCTTGATCCGGCTACCTGCCCATT
CGACCACCAAGCGAAACATCGCATCGAGCGAGCACGTACTCGGATGGAAGCCGGTCTTGTCGATCAGGATGA
TCTGGACGAAGAGCATCAGGGGCTCGCGCCAGCCGAACTGTTCGCCAGGCTCAAGGCGAGCATGCCCGACGG
CGAGGATCTCGTCGTGACCCATGGCGATGCCTGCTTGCCGAATATCATGGTGGAAAATGGCCGCTTTTCTGGA
TTCATCGACTGTGGCCGGCTGGGTGTGGCGGACCGCTATCAGGACATAGCGTTGGCTACCCGTGATATTGCTG
AAGAGCTTGGCGGCGAATGGGCTGACCGCTTCCTCGTGCTTTACGGTATCGCCGCTCCCGATTCGCAGCGCAT
CGCCTTCTATCGCCTTCTTGACGAGTTCTTCTGAcgaCACCCCAACTTGTTTATTGCAGCTTATAATGGTTACAAA
TAAAGCAATAGCATCACAAATTTCACAAATAAAGCATTTTTTTCACTGCATTCTAGTTGTGGTTTGTCCAAACT
CATCAATGTATCTTATCATGTCTGATAACTTCGTATAGCATACATTATACGAAGTTATGCGGCCGCGAGCTCAC
GGGGACAGCCCCCCCCCAAAGCCCCCAGGGATGTAATTACGTCCCTCCCCCGCTAGGGGGCAGCAGCGAGCC
GCCCGGGGCTCCGCTCCGGTCCGGCGCTCCCCCCGCATCCCCGAGCCGGCAGCGTGCGGGGACAGCCCGGGC
ACGGGGAAGGTGGCACGGGATCGCTTTCCTCTGAACGCTTCTCGCTGCTCTTTGAGCCTGCAGACACCTGGGG
GGATACGGGGAAAAAGCTTTAGGCTGAAAGAGAGATTTAGAATGACAGAATCATAGAACGGCCTGGGTTGCA
AAGGAGCACAGTGCTCATCCAGATCCAACCCCCTGCTATGTGCAGGGTCATCAACCAGCAGCCCAGGCTGCCC
AGAGCCACATCCAGCCTGGCCTTGAATGCCTGCAGGGATGGGGCATCCACAGCCTCCTTGGGCAACCTGTTCA
GTGCGTCACCACCCTCTGGGGGAAAAACTGCCTCCTCATATCCAACCCAAACCTCCCCTGTCTCAGTGTAAAG
CCATTCCCCCTTGTCCTATCAAGGGGGAGTTTGCTGTGACATTGTTGGTCTGGGGTGACACATGTTTGCCAATT
 
CAGTGCATCACGGAGAGGCAGATCTTGGGGATAAGGAAGTGCAGGACAGCATGGACGTGGGACATGCAGGTG
TTGAGGGCTCTGGGACACTCTCCAAGTCACAGCGTTCAGAACAGCCTTAAGGATAAGAAGATAGGATAGAAG
GACAAAGAGCAAGTTAAAACCCAGCATGGAGAGGAGCACAAAAAGGCCACAGACACTGCTGGTCCCTGTGTC
TGAGCCTGCATGTTTGATGGTGTCTGGATGCAAGCAGAAGGGGTGGAAGAGCTTGCCTGGAGAGATACAGCT
GGGTCAGTAGGACTGGGACAGGCAGCTGGAGAATTGCCATGTAGATGTTCATACAATCGTCAAATCATGAAG
GCTGGAAAAGCCCTCCAAGATCCCCAAGACCAACCCCAACCCACCCACCGTGCCCACTGGCCATGTCCCTCAG
TGCCACATCCCCACAGTTCTTCATCACCTCCAGGGACGGTGACCCCCCCACCTCCGTGGGCAGCTGTGCCACTG
CAGCACCGCTCTTTGGAGAAGGTAAATCTTGCTAAATCCAGCCCGACCCTCCCCTGGCACAACGTAAGGCCAT
TATCTCTCATCCAACTCCAGGACGGAGTCAGTGAGAATATTgcGGCCGCtcttgaaaggagtgggaattggctccggtgcccgtcagtg
ggcagagcgcacatcgcccacagtccccgagaagttggggggaggggtcggcaattgaaccggtgcctagagaaggtggcgcggggtaaactgggaaagtgatgtcgtgtact
ggctccgcctttttcccgagggtgggggagaaccgtatataagtgcagtagtcgccgtgaacgttctttttcgcaacgggtttgccgccagaacacaggtaagtgccgtgtgtggttcc
cgcgggcctggcctctttacgggttatggcccttgcgtgccttgaattacttccacctggctgcagtacgtgattcttgatcccgagcttcgggttggaagtgggtgggagagttcgagg
ccttgcgcttaaggagccccttcgcctcgtgcttgagttgaggcctggcctgggcgctggggccgccgcgtgcgaatctggtggcaccttcgcgcctgtctcgctgctttcgataagtc
tctagccatttaaaatttttgatgacctgctgcgacgctttttttctggcaagatagtcttgtaaatgcgggccaagatctgcacactggtatttcggtttttggggccgcgggcggcgacgg
ggcccgtgcgtcccagcgcacatgttcggcgaggcggggcctgcgagcgcggccaccgagaatcggacgggggtagtctcaagctggccggcctgctctggtgcctggcctcg
cgccgccgtgtatcgccccgccctgggcggcaaggctggcccggtcggcaccagttgcgtgagcggaaagatggccgcttcccggccctgctgcagggagctcaaaatggagg
acgcggcgctcgggagagcgggcgggtgagtcacccacacaaaggaaaagggcctttccgtcctcagccgtcgcttcatgtgactccacggagtaccgggcgccgtccaggcac
ctcgattagttctcgagcttttggagtacgtcgtctttaggttggggggaggggttttatgcgatggagtttccccacactgagtgggtggagactgaagttaggccagcttggcacttga
tgtaattctccttggaatttgccctttttgagtttggatcttggttcattctcaagcctcagacagtggttcaaagtttttttcttccatttcaggtgtcgtgaggAATTAGCTAGCTT
CGAGGGGCTCGCATCTCTCCTTCACGCGCCCGCCGCCCTACCTGAGGCCGCCATCCACGCCGGTTGAGTCGCG
TTCTGCCGCCTCCCGCCTGTGGTGCCTCCTGAACTGCGTCCGCCGTCTAGGTAAGTTTAAAGCTCAGGTCGAGA
CCGGGCCTTTGTCCGGCGCTCCCTTGGAGCCTACCTAGACTCAGCCGGCTCTCCACGCTTTGCCTGACCCTGCT
TGCTCAACTCTACGTCTTTGTTTCGTTTTCTGTTCTGCGCCGTTACAGATCCAAGCTGTGACCGGCGCCTACGGT
ACCATGGTGAGCAAGGGCGAGGAGCTGTTCACCGGGGTGGTGCCCATCCTGGTCGAGCTGGACGGCGACGTA
AACGGCCACAAGTTCAGCGTGTCCGGCGAGGGCGAGGGCGATGCCACCTACGGCAAGCTGACCCTGAAGTTC
ATCTGCACCACCGGCAAGCTGCCCGTGCCCTGGCCCACCCTCGTGACCACCCTGACCTACGGCGTGCAGTGCT
TCAGCCGCTACCCCGACCACATGAAGCAGCACGACTTCTTCAAGTCCGCCATGCCCGAAGGCTACGTCCAGGA
GCGCACCATCTTCTTCAAGGACGACGGCAACTACAAGACCCGCGCCGAGGTGAAGTTCGAGGGCGACACCCT
GGTGAACCGCATCGAGCTGAAGGGCATCGACTTCAAGGAGGACGGCAACATCCTGGGGCACAAGCTGGAGTA
CAACTACAACAGCCACAACGTCTATATCATGGCCGACAAGCAGAAGAACGGCATCAAGGTGAACTTCAAGAT
CCGCCACAACATCGAGGACGGCAGCGTGCAGCTCGCCGACCACTACCAGCAGAACACCCCCATCGGCGACGG
CCCCGTGCTGCTGCCCGACAACCACTACCTGAGCACCCAGTCCGCCCTGAGCAAAGACCCCAACGAGAAGCG
CGATCACATGGTCCTGCTGGAGTTCGTGACCGCCGCCGGGATCACTCTCGGCATGGACGAGCTGTACAAGTAA
GAATTCGTCGACTGTACAACGCGTGTTTAAACTTCGAATCTAGATACGTACATATGATTTAaagctcgctttcttgctgtcca
atttctattaaaggttcctttgttccctaagtccaactactaaactgggggatattatgaagggccttgagcatctggattctgcctaataaaaaacatttattttcattgcaatgatgtatttaaa
ttatttctgaatattttactaaaaagggaatgtgggaggtcagtgcatttaaaacataaagaaatgaagagctagttcaaaccttgggaaaatacactatatcttaaactccatgaaagaag
gtgaggctgcaaacagctaatgcacattggcaacagcccctgatgcctatgccttattcatccctcagaaaaggattcaagtagaggcttgatttggaggttaaagttttgctatgctgtat
tttacGATATCGAGCTCACGGGGACAGCCCCCCCCCAAAGCCCCCAGGGATGTAATTACGTCCCTCCCCCGCTAG
 
GGGGCAGCAGCGAGCCGCCCGGGGCTCCGCTCCGGTCCGGCGCTCCCCCCGCATCCCCGAGCCGGCAGCGTG
CGGGGACAGCCCGGGCACGGGGAAGGTGGCACGGGATCGCTTTCCTCTGAACGCTTCTCGCTGCTCTTTGAGC
CTGCAGACACCTGGGGGGATACGGGGAAAAAGCTTTAGGCTGAAAGAGAGATTTAGAATGACAGAATCATAG
AACGGCCTGGGTTGCAAAGGAGCACAGTGCTCATCCAGATCCAACCCCCTGCTATGTGCAGGGTCATCAACCA
GCAGCCCAGGCTGCCCAGAGCCACATCCAGCCTGGCCTTGAATGCCTGCAGGGATGGGGCATCCACAGCCTCC
TTGGGCAACCTGTTCAGTGCGTCACCACCCTCTGGGGGAAAAACTGCCTCCTCATATCCAACCCAAACCTCCC
CTGTCTCAGTGTAAAGCCATTCCCCCTTGTCCTATCAAGGGGGAGTTTGCTGTGACATTGTTGGTCTGGGGTGA
CACATGTTTGCCAATTCAGTGCATCACGGAGAGGCAGATCTTGGGGATAAGGAAGTGCAGGACAGCATGGAC
GTGGGACATGCAGGTGTTGAGGGCTCTGGGACACTCTCCAAGTCACAGCGTTCAGAACAGCCTTAAGGATAA
GAAGATAGGATAGAAGGACAAAGAGCAAGTTAAAACCCAGCATGGAGAGGAGCACAAAAAGGCCACAGACA
CTGCTGGTCCCTGTGTCTGAGCCTGCATGTTTGATGGTGTCTGGATGCAAGCAGAAGGGGTGGAAGAGCTTGC
CTGGAGAGATACAGCTGGGTCAGTAGGACTGGGACAGGCAGCTGGAGAATTGCCATGTAGATGTTCATACAA
TCGTCAAATCATGAAGGCTGGAAAAGCCCTCCAAGATCCCCAAGACCAACCCCAACCCACCCACCGTGCCCAC
TGGCCATGTCCCTCAGTGCCACATCCCCACAGTTCTTCATCACCTCCAGGGACGGTGACCCCCCCACCTCCGTG
GGCAGCTGTGCCACTGCAGCACCGCTCTTTGGAGAAGGTAAATCTTGCTAAATCCAGCCCGACCCTCCCCTGG
CACAACGTAAGGCCATTATCTCTCATCCAACTCCAGGACGGAGTCAGTGAGAATATTGATATCTATAACAAGA
AAATATATATATAATAAGTTATCACGTAAGTAGAACATGAAATAACAATATAATTATCGTATGAGTTAAATCT
TAAAAGTCACGTAAAAGATAATCATGCGTCATTTTGACTCACGCGGTCGTTATAGTTCAAAATCAGTGACACT
TACCGCATTGACAAGCACGCCTCACGGGAGCTCCAAGCGGCGACTGAGATGTCCTAAATGCACAGCGACGGA
TTCGCGCTATTTAGAAAGAGAGAGCAATATTTCAAGAATGCATGCGTCAATTTTACGCAGACTATCTTTCTAG
GGTTAATCTAGCTGCATCAGGATCATATCGTCGGGTCTTTTTTCCGGCTCAGTCATCGCCCAAGCTGGCGCTAT
CTGGGCATCGGGGAGGAAGAAGCCCGTGCCTTTTCCCGCGAGGTTGAAGCGGCATGGAAAGAGTTTGCCGAG
GATGACTGCTGCTGCATTGACGTTGAGCGAAAACGCACGTTTACCATGATGATTCGGGAAGGTGTGGCCATGC
ACGCCTTTAACGGTGAACTGTTCGTTCAGGCCACCTGGGATACCAGTTCGTCGCGGCTTTTCCGGACACAGTTC
CGGATGGTCAGCCCGAAGCGCATCAGCAACCCGAACAATACCGGCGACAGCCGGAACTGCCGTGCCGGTGTG
CAGATTAATGACAGCGGTGCGGCGCTGGGATATTACGTCAGCGAGGACGGGTATCCTGGCTGGATGCCGCAG
AAATGGACATGGATACCCCGTGAGTTACCCGGCGGGCGCGCTTGGCGTAATCATGGTCATAGCTGTTTCCTGT
GTGAAATTGTTATCCGCTCACAATTCCACACAACATACGAGCCGGAAGCATAAAGTGTAAAGCCTGGGGTGCC
TAATGAGTGAGCTAACTCACATTAATTGCGTTGCGCTCACTGCCCGCTTTCCAGTCGGGAAACCTGTCGTGCCA
GCTGCATTAATGAATCGGCCAACGCGCGGGGAGAGGCGGTTTGCGTATTGGGCGCTCTTCCGCTTCCTCGCTC
ACTGACTCGCTGCGCTCGGTCGTTCGGCTGCGGCGAGCGGTATCAGCTCACTCAAAGGCGGTAATACGGTTAT
CCACAGAATCAGGGGATAACGCAGGAAAGAACATGTGAGCAAAAGGCCAGCAAAAGGCCAGGAACCGTAAA
AAGGCCGCGTTGCTGGCGTTTTTCCATAGGCTCCGCCCCCCTGACGAGCATCACAAAAATCGACGCTCAAGTC
AGAGGTGGCGAAACCCGACAGGACTATAAAGATACCAGGCGTTTCCCCCTGGAAGCTCCCTCGTGCGCTCTCC
TGTTCCGACCCTGCCGCTTACCGGATACCTGTCCGCCTTTCTCCCTTCGGGAAGCGTGGCGCTTTCTCATAGCT
CACGCTGTAGGTATCTCAGTTCGGTGTAGGTCGTTCGCTCCAAGCTGGGCTGTGTGCACGAACCCCCCGTTCAG
CCCGACCGCTGCGCCTTATCCGGTAACTATCGTCTTGAGTCCAACCCGGTAAGACACGACTTATCGCCACTGG
CAGCAGCCACTGGTAACAGGATTAGCAGAGCGAGGTATGTAGGCGGTGCTACAGAGTTCTTGAAGTGGTGGC
 
CTAACTACGGCTACACTAGAAGAACAGTATTTGGTATCTGCGCTCTGCTGAAGCCAGTTACCTTCGGAAAAAG
AGTTGGTAGCTCTTGATCCGGCAAACAAACCACCGCTGGTAGCGGTGGTTTTTTTGTTTGCAAGCAGCAGATT
ACGCGCAGAAAAAAAGGATCTCAAGAAGATCCTTTGATCTTTTCTACGGGGTCTGACGCTCAGTGGAACGAAA
ACTCACGTTAAGGGATTTTGGTCATGAgtgcgcggaacccctatttgtttatttttctaaatacattcaaatatgtatccgctcatgagacaataaccctgataaat
gcttcaataatattgaaaaaggaagagtATGAGGGAAGCGGTGATCGCCGAAGTATCGACTCAACTATCAGAGGTAGTTGGCG
TCATCGAGCGCCATCTCGAACCGACGTTGCTGGCCGTACATTTGTACGGCTCCGCAGTGGATGGCGGCCTGAA
GCCACACAGTGATATTGATTTGCTGGTTACGGTGACCGTAAGGCTTGATGAAACAACGCGGCGAGCTTTGATC
AACGACCTTTTGGAAACTTCGGCTTCCCCTGGAGAGAGCGAGATTCTCCGCGCTGTAGAAGTCACCATTGTTG
TGCACGACGACATCATTCCGTGGCGTTATCCAGCTAAGCGCGAACTGCAATTTGGAGAATGGCAGCGCAATGA
CATTCTTGCAGGTATCTTCGAGCCAGCCACGATCGACATTGATCTGGCTATCTTGCTGACAAAAGCAAGAGAA
CATAGCGTTGCCTTGGTAGGTCCAGCGGCGGAGGAACTCTTTGATCCGGTTCCTGAACAGGATCTATTTGAGG
CGCTAAATGAAACCTTAACGCTATGGAACTCGCCGCCCGACTGGGCTGGCGATGAGCGAAATGTAGTGCTTAC
GTTGTCCCGCATTTGGTACAGCGCAGTAACCGGCAAAATCGCGCCGAAGGATGTCGCTGCCGACTGGGCAATG
GAGCGCCTGCCGGCCCAGTATCAGCCCGTCATACTTGAAGCTAGACAGGCTTATCTTGGACAAGAAGATC
GCTTGGCCTCGCGCGCAGATCAGTTGGAAGAATTTGTCCACTACGTGAAAGGCGAGATCACCAAGGTAGTCGG
CAAATAACCCTCGAGCCACCCATGACCAAAATCCCTTAACGTGAGTTA
U6-ROSA sgRNA(SEQ ID NO:131) 
GAGGGCCTATTTCCCATGATTCCTTCATATTTGCATATACGATACAAGGCTGTTAGAGAGATAATTGGAA
TTAATTTGACTGTAAACACAAAGATATTAGTACAAAATACGTGACGTAGAAAGTAATAATTTCTTGGGTAGTT
TGCAGTTTTAAAATTATGTTTTAAAATGGACTATCATATGCTTACCGTAACTTGAAAGTATTTCGATTTCTTGG
CTTTATATATCTTGTGGAAAGGACGAAACACCGTCGAGTCGCTTCTCGATTAGTTTTAGAGCTAGAAATAGCA
AGTTAAAATAAGGCTAGTCCGTTATCAACTTGAAAAAGTGGCACCGAGTCGGTGCTTTTTTGTTTTAGAGCTA
GAAATAGCAAGTTAAAATAAGGCTAGTCCGTT
wt Hyperactive PBase encoding DNA sequence(SEQ ID NO:138) 
ATGGGCTCTAGCCTGGACGACGAGCACATCCTGAGCGCCCTGCTGCAGAGCGACGACGAACTGGTGGGC
GAGGACAGCGACAGCGAGGTCAGCGACCACGTGTCCGAGGACGACGTGCAGTCCGACACCGAGGAAGCCTTC
ATCGACGAGGTGCACGAAGTGCAGCCTACCAGCAGCGGCTCCGAGATCCTGGACGAGCAGAACGTGATCGAG
CAGCCTGGCAGCTCCCTGGCCAGCAACAGAATCCTGACCCTGCCCCAGAGAACCATCAGAGGCAAGAACAAG
CACTGCTGGTCCACCTCCAAGAGCACCAGGCGGAGCAGAGTGTCCGCCCTGAACATCGTGCGGAGCCAGAGG
GGCCCCACCAGAATGTGCAGAAACATCTACGACCCCCTGCTGTGCTTCAAGCTGTTCTTCACCGACGAGATCA
TCAGCGAGATCGTGAAGTGGACCAACGCCGAGATCAGCCTGAAGAGGCGGGAGAGCATGACCAGCGCCACCT
TCAGAGACACCAACGAGGACGAGATCTACGCCTTCTTCGGCATCCTGGTGATGACCGCCGTGAGAAAGGACA
ACCACATGAGCACCGACGACCTGTTCGACAGATCCCTGAGCATGGTGTACGTGTCCGTGATGAGCAGAGACA
GATTCGACTTCCTGATCAGATGCCTGAGAATGGACGACAAGAGCATCAGACCCACCCTGCGGGAGAACGACG
TGTTCACCCCCGTGCGGAAGATCTGGGACCTGTTCATCCACCAGTGCATCCAGAACTACACCCCTGGCGCCCA
CCTGACCATCGATGAGCAGCTGCTGGGCTTCAGAGGCAGATGCCCCTTCAGAGTGTACATCCCCAACAAGCCC
 
AGCAAGTACGGCATCAAGATCCTGATGATGTGCGACAGCGGCACCAAGTACATGATCAACGGCATGCCCTAC
CTGGGCAGAGGCACCCAGACAAACGGCGTGCCCCTGGGCGAGTACTACGTGAAAGAACTGAGCAAGCCTGTG
CATGGCAGCTGCAGGAACATCACCTGCGACAACTGGTTCACCAGCATCCCCCTGGCCAAGAACCTGCTGCAGG
AACCCTACAAGCTGACCATCGTGGGCACCGTGCGGAGCAACAAGCGGGAGATCCCAGAGGTGCTGAAGAACA
GCAGATCCAGACCTGTGGGAACAAGCATGTTCTGCTTCGACGGCCCCCTGACCCTGGTGTCCTACAAGCCCAA
GCCCGCCAAGATGGTGTACCTGCTGTCCAGCTGCGACGAGGACGCCAGCATCAACGAGAGCACCGGCAAGCC
CCAGATGGTGATGTACTACAACCAGACCAAGGGCGGCGTGGACACCCTGGACCAGATGTGCAGCGTGATGAC
CTGCAGCAGAAAGACCAACAGATGGCCCATGGCCCTGCTGTACGGCATGATCAATATCGCCTGCATCAACAGC
TTCATCATCTACAGCCACAACGTGTCCAGCAAGGGCGAGAAGGTGCAGAGCCGGAAGAAATTCATGCGGAAC
CTGTACATGAGCCTGACCTCCAGCTTCATGAGAAAGAGACTGGAAGCCCCCACCCTGAAGAGATACCTGCGG
GACAACATCAGCAACATCCTGCCCAAGGAAGTGCCAGGAACAAGCGACGACAGCACCGAGGAACCCGTGATG
AAGAAGAGGACCTACTGCACCTACTGTCCCAGCAAGATCAGAAGAAAGGCCAACGCCAGCTGCAAGAAATGC
AAAAAAGTGATCTGCCGGGAGCACAACATCGACATGTGCCAGAGCTGTTTCTGA
Cas9-PB-ROSA sgRNA vector(SEQ ID NO:139): 
GTGATGCGGTTTTGGCAGTACATCAATGGGCGTGGATAGCGGTTTGACTCACGGGGATTTCCAAGTCTCC
ACCCCATTGACGTCAATGGGAGTTTGTTTTGGCACCAAAATCAACGGGACTTTCCAAAATGTCGTAACAACTC
CGCCCCATTGACGCAAATGGGCGGTAGGCGTGTACGGTGGGAGGTCTATATAAGCAGAGCTCGTTTAGTGAAC
CGTCAGATCGCCTGGAGACGCCATCCACGCTGTTTTGACCTCCATAGAAGACACCGGGACCGATCCAGCCTCC
GGACTCTAGAGGATCGAACCCTTGCCACCATGGACAAGAAGTACTCCATTGGGCTCGacATCGGCACAAACAG
CGTCGGCTGGGCCGTCATTACGGACGAGTACAAGGTGCCGAGCAAAAAATTCAAAGTTCTGGGCAATACCGA
TCGCCACAGCATAAAGAAGAACCTCATTGGCGCCCTCCTGTTCGACTCCGGGGAGACGGCCGAAGCCACGCG
GCTCAAAAGAACAGCACGGCGCAGATATACCCGCAGAAAGAATCGGATCTGCTACCTGCAGGAGATCTTTAG
TAATGAGATGGCTAAGGTGGATGACTCTTTCTTCCATAGGCTGGAGGAGTCCTTTTTGGTGGAGGAGGATAAA
AAGCACGAGCGCCACCCAATCTTTGGCAATATCGTGGACGAGGTGGCGTACCATGAAAAGTACCCAACCATA
TATCATCTGAGGAAGAAGCTTGTAGACAGTACTGATAAGGCTGACTTGCGGTTGATCTATCTCGCGCTGGCGC
ATATGATCAAATTTCGGGGACACTTCCTCATCGAGGGGGACCTGAACCCAGACAACAGCGATGTCGACAAACT
CTTTATCCAACTGGTTCAGACTTACAATCAGCTTTTCGAAGAGAACCCGATCAACGCATCCGGAGTTGACGCC
AAAGCAATCCTGAGCGCTAGGCTGTCCAAATCCCGGCGGCTCGAAAACCTCATCGCACAGCTCCCTGGGGAG
AAGAAGAACGGCCTGTTTGGTAATCTTATCGCCCTGTCACTCGGGCTGACCCCCAACTTTAAATCTAACTTCGA
CCTGGCCGAAGATGCCAAGCTTCAACTGAGCAAAGACACCTACGATGATGATCTCGACAATCTGCTGGCCCAG
ATCGGCGACCAGTACGCAGACCTTTTTTTGGCGGCAAAGAACCTGTCAGACGCCATTCTGCTGAGTGATATTC
TGCGAGTGAACACGGAGATCACCAAAGCTCCGCTGAGCGCTAGTATGATCAAGCGCTATGATGAGCACCACC
AAGACTTGACTTTGCTGAAGGCCCTTGTCAGACAGCAACTGCCTGAGAAGTACAAGGAAATTTTCTTCGATCA
GTCTAAAAATGGCTACGCCGGATACATTGACGGCGGAGCAAGCCAGGAGGAATTTTACAAATTTATTAAGCCC
ATCTTGGAAAAAATGGACGGCACCGAGGAGCTGCTGGTAAAGCTTAACAGAGAAGATCTGTTGCGCAAACAG
CGCACTTTCGACAATGGAAGCATCCCCCACCAGATTCACCTGGGCGAACTGCACGCTATCCTCAGGCGGCAAG
AGGATTTCTACCCCTTTTTGAAAGATAACAGGGAAAAGATTGAGAAAATCCTCACATTTCGGATACCCTACTA
 
TGTAGGCCCCCTCGCCCGGGGAAATTCCAGATTCGCGTGGATGACTCGCAAATCAGAAGAGACCATCACTCCC
TGGAACTTCGAGGAAGTCGTGGATAAGGGGGCCTCTGCCCAGTCCTTCATCGAAAGGATGACTAACTTTGATA
AAAATCTGCCTAACGAAAAGGTGCTTCCTAAACACTCTCTGCTGTACGAGTACTTCACAGTTTATAACGAGCT
CACCAAGGTCAAATACGTCACAGAAGGGATGAGAAAGCCAGCATTCCTGTCTGGAGAGCAGAAGAAAGCTAT
CGTGGACCTCCTCTTCAAGACGAACCGGAAAGTTACCGTGAAACAGCTCAAAGAAGACTATTTCAAAAAGATT
GAATGTTTCGACTCTGTTGAAATCAGCGGAGTGGAGGATCGCTTCAACGCATCCCTGGGAACGTATCACGATC
TCCTGAAAATCATTAAAGACAAGGACTTCCTGGACAATGAGGAGAACGAGGACATTCTTGAGGACATTGTCCT
CACCCTTACGTTGTTTGAAGATAGGGAGATGATTGAAGAACGCTTGAAAACTTACGCTCATCTCTTCGACGAC
AAAGTCATGAAACAGCTCAAGAGGCGCCGATATACAGGATGGGGGCGGCTGTCAAGAAAACTGATCAATGGG
ATCCGAGACAAGCAGAGTGGAAAGACAATCCTGGATTTTCTTAAGTCCGATGGATTTGCCAACAGGAACTTCA
TGCAGTTGATCCATGATGACTCTCTCACCTTTAAGGAGGACATCCAGAAAGCACAAGTTTCTGGCCAGGGGGA
CAGTCTTCACGAGCACATCGCTAATCTTGCAGGTAGCCCAGCTATCAAAAAGGGAATACTGCAGACCGTTAAG
GTCGTGGATGAACTCGTCAAAGTAATGGGAAGGCATAAGCCCGAGAATATCGTTATCGAGATGGCCCGAGAG
AACCAAACTACCCAGAAGGGACAGAAGAACAGTAGGGAAAGGATGAAGAGGATTGAAGAGGGTATAAAAGA
ACTGGGGTCCCAAATCCTTAAGGAACACCCAGTTGAAAACACCCAGCTTCAGAATGAGAAGCTCTACCTGTAC
TACCTGCAGAACGGCAGGGACATGTACGTGGATCAGGAACTGGACATCAATCGGCTCTCCGACTACGACGTG
GATCATATCGTGCCCCAGTCTTTTCTCAAAGATGATTCTATTGATAATAAAGTGTTGACAAGATCCGATAAAA
ATAGAGGGAAGAGTGATAACGTCCCCTCAGAAGAAGTTGTCAAGAAAATGAAAAATTATTGGCGGCAGCTGC
TGAACGCCAAACTGATCACACAACGGAAGTTCGATAATCTGACTAAGGCTGAACGAGGTGGCCTGTCTGAGTT
GGATAAAGCCGGCTTCATCAAAAGGCAGCTTGTTGAGACACGCCAGATCACCAAGCACGTGGCCCAAATTCTC
GATTCACGCATGAACACCAAGTACGATGAAAATGACAAACTGATTCGAGAGGTGAAAGTTATTACTCTGAAG
TCTAAGCTGGTCTCAGATTTCAGAAAGGACTTTCAGTTTTATAAGGTGAGAGAGATCAACAATTACCACCATG
CGCATGATGCCTACCTGAATGCAGTGGTAGGCACTGCACTTATCAAAAAATATCCCAAGCTTGAATCTGAATT
TGTTTACGGAGACTATAAAGTGTACGATGTTAGGAAAATGATCGCAAAGTCTGAGCAGGAAATAGGCAAGGC
CACCGCTAAGTACTTCTTTTACAGCAATATTATGAATTTTTTCAAGACCGAGATTACACTGGCCAATGGAGAG
ATTCGGAAGCGACCACTTATCGAAACAAACGGAGAAACAGGAGAAATCGTGTGGGACAAGGGTAGGGATTTC
GCGACAGTCCGGAAGGTCCTGTCCATGCCGCAGGTGAACATCGTTAAAAAGACCGAAGTACAGACCGGAGGC
TTCTCCAAGGAAAGTATCCTCCCGAAAAGGAACAGCGACAAGCTGATCGCACGCAAAAAAGATTGGGACCCC
AAGAAATACGGCGGATTCGATTCTCCTACAGTCGCTTACAGTGTACTGGTTGTGGCCAAAGTGGAGAAAGGGA
AGTCTAAAAAACTCAAAAGCGTCAAGGAACTGCTGGGCATCACAATCATGGAGCGATCAAGCTTCGAAAAA
AACCCCATCGACTTTCTCGAGGCGAAAGGATATAAAGAGGTCAAAAAAGACCTCATCATTAAGCTTCCCAAGTA
CTCTCTCTTTGAGCTTGAAAACGGCCGGAAACGAATGCTCGCTAGTGCGGGCGAGCTGCAGAAAGGTAACGA
GCTGGCACTGCCCTCTAAATACGTTAATTTCTTGTATCTGGCCAGCCACTATGAAAAGCTCAAAGGGTCTCCCG
AAGATAATGAGCAGAAGCAGCTGTTCGTGGAACAACACAAACACTACCTTGATGAGATCATCGAGCAAATAA
GCGAATTCTCCAAAAGAGTGATCCTCGCCGACGCTAACCTCGATAAGGTGCTTTCTGCTTACAATAAGCACAG
GGATAAGCCCATCAGGGAGCAGGCAGAAAACATTATCCACTTGTTTACTCTGACCAACTTGGGCGCGCCTGCA
GCCTTCAAGTACTTCGACACCACCATAGACAGAAAGCGGTACACCTCTACAAAGGAGGTCCTGGACGCCACA
CTGATTCATCAGTCAATTACGGGGCTCTATGAAACAAGAATCGACCTCTCTCAGCTCGGTGGAGACAGCAGGG
 
CTGACCCCAAGAAGAAGAGGAAGGTGGGCGGAGGAGGGAGTGGCGGAGGCGGAAGCGGCGGCGGAGGCAG
CGGCTCTAGCCTGGACGACGAGCACATCCTGAGCGCCCTGCTGCAGAGCGACGACGAACTGGTGGGCGAGGA
CAGCGACAGCGAGGTCAGCGACCACGTGTCCGAGGACGACGTGCAGTCCGACACCGAGGAAGCCTTCATCGA
CGAGGTGCACGAAGTGCAGCCTACCAGCAGCGGCTCCGAGATCCTGGACGAGCAGAACGTGATCGAGCAGCC
TGGCAGCTCCCTGGCCAGCAACAGAATCCTGACCCTGCCCCAGAGAACCATCAGAGGCAAGAACAAGCACTG
CTGGTCCACCTCCAAGAGCACCAGGCGGAGCAGAGTGTCCGCCCTGAACATCGTGCGGAGCCAGAGGGGCCC
CACCAGAATGTGCAGAAACATCTACGACCCCCTGCTGTGCTTCAAGCTGTTCTTCACCGACGAGATCATCAGC
GAGATCGTGAAGTGGACCAACGCCGAGATCAGCCTGAAGAGGCGGGAGAGCATGACCAGCGCCACCTTCAGA
GACACCAACGAGGACGAGATCTACGCCTTCTTCGGCATCCTGGTGATGACCGCCGTGAGAAAGGACAACCAC
ATGAGCACCGACGACCTGTTCGACAGATCCCTGAGCATGGTGTACGTGTCCGTGATGAGCAGAGACAGATTCG
ACTTCCTGATCAGATGCCTGAGAATGGACGACAAGAGCATCAGACCCACCCTGCGGGAGAACGACGTGTTCA
CCCCCGTGCGGAAGATCTGGGACCTGTTCATCCACCAGTGCATCCAGAACTACACCCCTGGCGCCCACCTGAC
CATCGATGAGCAGCTGCTGGGCTTCAGAGGCAGATGCCCCTTCAGAGTGTACATCCCCAACAAGCCCAGCAAG
TACGGCATCAAGATCCTGATGATGTGCGACAGCGGCACCAAGTACATGATCAACGGCATGCCCTACCTGGGCA
GAGGCACCCAGACAAACGGCGTGCCCCTGGGCGAGTACTACGTGAAAGAACTGAGCAAGCCTGTGCATGGCA
GCTGCAGGAACATCACCTGCGACAACTGGTTCACCAGCATCCCCCTGGCCAAGAACCTGCTGCAGGAACCCTA
CAAGCTGACCATCGTGGGCACCGTGCGGAGCAACAAGCGGGAGATCCCAGAGGTGCTGAAGAACAGCAGATC
CAGACCTGTGGGAACAAGCATGTTCTGCTTCGACGGCCCCCTGACCCTGGTGTCCTACAAGCCCAAGCCCGCC
AAGATGGTGTACCTGCTGTCCAGCTGCGACGAGGACGCCAGCATCAACGAGAGCACCGGCAAGCCCCAGATG
GTGATGTACTACAACCAGACCAAGGGCGGCGTGGACACCCTGGACCAGATGTGCAGCGTGATGACCTGCAGC
AGAAAGACCAACAGATGGCCCATGGCCCTGCTGTACGGCATGATCAATATCGCCTGCATCAACAGCTTCATCA
TCTACAGCCACAACGTGTCCAGCAAGGGCGAGAAGGTGCAGAGCCGGAAGAAATTCATGCGGAACCTGTACA
TGAGCCTGACCTCCAGCTTCATGAGAAAGAGACTGGAAGCCCCCACCCTGAAGAGATACCTGCGGGACAACA
TCAGCAACATCCTGCCCAAGGAAGTGCCAGGAACAAGCGACGACAGCACCGAGGAACCCGTGATGAAGAAG
AGGACCTACTGCACCTACTGTCCCAGCAAGATCAGAAGAAAGGCCAACGCCAGCTGCAAGAAATGCAAAAAA
GTGATCTGCCGGGAGCACAACATCGACATGTGCCAGAGCTGTTTCTGAAAGGGTTCGATCCCTACCGGTTAGT
AATGAGTTTAAACGGGGGAGGCTAACTGAAACACGGAAGGAGACAATACCGGAAGGAACCCGCGCTATGAC
GGCAATAAAAAGACAGAATAAAACGCACGGGTGTTGGGTCGTTTGTTCATAAACGCGGGGTTCGGTCCCAGG
GCTGGCACTCTGTCGATACCCCACCGAGACCCCATTGGGGCCAATACGCCCGCGTTTCTTCCTTTTCCCCACCC
CACCCCCCAAGTTCGGGTGAAGGCCCAGGGCTCGCAGCCAACGTCGGGGCGGCAGGCCCTGCCATAGCAGAT
CTGCGCAGCTGGGGCTCTAGGGGGTATCCCCACGCGCCCTGTAGCGGCGCATTAAGCGCGGCGGGTGTGGTGG
TTACGCGCAGCGTGACCGCTACACTTGCCAGCGCCCTAGCGCCCGCTCCTTTCGCTTTCTTCCCTTCCTTTCTCG
CCACGTTCGCCGGCTTTCCCCGTCAAGCTCTAAATCGGGGGCTCCCTTTAGGGTTCCGATTTAGTGCTTTACGG
CACCTCGACCCCAAAAAACTTGATTAGGGTGATGGTTCACGTAGTGGGCCATCGCCCTGATAGACGGTTTTTC
GCCCTTTGACGTTGGAGTCCACGTTCTTTAATAGTGGACTCTTGTTCCAAACTGGAACAACACTCAACCCTATC
TCGGTCTATTCTTTTGATTTATAAGGGATTTTGCCGATTTCGGCCTATTGGTTAAAAAATGAGCTGATTTAACA
AAAATTTAACGCGAATTAATTCTGTGGAATGTGTGTCAGTTAGGGTGTGGAAAGTCCCCAGGCTCCCCAGCAG
GCAGAAGTATGCAAAGCATGCATCTCAATTAGTCAGCAACCAGGTGTGGAAAGTCCCCAGGCTCCCCAGCAG
 
GCAGAAGTATGCAAAGCATGCATCTCAATTAGTCAGCAACCATAGTCCCGCCCCTAACTCCGCCCATCCCGCC
CCTAACTCCGCCCAGTTCCGCCCATTCTCCGCCCCATGGCTGACTAATTTTTTTTATTTATGCAGAGGCCGAGG
CCGCCTCTGCCTCTGAGCTATTCCAGAAGTAGTGAGGAGGCTTTTTTGGAGGCCTAGGCTTTTGCAAAAAGCTC
CCGGGAGCTTGTATATCCATTTTCGGATCTGATCAAGAGACAGGATGAGGATCGTTTCGCATGATTGAACAAG
ATGGATTGCACGCAGGTTCTCCGGCCGCTTGGGTGGAGAGGCTATTCGGCTATGACTGGGCACAACAGACAAT
CGGCTGCTCTGATGCCGCCGTGTTCCGGCTGTCAGCGCAGGGGCGCCCGGTTCTTTTTGTCAAGACCGACCTGT
CCGGTGCCCTGAATGAACTGCAGGACGAGGCAGCGCGGCTATCGTGGCTGGCCACGACGGGCGTTCCTTGCGC
AGCTGTGCTCGACGTTGTCACTGAAGCGGGAAGGGACTGGCTGCTATTGGGCGAAGTGCCGGGGCAGGATCT
CCTGTCATCTCACCTTGCTCCTGCCGAGAAAGTATCCATCATGGCTGATGCAATGCGGCGGCTGCATACGCTTG
ATCCGGCTACCTGCCCATTCGACCACCAAGCGAAACATCGCATCGAGCGAGCACGTACTCGGATGGAAGCCG
GTCTTGTCGATCAGGATGATCTGGACGAAGAGCATCAGGGGCTCGCGCCAGCCGAACTGTTCGCCAGGCTCAA
GGCGCGCATGCCCGACGGCGAGGATCTCGTCGTGACCCATGGCGATGCCTGCTTGCCGAATATCATGGTGGAA
AATGGCCGCTTTTCTGGATTCATCGACTGTGGCCGGCTGGGTGTGGCGGACCGCTATCAGGACATAGCGTTGG
CTACCCGTGATATTGCTGAAGAGCTTGGCGGCGAATGGGCTGACCGCTTCCTCGTGCTTTACGGTATCGCCGCT
CCCGATTCGCAGCGCATCGCCTTCTATCGCCTTCTTGACGAGTTCTTCTGAGCGGGACTCTGGGGTTCGCGAAA
TGACCGACCAAGCGACGCCCAACCTGCCATCACGAGATTTCGATTCCACCGCCGCCTTCTATGAAAGGTTGGG
CTTCGGAATCGTTTTCCGGGACGCCGGCTGGATGATCCTCCAGCGCGGGGATCTCATGCTGGAGTTCTTCGCCC
ACCCCAACTTGTTTATTGCAGCTTATAATGGTTACAAATAAAGCAATAGCATCACAAATTTCACAAATAAAGC
ATTTTTTTCACTGCATTCTAGTTGTGGTTTGTCCAAACTCATCAATGTATCTTATCATGTCTGTATACCGTCGAC
CTCTAGCTAGAGCTTGGCGTAATCATGGTCATAGCTGTTTCCTGTGTGAAATTGTTATCCGCTCACAATTCCAC
ACAACATACGAGCCGGAAGCATAAAGTGTAAAGCCTGGGGTGCCTAATGAGTGAGCTAACTCACATTAATTG
CGTTGCGCTCACTGCCCGCTTTCCAGTCGGGAAACCTGTCGTGCCAGCTGcattaatgaatcggccaacgcgcggggagaggcggtt
tgcgtattgggcgctCTTCCGCTTCCTCGCTCACTGACTCGCTGCGCTCGGTCGTTCGGCTGCGGCGAGCGGTATCAGCT
CACTCAAAGGCGGTAATACGGTTATCCACAGAATCAGGGGATAACGCAGGAAAGAACATGTGAGCAAAAGGC
CAGCAAAAGGCCAGGAACCGTAAAAAGGCCGCGTTGCTGGCGTTTTTCCATAGGCTCCGCCCCCCTGACGAGC
ATCACAAAAATCGACGCTCAAGTCAGAGGTGGCGAAACCCGACAGGACTATAAAGATACCAGGCGTTTCCCC
CTGGAAGCTCCCTCGTGCGCTCTCCTGTTCCGACCCTGCCGCTTACCGGATACCTGTCCGCCTTTCTCCCTTCGG
GAAGCGTGGCGCTTTCTCATAGCTCACGCTGTAGGTATCTCAGTTCGGTGTAGGTCGTTCGCTCCAAGCTGGGC
TGTGTGCACGAACCCCCCGTTCAGCCCGACCGCTGCGCCTTATCCGGTAACTATCGTCTTGAGTCCAACCCGGT
AAGACACGACTTATCGCCACTGGCAGCAGCCACTGGTAACAGGATTAGCAGAGCGAGGTATGTAGGCGGTGC
TACAGAGTTCTTGAAGTGGTGGCCTAACTACGGCTACACTAGAAGAACAGTATTTGGTATCTGCGCTCTGCTG
AAGCCAGTTACCTTCGGAAAAAGAGTTGGTAGCTCTTGATCCGGCAAACAAACCACCGCTGGTAGCGGTGGTT
TTTTTGTTTGCAAGCAGCAGATTACGCGCAGAAAAAAAGGATCTCAAGAAGATCCTTTGATCTTTTCTACGGG
GTCTGACGCTCAGTGGAACGAAAACTCACGTTAAGGGATTTTGGTCATGAGATTATCAAAAAGGATCTTCACC
TAGATCCTTTTAAATTAAAAATGAAGTTTTAAATCAATCTAAAGTATATATGAGTAAACTTGGTCTGACAGTTA
CCAATGCTTAATCAGTGAGGCACCTATCTCAGCGATCTGTCTATTTCGTTCATCCATAGTTGCCTGACTCCCCG
TCGTGTAGATAACTACGATACGGGAGGGCTTACCATCTGGCCCCAGTGCTGCAATGATACCGCGAGACCCACG
CTCACCGGCTCCAGATTTATCAGCAATAAACCAGCCAGCCGGAAGGGCCGAGCGCAGAAGTGGTCCTGCAAC
 
TTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGT
TCCCAACGATCAAGGCGAGTTACATGATCCCCCATGTTGTGCAAAAAAGCGGTTAGCTCCTTCGGTCCTCCGA
TCGTTGTCAGAAGTAAGTTGGCCGCAGTGTTATCACTCATGGTTATGGCAGCACTGCATAATTCTCTTACTGTC
ATGCCATCCGTAAGATGCTTTTCTGTGACTGGTGAGTACTCAACCAAGTCATTCTGAGAATAGTGTATGCGGC
GACCGAGTTGCTCTTGCCCGGCGTCAATACGGGATAATACCGCGCCACATAGCAGAACTTTAAAAGTGCTCAT
CATTGGAAAACGTTCTTCGGGGCGAAAACTCTCAAGGATCTTACCGCTGTTGAGATCCAGTTCGATGTAACCC
ACTCGTGCACCCAACTGATCTTCAGCATCTTTTACTTTCACCAGCGTTTCTGGGTGAGCAAAAACAGGAAGGC
AAAATGCCGCAAAAAAGGGAATAAGGGCGACACGGAAATGTTGAATACTCATACTCTTCCTTTTTCAATATTA
TTGAAGCATTTATCAGGGTTATTGTCTCATGAGCGGATACATATTTGAATGTATTTAGAAAAATAAACAAATA
GGGGTTCCGCGCACATTTCCCCGAAAAGTGCCACCTGACGTCGACGGATCGGGAGATCTCCCGATCCCCTATG
GTGCACTCTCAGTACAATCTGCTCTGATGCCGCATAGTTAAGCCAGTATCTGCTCCCTGCTTGTGTGTTGGAGG
TCGCTGAGTAGTGCGCGAGCAAAATTTAAGCTACAACAAGGCAAGGCTTGACCGACAATTGCATGAAGAATC
TGCTTAGGGTTAGGCGTTTTGCGCTGCTTCGCGATGTACGGGCCAGAGGGCCTATTTCCCATGATTCCTTCATA
TTTGCATATACGATACAAGGCTGTTAGAGAGATAATTGGAATTAATTTGACTGTAAACACAAAGATATTAGTA
CAAAATACGTGACGTAGAAAGTAATAATTTCTTGGGTAGTTTGCAGTTTTAAAATTATGTTTTAAAATGGACTA
TCATATGCTTACCGTAACTTGAAAGTATTTCGATTTCTTGGCTTTATATATCTTGTGGAAAGGACGAAACACCG
TCGAGTCGCTTCTCGATTAGTTTTAGAGCTAGAAATAGCAAGTTAAAATAAGGCTAGTCCGTTATCAACTTGA
AAAAGTGGCACCGAGTCGGTGCTTTTTTGTTTTAGAGCTAGAAATAGCAAGTTAAAATAAGGCTAGTCCGTTT
TTAGCGCGTGCGCCAATTCTGCAGACAAATGATATACGCGTTGACATTGATTATTGACTAGTTATTAATAGTAA
TCAATTACGGGGTCATTAGTTCATAGCCCATATATGGAGTTCCGCGTTACATAACTTACGGTAAATGGCCCGCC
TGGCTGACCGCCCAACGACCCCCGCCCATTGACGTCAATAATGACGTATGTTCCCATAGTAACGCCAATAGGG
ACTTTCCATTGACGTCAATGGGTGGAGTATTTACGGTAAACTGCCCACTTGGCAGTACATCAAGTGTATCATAT
GCCAAGTACGCCCCCTATTGACGTCAATGACGGTAAATGGCCCGCCTGGCATTATGCCCAGTACATGACCTTA
TGGGACTTTCCTACTTGGCAGTACATCTACGTATTAGTCATCGCTATTACCATG
 Note: The underlined part is the part encoding PBase.
Cas9-PBase R372A_K375A_D450N encoding DNA sequence(SEQ ID NO:140) 
ATGGACAAGAAGTACTCCATTGGGCTCGacATCGGCACAAACAGCGTCGGCTGGGCCGTCATTACGGACG
AGTACAAGGTGCCGAGCAAAAAATTCAAAGTTCTGGGCAATACCGATCGCCACAGCATAAAGAAGAACCTCA
TTGGCGCCCTCCTGTTCGACTCCGGGGAGACGGCCGAAGCCACGCGGCTCAAAAGAACAGCACGGCGCAGAT
ATACCCGCAGAAAGAATCGGATCTGCTACCTGCAGGAGATCTTTAGTAATGAGATGGCTAAGGTGGATGACTC
TTTCTTCCATAGGCTGGAGGAGTCCTTTTTGGTGGAGGAGGATAAAAAGCACGAGCGCCACCCAATCTTTGGC
AATATCGTGGACGAGGTGGCGTACCATGAAAAGTACCCAACCATATATCATCTGAGGAAGAAGCTTGTAGAC
AGTACTGATAAGGCTGACTTGCGGTTGATCTATCTCGCGCTGGCGCATATGATCAAATTTCGGGGACACTTCCT
CATCGAGGGGGACCTGAACCCAGACAACAGCGATGTCGACAAACTCTTTATCCAACTGGTTCAGACTTACAAT
CAGCTTTTCGAAGAGAACCCGATCAACGCATCCGGAGTTGACGCCAAAGCAATCCTGAGCGCTAGGCTGTCCA
AATCCCGGCGGCTCGAAAACCTCATCGCACAGCTCCCTGGGGAGAAGAAGAACGGCCTGTTTGGTAATCTTAT
 
CGCCCTGTCACTCGGGCTGACCCCCAACTTTAAATCTAACTTCGACCTGGCCGAAGATGCCAAGCTTCAACTG
AGCAAAGACACCTACGATGATGATCTCGACAATCTGCTGGCCCAGATCGGCGACCAGTACGCAGACCTTTTTT
TGGCGGCAAAGAACCTGTCAGACGCCATTCTGCTGAGTGATATTCTGCGAGTGAACACGGAGATCACCAAAG
CTCCGCTGAGCGCTAGTATGATCAAGCGCTATGATGAGCACCACCAAGACTTGACTTTGCTGAAGGCCCTTGT
CAGACAGCAACTGCCTGAGAAGTACAAGGAAATTTTCTTCGATCAGTCTAAAAATGGCTACGCCGGATACATT
GACGGCGGAGCAAGCCAGGAGGAATTTTACAAATTTATTAAGCCCATCTTGGAAAAAATGGACGGCACCGAG
GAGCTGCTGGTAAAGCTTAACAGAGAAGATCTGTTGCGCAAACAGCGCACTTTCGACAATGGAAGCATCCCCC
ACCAGATTCACCTGGGCGAACTGCACGCTATCCTCAGGCGGCAAGAGGATTTCTACCCCTTTTTGAAAGATAA
CAGGGAAAAGATTGAGAAAATCCTCACATTTCGGATACCCTACTATGTAGGCCCCCTCGCCCGGGGAAATTCC
AGATTCGCGTGGATGACTCGCAAATCAGAAGAGACCATCACTCCCTGGAACTTCGAGGAAGTCGTGGATAAG
GGGGCCTCTGCCCAGTCCTTCATCGAAAGGATGACTAACTTTGATAAAAATCTGCCTAACGAAAAGGTGCTTC
CTAAACACTCTCTGCTGTACGAGTACTTCACAGTTTATAACGAGCTCACCAAGGTCAAATACGTCACAGAAGG
GATGAGAAAGCCAGCATTCCTGTCTGGAGAGCAGAAGAAAGCTATCGTGGACCTCCTCTTCAAGACGAACCG
GAAAGTTACCGTGAAACAGCTCAAAGAAGACTATTTCAAAAAGATTGAATGTTTCGACTCTGTTGAAATCAGC
GGAGTGGAGGATCGCTTCAACGCATCCCTGGGAACGTATCACGATCTCCTGAAAATCATTAAAGACAAGGACT
TCCTGGACAATGAGGAGAACGAGGACATTCTTGAGGACATTGTCCTCACCCTTACGTTGTTTGAAGATAGGGA
GATGATTGAAGAACGCTTGAAAACTTACGCTCATCTCTTCGACGACAAAGTCATGAAACAGCTCAAGAGGCGC
CGATATACAGGATGGGGGCGGCTGTCAAGAAAACTGATCAATGGGATCCGAGACAAGCAGAGTGGAAAGAC
AATCCTGGATTTTCTTAAGTCCGATGGATTTGCCAACAGGAACTTCATGCAGTTGATCCATGATGACTCTCTCA
CCTTTAAGGAGGACATCCAGAAAGCACAAGTTTCTGGCCAGGGGGACAGTCTTCACGAGCACATCGCTAATCT
TGCAGGTAGCCCAGCTATCAAAAAGGGAATACTGCAGACCGTTAAGGTCGTGGATGAACTCGTCAAAGTAAT
GGGAAGGCATAAGCCCGAGAATATCGTTATCGAGATGGCCCGAGAGAACCAAACTACCCAGAAGGGACAGA
AGAACAGTAGGGAAAGGATGAAGAGGATTGAAGAGGGTATAAAAGAACTGGGGTCCCAAATCCTTAAGGAA
CACCCAGTTGAAAACACCCAGCTTCAGAATGAGAAGCTCTACCTGTACTACCTGCAGAACGGCAGGGACATGT
ACGTGGATCAGGAACTGGACATCAATCGGCTCTCCGACTACGACGTGGATCATATCGTGCCCCAGTCTTTTCTC
AAAGATGATTCTATTGATAATAAAGTGTTGACAAGATCCGATAAAAATAGAGGGAAGAGTGATAACGTCCCC
TCAGAAGAAGTTGTCAAGAAAATGAAAAATTATTGGCGGCAGCTGCTGAACGCCAAACTGATCACACAACGG
AAGTTCGATAATCTGACTAAGGCTGAACGAGGTGGCCTGTCTGAGTTGGATAAAGCCGGCTTCATCAAAAGGC
AGCTTGTTGAGACACGCCAGATCACCAAGCACGTGGCCCAAATTCTCGATTCACGCATGAACACCAAGTACGA
TGAAAATGACAAACTGATTCGAGAGGTGAAAGTTATTACTCTGAAGTCTAAGCTGGTCTCAGATTTCAGAAAG
GACTTTCAGTTTTATAAGGTGAGAGAGATCAACAATTACCACCATGCGCATGATGCCTACCTGAATGCAGTGG
TAGGCACTGCACTTATCAAAAAATATCCCAAGCTTGAATCTGAATTTGTTTACGGAGACTATAAAGTGTACGA
TGTTAGGAAAATGATCGCAAAGTCTGAGCAGGAAATAGGCAAGGCCACCGCTAAGTACTTCTTTTACAGCAAT
ATTATGAATTTTTTCAAGACCGAGATTACACTGGCCAATGGAGAGATTCGGAAGCGACCACTTATCGAAACAA
ACGGAGAAACAGGAGAAATCGTGTGGGACAAGGGTAGGGATTTCGCGACAGTCCGGAAGGTCCTGTCCATGC
CGCAGGTGAACATCGTTAAAAAGACCGAAGTACAGACCGGAGGCTTCTCCAAGGAAAGTATCCTCCCGAAAA
GGAACAGCGACAAGCTGATCGCACGCAAAAAAGATTGGGACCCCAAGAAATACGGCGGATTCGATTCTCCTA
CAGTCGCTTACAGTGTACTGGTTGTGGCCAAAGTGGAGAAAGGGAAGTCTAAAAAACTCAAAAGCGTCAAGG
 
AACTGCTGGGCATCACAATCATGGAGCGATCAAGCTTCGAAAAAAACCCCATCGACTTTCTCGAGGCGAAAG
GATATAAAGAGGTCAAAAAAGACCTCATCATTAAGCTTCCCAAGTACTCTCTCTTTGAGCTTGAAAACGGCCG
GAAACGAATGCTCGCTAGTGCGGGCGAGCTGCAGAAAGGTAACGAGCTGGCACTGCCCTCTAAATACGTTAA
TTTCTTGTATCTGGCCAGCCACTATGAAAAGCTCAAAGGGTCTCCCGAAGATAATGAGCAGAAGCAGCTGTTC
GTGGAACAACACAAACACTACCTTGATGAGATCATCGAGCAAATAAGCGAATTCTCCAAAAGAGTGATCCTC
GCCGACGCTAACCTCGATAAGGTGCTTTCTGCTTACAATAAGCACAGGGATAAGCCCATCAGGGAGCAGGCA
GAAAACATTATCCACTTGTTTACTCTGACCAACTTGGGCGCGCCTGCAGCCTTCAAGTACTTCGACACCACCAT
AGACAGAAAGCGGTACACCTCTACAAAGGAGGTCCTGGACGCCACACTGATTCATCAGTCAATTACGGGGCT
CTATGAAACAAGAATCGACCTCTCTCAGCTCGGTGGAGACAGCAGGGCTGACCCCAAGAAGAAGAGGAAGGT
GGGCGGAGGAGGGAGTGGCGGAGGCGGAAGCGGCGGCGGAGGCAGCGGCTCTAGCCTGGACGACGAGCACA
TCCTGAGCGCCCTGCTGCAGAGCGACGACGAACTGGTGGGCGAGGACAGCGACAGCGAGGTCAGCGACCACG
TGTCCGAGGACGACGTGCAGTCCGACACCGAGGAAGCCTTCATCGACGAGGTGCACGAAGTGCAGCCTACCA
GCAGCGGCTCCGAGATCCTGGACGAGCAGAACGTGATCGAGCAGCCTGGCAGCTCCCTGGCCAGCAACAGAA
TCCTGACCCTGCCCCAGAGAACCATCAGAGGCAAGAACAAGCACTGCTGGTCCACCTCCAAGAGCACCAGGC
GGAGCAGAGTGTCCGCCCTGAACATCGTGCGGAGCCAGAGGGGCCCCACCAGAATGTGCAGAAACATCTACG
ACCCCCTGCTGTGCTTCAAGCTGTTCTTCACCGACGAGATCATCAGCGAGATCGTGAAGTGGACCAACGCCGA
GATCAGCCTGAAGAGGCGGGAGAGCATGACCAGCGCCACCTTCAGAGACACCAACGAGGACGAGATCTACGC
CTTCTTCGGCATCCTGGTGATGACCGCCGTGAGAAAGGACAACCACATGAGCACCGACGACCTGTTCGACAGA
TCCCTGAGCATGGTGTACGTGTCCGTGATGAGCAGAGACAGATTCGACTTCCTGATCAGATGCCTGAGAATGG
ACGACAAGAGCATCAGACCCACCCTGCGGGAGAACGACGTGTTCACCCCCGTGCGGAAGATCTGGGACCTGT
TCATCCACCAGTGCATCCAGAACTACACCCCTGGCGCCCACCTGACCATCGATGAGCAGCTGCTGGGCTTCAG
AGGCAGATGCCCCTTCAGAGTGTACATCCCCAACAAGCCCAGCAAGTACGGCATCAAGATCCTGATGATGTGC
GACAGCGGCACCAAGTACATGATCAACGGCATGCCCTACCTGGGCAGAGGCACCCAGACAAACGGCGTGCCC
CTGGGCGAGTACTACGTGAAAGAACTGAGCAAGCCTGTGCATGGCAGCTGCAGGAACATCACCTGCGACAAC
TGGTTCACCAGCATCCCCCTGGCCAAGAACCTGCTGCAGGAACCCTACAAGCTGACCATCGTGGGCACCGTGg
ccAGCAACgccCGGGAGATCCCAGAGGTGCTGAAGAACAGCAGATCCAGACCTGTGGGAACAAGCATGTTCTGC
TTCGACGGCCCCCTGACCCTGGTGTCCTACAAGCCCAAGCCCGCCAAGATGGTGTACCTGCTGTCCAGCTGCG
ACGAGGACGCCAGCATCAACGAGAGCACCGGCAAGCCCCAGATGGTGATGTACTACAACCAGACCAAGGGCG
GCGTGGACACCCTGaACCAGATGTGCAGCGTGATGACCTGCAGCAGAAAGACCAACAGATGGCCCATGGCCCT
GCTGTACGGCATGATCAATATCGCCTGCATCAACAGCTTCATCATCTACAGCCACAACGTGTCCAGCAAGGGC
GAGAAGGTGCAGAGCCGGAAGAAATTCATGCGGAACCTGTACATGAGCCTGACCTCCAGCTTCATGAGAAAG
AGACTGGAAGCCCCCACCCTGAAGAGATACCTGCGGGACAACATCAGCAACATCCTGCCCAAGGAAGTGCCA
GGAACAAGCGACGACAGCACCGAGGAACCCGTGATGAAGAAGAGGACCTACTGCACCTACTGTCCCAGCAAG
ATCAGAAGAAAGGCCAACGCCAGCTGCAAGAAATGCAAAAAAGTGATCTGCCGGGAGCACAACATCGACAT
GTGCCAGAGCTGTTTCTGA
 Note: The underlined part is the coding sequence of PBase mutant.

## Claims

1. A PBase protein, comprising amino acid mutations in at least three of following amino acid sites based on an amino acid sequence shown in SEQ ID NO: 3: positions 166, 286, 289, 302, 315, 347, 375, 376, 441, 450, 498, 520, 521, 523, and 557, wherein the PBase protein retains or enhances a gene integration ability, and a target site recognition ability of the PBase protein is reduced.

2. The PBase protein according to claim 1, wherein the PBase protein comprises the amino acid mutations in at least one of the following amino acid sites: R315, N347, K375, and R376, and at least one of the following amino acid sites: A166, N286, S289, G302, Q441, D450, S498, E520, A521, T523, and T557 based on the amino acid sequence shown in SEQ ID NO: 3;
preferably, the PBase protein comprises at least following combinations of the amino acid mutations based on the amino acid sequence shown in SEQ ID NO: 3:
(1) R315, N347, and D450; or
(2) N347, R376, and D450.

3. The PBase protein according to claim 1 or 2, wherein the amino acid mutation is an amino acid substitution, preferably as shown in Table 1:
**Table 1 Amino acid substitutions at PBase mutation sites**
| site | wild amino acid residues | mutant amino acid residues |
|---|---|---|
| 166 | A | H, R, K |
| 286 | N | H, R, K |
| 289 | S | H, R, K |
| 302 | G | H, R, K |
| 315 | R | A, E, D |
| 347 | N | S, T, Y, Q, C, G, A, E, D |
| 375 | K | A, E, D |
| 376 | R | A, E, D |
| 441 | Q | H, R, K |
| 450 | D | N, S, T, C, Y, A, R |
| 498 | S | N |
| 520 | E | H, R, K |
| 521 | A | H, R, K |
| 523 | T | R, N |
| 557 | T | H, R, K |
preferably, the PBase protein comprises at least the following combinations of the amino acid mutations based on the amino acid sequence shown in SEQ ID NO: 3:
(1) R315A1 E/ D, N347S/ T/ Y/ Q/ C/ G/ A/ E/ D, and D450N; or
(2) N347S/ T/ Y/ Q/ C/ G/ A/ E/ D, R376E and D450N;
more preferably, the PBase protein comprises at least the following combinations of the amino acid mutations based on the amino acid sequence shown in SEQ ID NO: 3:
(1) R315A, N347E, and D450N;
(2) N347A, R376E, and D450N;
(3) N347E, R376E, and D450N;
further preferably, the PBase protein comprises the combination of the amino acid mutations N347A, R376E, and D450N based on the amino acid sequence shown in SEQ ID NO: 3, and further comprises one or more of the following amino acid mutations: A166H/ R/ K, N286H/ R/ K, S289H/ R/ K, G302H/ R/ K, Q441H/ R/ K, S498N, E520H/ R/ K, A521H/ R/ K, T523R/ N and T557H/ R/ K, preferably further comprises one or more of the amino acid mutations A166K, N286R, S289R, G302R, Q441R, S498N, E520R, A521R, T523R, and T557R.

4. The PBase according to any one of claims 1-3, wherein the amino acid sequence of the PBase protein is as shown in SEQ ID NO: 5, 7 or 9.

5. A fusion protein, comprising the PBase protein according to any one of claims 1-4 fused to a site-specific DNA nuclease, and original functions of the PBase protein and the site-specific DNA nuclease are not changed after fusion.

6. The fusion protein according to claim 5, wherein the fusion protein comprises at least one of the following features:
in the fusion protein, the site-specific DNA nuclease and the PBase protein are directly connected by a chemical bond or connected through a linker, the linker is preferably 3 * G₄S, and the chemical bond is preferably a peptide bond; more preferably, a structure connected by the linker from a N-terminus to a C-terminus is a site-specific DNA nuclease linker PBase protein; further preferably, the specific DNA nuclease comprises an NLS sequence, and the structure of the fusion protein from the N-terminus to the C-terminus is: specific DNA nuclease NLS linker PBase protein;
or the PBase protein in the fusion protein lacks methionine M at position 1 of the amino acid sequence;
or the site-specific DNA nucleases are Cas, such as Cas9 and Cas12.

7. The fusion protein according to claim 5 or claim 6, wherein the amino acid sequence of the fusion protein is as shown in SEQ ID NO: 6, 8 or 10.

8. An isolated nucleic acid, encoding at least one of the PBase protein according to any one of claims 1-4 or the fusion protein according to any one of claims 5-7;
preferably, the isolated nucleic acid comprises a nucleotide sequence as shown at positions 4183-5964 of SEQ ID NO: 135-137, or the isolated nucleic acid comprises the nucleotide sequence as shown in SEQ ID NO: 135-137.

9. A gene integration system, comprising the PBase protein according to any one of claims 1-4, the fusion protein according to any one of claims 5-7, or the isolated nucleic acid according to claim 8, and Piggybac transposon donor nucleic acid carrying exogenous target genes;
preferably, the fusion protein comprises the PBase protein according to any one of claims 1-4 and a Cas system, wherein the Cas system comprises Cas proteases such as Cas9 and Cas12 proteases and guide RNA (sgRNA).

10. A gene delivery system, comprising one or more vectors, wherein the one or more vectors comprise one or more of the PBase protein according to any one of claims 1-4, the fusion protein according to any one of claims 5-7, the isolated nucleic acid according to claim 8, and the gene integration system according to claim 9; and the one or more vectors also comprise Piggybac transposon donor nucleic acid carrying exogenous target genes, the one or more vectors are such as adenovirus vectors, lipid nanoparticles, lentiviral vectors, ribonucleoprotein complexes, and virus like particles.

11. A gene integration method, comprising the following steps: using the gene integration system according to claim 9 to integrate an exogenous target gene into a genome of a host cell;
preferably, the gene integration system enters the host cell through the gene delivery system according to claim 10;
more preferably, the method is for at least one of non-diagnostic or non-therapeutic purposes.

12. A pharmaceutical composition, comprising one or more of the PBase protein according to any one of claims 1-4, the fusion protein according to any one of claims 5-7, the isolated nucleic acid according to claim 8, the gene integration system according to claim 9, and the gene delivery system according to claim 10.

13. A reagent kit, comprising one or more of the PBase protein according to any one of claims 1-4, the fusion protein according to any one of claims 5-7, the isolated nucleic acid according to claim 8, the gene integration system according to claim 9, the gene delivery system according to claim 10, and the pharmaceutical composition according to claim 12.

14. A kit set, comprising a kit A, wherein the kit A comprises one or more of the PBase protein according to any one of claims 1-4, the fusion protein according to any one of claims 5-7, the isolated nucleic acid according to claim 8, the gene integration system according to claim 9, the gene delivery system according to claim 10, the pharmaceutical composition according to claim 12, and the reagent kit according to claim 13;
preferably, the kit set further comprises a kit B, wherein the kit B comprises reagents for detecting or screening whether gene integration is successful, such as detection reagents that specifically bind to the integrated genes, such as probes and antibodies.

15. A use of one or more of the PBase protein according to any one of claims 1-4, the fusion protein according to any one of claims 5-7, the isolated nucleic acid according to claim 8, the gene integration system according to claim 9, the gene delivery system according to claim 10, the pharmaceutical composition according to claim 12, and the reagent kit according to claim 13 in the preparation of reagents for integrating genes or drugs for treating genetic diseases; preferably, the use is for non-diagnostic or non-therapeutic purposes, and the genetic disease is epidermolysis bullosa.
